# EUROPEAN PATENT SPECIFICATION

(11) **EP 2 510 012 B1**
(45) Date of publication and mention of the grant of the patent: **19.04.2017**
(21) Application number: 10798996.4
(22) Date of filing: 08.12.2010
(51) Int. Cl.: C07K 16/30, A61K 39/395, G01N 33/53, A61P 35/00

(54) **ANTI-C4.4A ANTIBODIES AND USES THEREOF**
ANTIKÖRPER GEGEN C4.4A UND DEREN VERWENDUNG
ANTICORPS CONTRE C4.4A ET LEUR UTILISATION

(30) Priority: 09.12.2009 EP 09178474; 26.07.2010 EP 10170797
(43) Date of publication of application: 17.10.2012
(60) Divisional application: 16191498.1
(73) Proprietor: Bayer Pharma Aktiengesellschaft, 13353 Berlin (DE)
(72) Inventor: LINDEN, Lars, 40235 Düsseldorf (DE); CAO, Yong-Jiang, 13127 Berlin (DE); LEDER, Gabriele, 14163 Berlin (DE); STELTE-LUDWIG, Beatrix, 42489 Wülfrath (DE); HARRENGA, Axel, 42115 Wuppertal (DE); FINNERN, Ricarda, 52074 Aachen (DE); DITTMER, Frank, 40627 Düsseldorf (DE); MAYER-BARTSCHMID, Anke, 42489 Wülfrath (DE); FRANZ, Juergen, 58456 Witten (DE); GREVEN, Simone, 41541 Dormagen (DE); WILLUDA, Jörg, 16548 Glienicke/Nordbahn (DE); TEBBE, Jan, 50733 Köln (DE)
(74) Representative: BIP Patents
(86) International application number: PCT/EP2010/069216
(87) International publication number: WO 2011/070088

(56) References cited:
- WO-A2-01/23553
- HANSEN L V ET AL: "Production, characterization and use of mono- and polyclonal antibodies against C4.4A, a homologue of the urokinase receptor", THROMBOSIS AND HAEMOSTASIS, vol. 93, no. 4, April 2005 (2005-04), page A33, XP009145645, & 10TH INTERENATIONAL WORKSHOP ON MOLECULAR AND CELLULAR BIOLOGY OF PLASMINOGEN ACTIVATION; WASHINGTON, DC, USA; APRIL 09 -13, 2005 ISSN: 0340-6245
- MATZKU S ET AL: "ANTIGENIC DIFFERENCES BETWEEN METASTATIC AND NONMETASTATIC BSP73 RAT TUMOR VARIANTS CHARACTERIZED BY MONOCLONAL ANTIBODIES", CANCER RESEARCH, vol. 49, no. 5, 1989, pages 1294-1299, XP002626953, ISSN: 0008-5472
- PARET C ET AL: "C4.4A as a candidate marker in the diagnosis of colorectal cancer", BRITISH JOURNAL OF CANCER, vol. 97, no. 8, October 2007 (2007-10), pages 1146-1156, XP002626954,
- HANSEN LINE V ET AL: "Structural analysis and tissue localization of human C4.4A: a protein homologue of the urokinase receptor", BIOCHEMICAL JOURNAL, vol. 380, no. Part 3, 15 June 2004 (2004-06-15), pages 845-857, XP002626955, ISSN: 0264-6021
- HANSEN ET AL: "Tumour cell expression of C4.4A, a structural homologue of the urokinase receptor, correlates with poor prognosis in non-small cell lung cancer", LUNG CANCER, ELSEVIER, AMSTERDAM, NL, vol. 58, no. 2, 6 October 2007 (2007-10-06), pages 260-266, XP022288745, ISSN: 0169-5002, DOI: DOI:10.1016/J.LUNGCAN.2007.06.025
- JACOBSEN B ET AL: "The Urokinase Receptor and its Structural Homologue C4.4A in Human Cancer: Expression, Prognosis and Pharmacological Inhibition", CURRENT MEDICINAL CHEMISTRY, vol. 15, no. 25, October 2008 (2008-10), pages 2559-2573, XP002626956, ISSN: 0929-8673
- DATABASE MEDLINE [Online] US NATIONAL LIBRARY OF MEDICINE (NLM), BETHESDA, MD, US 10 January 2001 WÜRFEL J ET AL: 'Cloning of the human homologue of the metastasis-associated rat C4.4A.' Database accession no. NLM11179665 & WÜRFEL J ET AL: 'Cloning of the human homologue of the metastasis-associated rat C4.4A.' GENE vol. 262, no. 1-2, 10 January 2001, pages 35 - 41 ISSN: 0378-1119

### SEQUENCE LISTING

<110> Bayer Schering Pharma AG
<120> Anti-C4.4a Antibodies and Uses Thereof
<130> BHC 09 1 039
<160> 345
<170> PatentIn version 3.5
<210> 1
   <211> 278
   <212> PRT
   <213> Homo Sapiens
<400> 1
   Leu Glu Cys Tyr Ser Cys Val Gln Lys Ala Asp Asp Gly Cys Ser Pro
   1               5                   10                  15
   Asn Lys Met Lys Thr Val Lys Cys Ala Pro Gly Val Asp Val Cys Thr
               20                  25                  30
   Glu Ala Val Gly Ala Val Glu Thr Ile His Gly Gln Phe Ser Leu Ala
           35                  40                  45
   Val Arg Gly Cys Gly Ser Gly Leu Pro Gly Lys Asn Asp Arg Gly Leu
       50                  55                  60
   Asp Leu His Gly Leu Leu Ala Phe Ile Gln Leu Gln Gln Cys Ala Gln
   65                  70                  75                  80
   Asp Arg Cys Asn Ala Lys Leu Asn Leu Thr Ser Arg Ala Leu Asp Pro
                   85                  90                  95
   Ala Gly Asn Glu Ser Ala Tyr Pro Pro Asn Gly Val Glu Cys Tyr Ser
               100                 105                 110
   Cys Val Gly Leu Ser Arg Glu Ala Cys Gln Gly Thr Ser Pro Pro Val
           115                 120                 125
   Val Ser Cys Tyr Asn Ala Ser Asp His Val Tyr Lys Gly Cys Phe Asp
       130                 135                 140
   Gly Asn Val Thr Leu Thr Ala Ala Asn Val Thr Val Ser Leu Pro Val
   145                 150                 155                 160
   Arg Gly Cys Val Gln Asp Glu Phe Cys Thr Arg Asp Gly Val Thr Gly
                   165                 170                 175
   Pro Gly Phe Thr Leu Ser Gly Ser Cys Cys Gln Gly Ser Arg Cys Asn
               180                 185                 190
   Ser Asp Leu Arg Asn Lys Thr Tyr Phe Ser Pro Arg Ile Pro Pro Leu
           195                 200                 205
   Val Arg Leu Pro Pro Pro Glu Pro Thr Thr Val Ala Ser Thr Thr Ser
       210                 215                 220
   Val Thr Thr Ser Thr Ser Ala Pro Val Arg Pro Thr Ser Thr Thr Lys
   225                 230                 235                 240
   Pro Met Pro Ala Pro Thr Ser Gln Thr Pro Arg Gln Gly Val Glu His
                   245                 250                 255
   Glu Ala Ser Arg Asp Glu Glu Pro Arg Leu Thr Gly Gly Ala Ala Gly
               260                 265                 270
   His Gln Asp Arg Ser Asn
           275
<210> 2
   <211> 293
   <212> PRT
   <213> Mus musculus
<400> 2
   Leu Glu Cys Tyr Ser Cys Val Gln Lys Ala Asp Asp Gly Cys Ser Pro
   1               5                   10                  15
   His Arg Met Lys Thr Val Lys Cys Gly Pro Gly Val Asp Val Cys Thr
               20                  25                  30
   Glu Ala Val Gly Ala Val Glu Thr Ile His Gly Gln Phe Ser Val Ala
           35                  40                  45
   Val Arg Gly Cys Gly Ser Gly Ile Pro Gly Lys Asn Asp Arg Gly Leu
       50                  55                  60
   Asp Leu His Gly Leu Leu Ala Phe Phe Gln Leu Gln Gln Cys Ser Glu
   65                  70                  75                  80
   Asp Arg Cys Asn Ala Lys Leu Asn Leu Thr Leu Arg Gly Leu Asn Pro
                   85                  90                  95
   Ala Gly Asn Glu Ser Ala Tyr Glu Pro Asn Gly Ala Glu Cys Tyr Ser
               100                 105                 110
   Cys Val Gly Leu Ser Arg Glu Lys Cys Gln Gly Ser Met Pro Pro Val
           115                 120                 125
   Val Asn Cys Tyr Asn Ala Ser Gly Arg Val Tyr Lys Gly Cys Phe Asp
       130                 135                 140
   Gly Asn Val Thr Leu Thr Ala Ala Asn Val Thr Val Ser Leu Pro Val
   145                 150                 155                 160
   Arg Gly Cys Val Gln Asp Glu Thr Cys Thr Arg Asp Gly Val Thr Gly
                   165                 170                 175
   Pro Gly Phe Thr Leu Ser Gly Ser Cys Cys Gln Gly Pro Arg Cys Asn
               180                 185                 190
   Ala Asp Leu Arg Asn Lys Thr Tyr Phe Ser Pro Arg Ile Pro Pro Leu
           195                 200                 205
   Val Leu Leu Pro Pro Pro Thr Thr Ala Ala Pro Ser Thr Arg Ala Gln
       210                 215                 220
   Asn Ser Ser Ser Thr Thr Ser Thr Ala Ala Pro Thr Thr Thr Thr Ser
   225                 230                 235                 240
   Ile Ile Lys Pro Thr Thr Ala Gln Ala Ser His Thr Ser Pro His Glu
                   245                 250                 255
   Met Asp Leu Glu Val Ile Gln Glu Glu Gly Ala Ser Leu Ser Gly Gly
               260                 265                 270
   Ala Ala Gly His Gly Gly Thr Ala Gly His Gly Gly Ala Ala Gly His
           275                 280                 285
   Gln Asp Arg Ser Asn
       290
<210> 3
   <211> 346
   <212> PRT
   <213> Homo Sapiens
<400> 3
<210> 4
   <211> 363
   <212> PRT
   <213> Mus musculus
<400> 4
   Met Asp Ala Ala Arg Arg Gly Asp Thr Gln Pro Val Met Trp Thr Thr
   1               5                   10                  15
   Gly Trp Leu Leu Leu Leu Pro Leu Leu Leu Cys Glu Gly Ala Gln Ala
               20                  25                  30
   Leu Glu Cys Tyr Ser Cys Val Gln Lys Ala Asp Asp Gly Cys Ser Pro
           35                  40                  45
   His Arg Met Lys Thr Val Lys Cys Gly Pro Gly Val Asp Val Cys Thr
       50                  55                  60
   Glu Ala Val Gly Ala Val Glu Thr Ile His Gly Gln Phe Ser Val Ala
   65                  70                  75                  80
   Val Arg Gly Cys Gly Ser Gly Ile Pro Gly Lys Asn Asp Arg Gly Leu
                   85                  90                  95
   Asp Leu His Gly Leu Leu Ala Phe Phe Gln Leu Gln Gln Cys Ser Glu
               100                 105                 110
   Asp Arg Cys Asn Ala Lys Leu Asn Leu Thr Leu Arg Gly Leu Asn Pro
           115                 120                 125
   Ala Gly Asn Glu Ser Ala Tyr Glu Pro Asn Gly Ala Glu Cys Tyr Ser
       130                 135                 140
   Cys Val Gly Leu Ser Arg Glu Lys Cys Gln Gly Ser Met Pro Pro Val
   145                 150                 155                 160
   Val Asn Cys Tyr Asn Ala Ser Gly Arg Val Tyr Lys Gly Cys Phe Asp
                   165                 170                 175
   Gly Asn Val Thr Leu Thr Ala Ala Asn Val Thr Val Ser Leu Pro Val
               180                 185                 190
   Arg Gly Cys Val Gln Asp Glu Thr Cys Thr Arg Asp Gly Val Thr Gly
           195                 200                 205
   Pro Gly Phe Thr Leu Ser Gly Ser Cys Cys Gln Gly Pro Arg Cys Asn
       210                 215                 220
   Ala Asp Leu Arg Asn Lys Thr Tyr Phe Ser Pro Arg Ile Pro Pro Leu
   225                 230                 235                 240
   Val Leu Leu Pro Pro Pro Thr Thr Ala Ala Pro Ser Thr Arg Ala Gln
                   245                 250                 255
   Asn Ser Ser Ser Thr Thr Ser Thr Ala Ala Pro Thr Thr Thr Thr Ser
               260                 265                 270
   Ile Ile Lys Pro Thr Thr Ala Gln Ala Ser His Thr Ser Pro His Glu
           275                 280                 285
   Met Asp Leu Glu Val Ile Gln Glu Glu Gly Ala Ser Leu Ser Gly Gly
       290                 295                 300
   Ala Ala Gly His Gly Gly Thr Ala Gly His Gly Gly Ala Ala Gly His
   305                 310                 315                 320
   Gln Asp Arg Ser Asn Met Glu Lys Tyr Pro Gly Lys Gly Gly Ala Gln
                   325                 330                 335
   Ile Pro Ala Lys Gly Gly Ser Gly Thr Leu Gly Ser Trp Leu Ser Ala
               340                 345                 350
   Val Leu Leu Thr Val Val Ala Gly Ala Met Leu
           355                 360
<210> 5
   <211> 9
   <212> PRT
   <213> Artificial Sequence
<220>
   <223> C4.4a binder
<400> 5
   Phe Ser Asn Ala Trp Met Ser Trp Val
   1               5
<210> 6
   <211> 9
   <212> PRT
   <213> Artificial Sequence
<220>
   <223> C4.4a binder
<400> 6
   Phe Ser Asp Tyr Gln Met Thr Trp Ile
   1 5
<210> 7
   <211> 9
   <212> PRT
   <213> Artificial Sequence
<220>
   <223> C4.4a binder
<400> 7
   Phe Gly His Tyr Tyr Met Phe Trp Ile
   1 5
<210> 8
   <211> 8
   <212> PRT
   <213> Artificial Sequence
<220>
   <223> C4.4a binder
<400> 8
   Phe Ser Ser Asn Tyr Met Ser Trp
   1 5
<210> 9
   <211> 20
   <212> PRT
   <213> Artificial Sequence
<220>
   <223> C4.4a binder
<400> 9
   Val Ser Tyr Ile Ser Ser Ser Gly Ser Thr Ile Tyr Tyr Ala Asp Ser
   1 5 10 15
   Val Lys Gly Arg
   20
<210> 10
   <211> 20
   <212> PRT
   <213> Artificial Sequence
<220>
   <223> C4.4a binder
<400> 10
   Val Ser Gly Val Ser Trp Asn Gly Ala Arg Thr His Tyr Ala Asp Ser
   1               5                   10                  15
   Val Lys Gly Arg
               20
<210> 11
   <211> 20
   <212> PRT
   <213> Artificial Sequence
<220>
   <223> C4.4a binder
<400> 11
   Val Ser Ala Ile Ser Gly Ser Gly Tyr Ser Thr His Tyr Ala Asp Ser
   1               5                   10                  15
   Val Lys Gly Arg
               20
<210> 12
   <211> 20
   <212> PRT
   <213> Artificial Sequence
<220>
   <223> C4.4a binder
<400> 12
   Val Ser Ala Ile Ser Ser Ser Gly Ser Ser Thr Tyr Tyr Ala Asp Ser
   1               5                   10                  15
   Val Lys Gly Arg
               20
<210> 13
   <211> 10
   <212> PRT
   <213> Artificial Sequence
<220>
   <223> C4.4a binder
<400> 13
   Ala Arg Glu Gly Leu Trp Ala Phe Asp Tyr
   1               5                   10
<210> 14
   <211> 15
   <212> PRT
   <213> Artificial Sequence
<220>
   <223> C4.4a binder
<400> 14
   Ala Lys Gly Asp Tyr Leu Val Tyr Ser Ala Tyr Tyr Phe Asp Ser
   1 5 10 15
<210> 15
   <211> 13
   <212> PRT
   <213> Artificial Sequence
<220>
   <223> C4.4a binder
<400> 15
   Ala Arg Leu Pro Tyr Gly Ser Gln Ser Gly Val Asp Tyr
   1 5 10
<210> 16
   <211> 17
   <212> PRT
   <213> Artificial Sequence
<220>
   <223> C4.4a binder
<400> 16
   Ala Arg Glu Ser Gly Gly Ser Gly Pro Asn Tyr Tyr Tyr Gly Met Asp
   1 5 10 15
   Val
<210> 17
   <211> 14
   <212> PRT
   <213> Artificial Sequence
<220>
   <223> C4.4a binder
<400> 17
   Thr Gly Ser Ser Ser Asn Ile Gly Ala Gly Tyr Val Val His
   1 5 10
<210> 18
   <211> 13
   <212> PRT
   <213> Artificial Sequence
<220>
   <223> C4.4a binder
<400> 18
   Ser Gly Ser Ser Ser Asn Val Gly Ser Asn Pro Val Asn
   1 5 10
<210> 19
   <211> 14
   <212> PRT
   <213> Artificial Sequence
<220>
   <223> C4.4a binder
<400> 19
   Thr Gly Ser Ser Ser Asn Ile Gly Ala Gly Tyr Val Val His
   1 5 10
<210> 20
   <211> 14
   <212> PRT
   <213> Artificial Sequence
<220>
   <223> C4.4a binder
<400> 20
   Thr Gly Ser Ser Ser Asn Ile Gly Ala Gly Tyr Val Val His
   1 5 10
<210> 21
   <211> 7
   <212> PRT
   <213> Artificial Sequence
<220>
   <223> C4.4a binder
<400> 21
   Asp Asn Asn Lys Arg Pro Ser
   1 5
<210> 22
   <211> 7
   <212> PRT
   <213> Artificial Sequence
<220>
   <223> C4.4a binder
<400> 22
   Arg Asn Asn Gln Arg Pro Ser
   1 5
<210> 23
   <211> 7
   <212> PRT
   <213> Artificial Sequence
<220>
   <223> C4.4a binder
<400> 23
   Ser Asn Asn Gln Arg Pro Ser
   1               5
<210> 24
   <211> 7
   <212> PRT
   <213> Artificial Sequence
<220>
   <223> C4.4a binder
<400> 24
   Ser Asn Asn Gln Arg Pro Ser
   1               5
<210> 25
   <211> 12
   <212> PRT
   <213> Artificial Sequence
<220>
   <223> C4.4a binder
<400> 25
   Cys Ala Ala Trp Asp Asp Arg Leu Asn Gly Pro Val
   1               5                   10
<210> 26
   <211> 12
   <212> PRT
   <213> Artificial Sequence
<220>
   <223> C4.4a binder
<400> 26
   Cys Ala Ala Trp Asp Asp Arg Leu Asn Gly Trp Val
   1               5                   10
<210> 27
   <211> 10
   <212> PRT
   <213> Artificial Sequence
<220>
   <223> C4.4a binder
<400> 27
   Cys Gln Ser Tyr Asp Ser Ser His Val Leu
   1               5                   10
<210> 28
   <211> 12
   <212> PRT
   <213> Artificial Sequence
<220>
   <223> C4.4a binder
<400> 28
   Cys Gln Ser Tyr Asp Arg Ser Leu Arg Gly Trp Val
   1               5                   10
<210> 29
   <211> 113
   <212> PRT
   <213> Artificial Sequence
<220>
   <223> C4.4a binder
<400> 29
   Gln Ser Val Leu Thr Gln Pro Pro Ser Ala Ser Gly Thr Pro Gly Gln
   1               5                   10                  15
   Arg Val Thr Ile Ser Cys Thr Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Lys Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Trp Asp Asp Arg
                   85                  90                  95
   Leu Asn Gly Pro Val Phe Gly Gly Gly Thr Lys Leu Thr Val Leu Gly
               100                 105                 110
   Gln
<210> 30
   <211> 112
   <212> PRT
   <213> Artificial Sequence
<220>
   <223> C4.4a binder
<400> 30
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Val Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
                   85                  90                  95
   Asn Gly Trp Val Phe Gly Gly Gly Thr Lys Leu Thr Val Leu Gly Gln
               100                 105                 110
<210> 31
   <211> 111
   <212> PRT
   <213> Artificial Sequence
<220>
   <223> C4.4a binder
<400> 31
   Gln Ser Val Leu Thr Gln Pro Pro Ser Ala Ser Gly Thr Pro Gly Gln
   1               5                   10                  15
   Arg Val Thr Ile Ser Cys Thr Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Ser Asn Asn Gln Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Gln Ser Tyr Asp Ser Ser
                   85                  90                  95
   His Val Leu Phe Gly Gly Gly Thr Lys Leu Thr Val Leu Gly Gln
               100                 105                 110
<210> 32
   <211> 113
   <212> PRT
   <213> Artificial Sequence
<220>
   <223> C4.4a binder
<400> 32
   Gln Ser Val Leu Thr Gln Pro Pro Ser Ala Ser Gly Thr Pro Gly Gln
   1 5 10 15
   Arg Val Thr Ile Ser Cys Thr Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Ser Asn Asn Gln Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Gln Ser Tyr Asp Arg Ser
   85 90 95
   Leu Arg Gly Trp Val Phe Gly Gly Gly Thr Lys Leu Thr Val Leu Gly
   100 105 110
   Gln
<210> 33
   <211> 117
   <212> PRT
   <213> Artificial Sequence
<220>
   <223> C4.4a binder
<400> 33
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Ser Tyr Ile Ser Ser Ser Gly Ser Thr Ile Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Glu Gly Leu Trp Ala Phe Asp Tyr Trp Gly Gln Gly Thr Leu
   100 105 110
   Val Thr Val Thr Ser
   115
<210> 34
   <211> 122
   <212> PRT
   <213> Artificial Sequence
<220>
   <223> C4.4a binder
<400> 34
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asp Tyr
   20 25 30
   Gln Met Thr Trp Ile Arg Gln Thr Pro Gly Lys Gly Leu Glu Trp Val
   35 40 45
   Ser Gly Val Ser Trp Asn Gly Ala Arg Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Asp Tyr Leu Val Tyr Ser Ala Tyr Tyr Phe Asp Ser Trp
   100 105 110
   Gly Gln Gly Thr Leu Val Thr Val Thr Ser
   115 120
<210> 35
   <211> 120
   <212> PRT
   <213> Artificial Sequence
<220>
   <223> C4.4a binder
<400> 35
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Gly His Tyr
               20                  25                  30
   Tyr Met Phe Trp Ile Arg Gln Ala Pro Gly Lys Gly Leu Glu Trp Val
           35                  40                  45
   Ser Ala Ile Ser Gly Ser Gly Tyr Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Leu Pro Tyr Gly Ser Gln Ser Gly Val Asp Tyr Trp Gly Gln
               100                 105                 110
   Gly Thr Leu Val Thr Val Thr Ser
           115                 120
<210> 36
   <211> 124
   <212> PRT
   <213> Artificial Sequence
<220>
   <223> C4.4a binder
<400> 36
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Asn
               20                  25                  30
   Tyr Met Ser Trp Val Arg Gln Ala Pro Gly Lys Gly Leu Glu Trp Val
           35                  40                  45
   Ser Ala Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Ser Gly Gly Ser Gly Pro Asn Tyr Tyr Tyr Gly Met Asp
               100                 105                 110
   Val Trp Gly Gln Gly Thr Leu Val Thr Val Thr Ser
           115                 120
<210> 37
   <211> 339
   <212> DNA
   <213> Artificial Sequence
<220>
   <223> C4.4a binder
<400> 37
   cagtctgtgc tgactcagcc accctcagcg tctgggaccc ctgggcagag ggtcaccatc      60
   tcctgcactg ggagcagctc caacattggg gcgggttatg ttgtacattg gtatcagcag     120
   ctcccaggaa cggcccccaa actcctcatc tatgacaata ataagcgacc ctcaggggtc     180
   cctgaccgat tctctggctc caagtctggc acctcagcct ccctggccat cagtgggctc     240
   cggtccgagg atgaggctga ttattactgt gcagcatggg atgacaggct gaatggtccg     300
   gtgttcggcg gaggaaccaa gttaaccgtc ctaggtcag                            339
<210> 38
   <211> 336
   <212> DNA
   <213> Artificial Sequence
<220>
   <223> C4.4a binder
<400> 38
   cagtctgtgc tgactcagcc accctcagcg tctgggaccc ccgggcagag ggtcaccatc 60
   tcttgttctg gaagcagctc caacgtcggg agtaatcctg taaactggta tcagcagctc 120
   ccaggaacgg cccccaaact cctcatctat aggaataatc agcggccctc aggggtccct 180
   gaccgattct ctggctccaa gtctggcacc tcagcctccc tggccatcag tgggctccgg 240
   tccgaggatg aggctgatta ttactgtgca gcatgggatg acaggctgaa tggttgggtg 300
   ttcggcggag gaaccaagct gacggtccta ggtcag 336
<210> 39
   <211> 333
   <212> DNA
   <213> Artificial Sequence
<220>
   <223> C4.4a binder
<400> 39
   cagtctgtgc tgactcagcc accctcagcg tctgggaccc ccgggcagag ggtcaccatc 60
   tcctgcactg ggagcagctc caacattggg gcgggttatg ttgtacattg gtatcagcag 120
   ctcccaggaa cggcccccaa actcctcatc tatagtaata atcagcggcc ctcaggagtc 180
   cctgaccgat tctctggctc caagtctggc acctcagcct ccctggccat cagtgggctc 240
   cggtccgagg atgaggctga ttattactgc cagtcctatg acagcagcca tgttttattc 300
   ggcggaggaa ccaagctgac ggtcctaggt cag 333
<210> 40
   <211> 339
   <212> DNA
   <213> Artificial Sequence
<220>
   <223> C4.4a binder
<400> 40
   cagtctgtgc tgactcagcc accctcagcg tctgggaccc ccgggcagag ggtcaccatc 60
   tcctgcactg ggagcagctc caacattggg gcgggttatg ttgtacattg gtatcagcag 120
   ctcccaggaa cggcccccaa actcctcatc tatagtaata atcagcggcc ctcaggggtc 180
   cctgaccgat tctctggctc caagtctggc acctcagcct ccctggccat cagtgggctc 240
   cggtccgagg atgaggctga ttattactgc cagtcctatg acagaagcct gcgtggttgg 300
   gtgttcggcg gaggaaccaa gctgacggtc ctaggtcag 339
<210> 41
   <211> 351
   <212> DNA
   <213> Artificial Sequence
<220>
   <223> C4.4a binder
<400> 41
   gaggtgcagc tgttggagtc tgggggaggc ttggtacagc ctggggggtc cctgagactc     60
   tcctgtgcag cctctggatt caccttcagt aacgcctgga tgagctgggt ccgccaggct    120
   ccagggaagg ggctggagtg ggtttcatac attagtagta gtggtagtac catatactac    180
   gcagactctg tgaagggccg attcaccatc tccagagaca attccaagaa cacgctgtat    240
   ctgcaaatga acagcctgag agccgaggac actgccgtgt attactgtgc gagagaaggg    300
   ttatgggcct ttgactactg gggccagggt accctggtca ccgtgactag t             351
<210> 42
   <211> 366
   <212> DNA
   <213> Artificial Sequence
<220>
   <223> C4.4a binder
<400> 42
   gaggtgcagc tgttggagtc tgggggaggc ttggtacagc ctggggggtc cctgagactc     60
   tcctgtgcag cctctggatt caccttcagt gactatcaga tgacctggat ccgccagact    120
   ccagggaagg ggctggagtg ggtatcgggt gttagttgga atggcgctag gacgcactat    180
   gcagactctg tgaagggccg attcaccatc tccagagaca attccaagaa cacgctgtat    240
   ctacaaatga acagcctgag agccgaggac actgccgtgt attactgtgc gaagggcgac    300
   tacctggttt actccgcata ctactttgac tcctggggcc agggtaccct ggtcaccgtg    360
   actagt                                                               366
<210> 43
   <211> 360
   <212> DNA
   <213> Artificial Sequence
<220>
   <223> C4.4a binder
<400> 43
   gaggtgcagc tgttggagtc tgggggaggc ttggtacagc ctggggggtc cctgagactc       60
   tcctgtgcag cctctggatt caccttcggt cactactata tgttctggat ccgtcaggct      120
   ccagggaagg ggctggagtg ggtctcagct attagtggta gtggttatag cacacactac      180
   gcagactccg tgaagggccg gttcaccatc tccagagaca attccaagaa cacgctgtat      240
   ctgcaaatga acagcctgag agccgaggac actgccgtgt attactgtgc gagactgcca      300
   tatggttcgc agagtggcgt tgactactgg ggccagggta ccctggtcac cgtgactagt      360
<210> 44
   <211> 372
   <212> DNA
   <213> Artificial Sequence
<220>
   <223> C4.4a binder
<400> 44
   gaggtgcagc tgttggagtc tgggggaggc ttggtacagc ctggggggtc cctgagactc       60
   tcctgtgcag cctctggatt caccttcagt agcaactaca tgagctgggt ccgccaggct      120
   ccagggaagg ggctggagtg ggtctcagct attagtagta gtggtagtag cacatactac      180
   gcagactccg tgaagggccg gttcaccatc tccagagaca attccaagaa cacgctgtat      240
   ctgcaaatga acagcctgag agccgaggac actgccgtgt attactgtgc gagagaatct      300
   ggtgggagcg gaccgaacta ctactacggt atggacgtct ggggccaagg taccctggtc      360
   accgtgacta gt                                                          372
<210> 45
   <211> 9
   <212> PRT
   <213> Artificial Sequence
<220>
   <223> C4.4a binder
<400> 45
   Phe Ser Asn Ala Trp Met Ser Trp Val
   1               5
<210> 46
   <211> 20
   <212> PRT
   <213> Artificial Sequence
<220>
   <223> C4.4a binder
<400> 46
   Val Ser Tyr Ile Ser Ser Ser Gly Ser Thr Ile Tyr Tyr Ala Asp Ser
   1 5 10 15
   Val Lys Gly Arg
   20
<210> 47
   <211> 10
   <212> PRT
   <213> Artificial Sequence
<220>
   <223> C4.4a binder
<400> 47
   Ala Arg Glu Gly Leu Trp Ala Phe Asp Tyr
   1 5 10
<210> 48
   <211> 14
   <212> PRT
   <213> Artificial Sequence
<220>
   <223> C4.4a binder
<400> 48
   Thr Gly Ser Ser Ser Asn Ile Gly Ala Gly Tyr Val Val His
   1 5 10
<210> 49
   <211> 7
   <212> PRT
   <213> Artificial Sequence
<220>
   <223> C4.4a binder
<400> 49
   Asp Asn Asn Lys Arg Pro Ser
   1 5
<210> 50
   <211> 12
   <212> PRT
   <213> Artificial Sequence
<220>
   <223> C4.4a binder
<400> 50
   Cys Ala Ala Trp Asp Asp Arg Leu Asn Gly Pro Val
   1 5 10
<210> 51
   <211> 117
   <212> PRT
   <213> Artificial Sequence
<220>
   <223> C4.4a binder
<400> 51
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Tyr Trp Gly Gln Gly Thr Leu
               100                 105                 110
   Val Thr Val Ser Ser
           115
<210> 52
   <211> 113
   <212> PRT
   <213> Artificial Sequence
<220>
   <223> C4.4a binder
<400> 52
   Glu Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Lys Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Trp Asp Asp Arg
                   85                  90                  95
   Leu Asn Gly Pro Val Phe Gly Gly Gly Thr Lys Leu Thr Val Leu Gly
               100                 105                 110
   Gln
<210> 53
   <211> 351
   <212> DNA
   <213> Artificial Sequence
<220>
   <223> C4.4a binder
<400> 53
   gaggtgcagc tgctggaaag cggcggagga ctggtgcagc ctggaggcag cctgagactg      60
   tcttgcgccg ccagcggctt caccttcagc aacgcctgga tgagctgggt ccgacaggct     120
   cctggcaagg gcctggaatg ggtgtcctac atcagcagca gcggcagcac catctactac     180
   gccgacagcg tgaagggccg gttcaccatc agccgggaca acagcaagaa caccctgtac     240
   ctgcagatga acagcctgcg ggccgaggac accgccgtgt actactgcgc cagagaaggc     300
   ctgtgggcct tcgactactg gggccagggc accctggtca ccgtgtctag c             351
<210> 54
   <211> 339
   <212> DNA
   <213> Artificial Sequence
<220>
   <223> C4.4a binder
<400> 54
   gagagcgtgc tgacccagcc tcctagcgtg tccggcgctc ctggccagag agtgaccatc      60
   agctgcaccg gcagcagcag caacatcgga gccggctacg tggtgcactg gtatcagcag     120
   ctgcccggca ccgcccccaa gctgctgatc tacgacaaca acaagcggcc tagcggcgtg     180
   cccgacagat tcagcggcag caagagcggc accagcgcca gcctggccat cagcggcctg     240
   agaagcgagg acgaggccga ctactactgc gccgcctggg acgacagact gaacggccct     300
   gtgttcggcg gaggcaccaa gctgaccgtg ctgggacag                            339
<210> 55
   <211> 9
   <212> PRT
   <213> Artificial Sequence
<220>
   <223> C4.4a binder
<400> 55
   Phe Ser Asn Ala Trp Met Ser Trp Val
   1               5
<210> 56
   <211> 20
   <212> PRT
   <213> Artificial Sequence
<220>
   <223> C4.4a binder
<400> 56
   Val Ser Tyr Ile Ser Ser Ser Gly Ser Thr Ile Tyr Tyr Ala Asp Ser
   1               5                   10                  15
   Val Lys Gly Arg
               20
<210> 57
   <211> 10
   <212> PRT
   <213> Artificial Sequence
<220>
   <223> C4.4a binder
<400> 57
   Ala Arg Glu Gly Leu Trp Ala Phe Asp Tyr
   1               5                   10
<210> 58
   <211> 14
   <212> PRT
   <213> Artificial Sequence
<220>
   <223> C4.4a binder
<400> 58
   Thr Gly Ser Ser Ser Asn Ile Gly Ala Gly Tyr Val Val His
   1               5                   10
<210> 59
   <211> 7
   <212> PRT
   <213> Artificial Sequence
<220>
   <223> C4.4a binder
<400> 59
   Asp Asn Asn Lys Arg Pro Ser
   1               5
<210> 60
   <211> 12
   <212> PRT
   <213> Artificial Sequence
<220>
   <223> C4.4a binder
<400> 60
   Cys Ala Ala Tyr Asp Asp Ser Leu Ser Gly Pro Val
   1               5                   10
<210> 61
   <211> 117
   <212> PRT
   <213> Artificial Sequence
<220>
   <223> C4.4a binder
<400> 61
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Tyr Trp Gly Gln Gly Thr Leu
               100                 105                 110
   Val Thr Val Ser Ser
           115
<210> 62
   <211> 113
   <212> PRT
   <213> Artificial Sequence
<220>
   <223> C4.4a binder
<400> 62
   Gln Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Lys Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Tyr Asp Asp Ser
                   85                  90                  95
   Leu Ser Gly Pro Val Phe Gly Gly Gly Thr Lys Leu Thr Val Leu Gly
               100                 105                 110
   Gln
<210> 63
   <211> 351
   <212> DNA
   <213> Artificial Sequence
<220>
   <223> C4.4a binder
<400> 63
   gaggtgcagc tgctggaatc cggcggaggc ctggtgcagc ctggcggatc tctgagactg      60
   tcctgcgccg ccagcggctt taccttctcc aacgcctgga tgtcctgggt ccgacaggcc     120
   cctggcaagg gactggaatg ggtgtcctac atctcctcct ccggctccac catctactac     180
   gccgactccg tgaagggccg gttcaccatc tcccgggaca actccaagaa caccctgtac     240
   ctgcagatga actccctgcg ggccgaggac accgccgtgt actactgcgc ccgagagggc     300
   ctgtgggcct tcgattattg gggccagggc accctggtca ccgtcagctc a              351
<210> 64
   <211> 339
   <212> DNA
   <213> Artificial Sequence
<220>
   <223> C4.4a binder
<400> 64
   cagtccgtgc tgacccagcc cccttctgtg tctggcgccc ctggccagag agtgaccatc 60
   tcttgcaccg gctcctccag caacatcggc gctggctacg tggtgcactg gtatcagcag 120
   ctgcccggca ccgcccccaa gctgctgatc tacgacaaca acaagcggcc ctccggcgtg 180
   cccgacagat tctccggctc caagtccggc acctccgcct ccctggccat ctccggcctg 240
   agatctgagg acgaggccga ctactactgc gccgcctacg acgactccct gtccggccct 300
   gtgttcggcg gaggcacaaa gttaaccgtg ctgggccag 339
<210> 65
   <211> 9
   <212> PRT
   <213> Artificial Sequence
<220>
   <223> C4.4a binder
<400> 65
   Phe Ser Asn Ala Trp Met Ser Trp Val
   1 5
<210> 66
   <211> 20
   <212> PRT
   <213> Artificial Sequence
<220>
   <223> C4.4a binder
<400> 66
   Val Ser Tyr Ile Ser Ser Ser Gly Ser Thr Ile Tyr Tyr Ala Asp Ser
   1 5 10 15
   Val Lys Gly Arg
   20
<210> 67
   <211> 10
   <212> PRT
   <213> Artificial Sequence
<220>
   <223> C4.4a binder
<400> 67
   Ala Arg Glu Gly Leu Trp Ala Phe Asp Tyr
   1 5 10
<210> 68
   <211> 14
   <212> PRT
   <213> Artificial Sequence
<220>
   <223> C4.4a binder
<400> 68
   Thr Gly Ser Ser Ser Asn Ile Gly Ala Gly Tyr Val Val His
   1               5                   10
<210> 69
   <211> 7
   <212> PRT
   <213> Artificial Sequence
<220>
   <223> C4.4a binder
<400> 69
   Asp Asn Asn Lys Arg Pro Ser
   1               5
<210> 70
   <211> 12
   <212> PRT
   <213> Artificial Sequence
<220>
   <223> C4.4a binder
<400> 70
   Cys Ala Ala Phe Asp Asp Ser Leu Asn Gly Pro Val
   1               5                   10
<210> 71
   <211> 117
   <212> PRT
   <213> Artificial Sequence
<220>
   <223> C4.4a binder
<400> 71
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Ser Tyr Ile Ser Ser Ser Gly Ser Thr Ile Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Glu Gly Leu Trp Ala Phe Asp Lys Trp Gly Gln Gly Thr Leu
   100 105 110
   Val Thr Val Ser Ser
   115
<210> 72
   <211> 113
   <212> PRT
   <213> Artificial Sequence
<220>
   <223> C4.4a binder
<400> 72
   Gln Ser Val Leu Thr Gln Pro Pro Ser Ala Ser Gly Thr Pro Gly Gln
   1 5 10 15
   Arg Val Thr Ile Ser Cys Thr Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Lys Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Phe Asp Asp Arg
   85 90 95
   Leu Asn Gly Pro Val Phe Gly Gly Gly Thr Lys Leu Thr Val Leu Gly
   100 105 110
   Gln
<210> 73
   <211> 351
   <212> DNA
   <213> Artificial Sequence
<220>
   <223> C4.4a binder
<400> 73
   gaggtgcagc tgctggaatc cggcggaggc ctggtgcagc ctggcggatc tctgagactg 60
   tcctgcgccg cctccggctt taccttctcc aacgcctgga tgtcctgggt ccgacaggct 120
   cctggcaagg gcctggaatg ggtgtcctac atctcctcct ccggctccac catctactac 180
   gccgactccg tgaagggccg gttcaccatc tcccgggaca actccaagaa caccctgtac 240
   ctgcagatga actccctgcg ggccgaggac accgccgtgt actactgcgc ccgagagggc 300
   ctgtgggcct tcgataagtg gggccagggc accctggtca ccgtcagctc a 351
<210> 74
   <211> 339
   <212> DNA
   <213> Artificial Sequence
<220>
   <223> C4.4a binder
<400> 74
   cagtccgtgc tgacccagcc tccttccgcc tctggcaccc ctggccagag agtgaccatc 60
   tcctgcaccg gctcctccag caacatcggc gctggctacg tggtgcactg gtatcagcag 120
   ctgcccggca ccgcccccaa gctgctgatc tacgacaaca acaagcggcc ctccggcgtg 180
   cccgacagat tctccggctc caagtccggc acctccgcct ccctggccat ctccggcctg 240
   agatctgagg acgaggccga ctactactgc gccgccttcg acgaccggct gaacggccct 300
   gtgttcggcg gaggcacaaa gttaaccgtg ctgggccag 339
<210> 75
   <211> 9
   <212> PRT
   <213> Artificial Sequence
<220>
   <223> C4.4a binder
<400> 75
   Phe Ser Ser Ala Trp Met Ser Trp Val
   1               5
<210> 76
   <211> 20
   <212> PRT
   <213> Artificial Sequence
<220>
   <223> C4.4a binder
<400> 76
   Val Ser Tyr Ile Ser Ser Ser Gly Ser Thr Ile Tyr Tyr Ala Asp Ser
   1               5                   10                  15
   Val Lys Gly Arg
               20
<210> 77
   <211> 10
   <212> PRT
   <213> Artificial Sequence
<220>
   <223> C4.4a binder
<400> 77
   Ala Arg Glu Gly Leu Trp Ala Phe Asp Tyr
   1               5                   10
<210> 78
   <211> 14
   <212> PRT
   <213> Artificial Sequence
<220>
   <223> C4.4a binder
<400> 78
   Thr Gly Ser Ser Ser Asn Ile Gly Ala Gly Tyr Val Val His
   1               5                   10
<210> 79
   <211> 7
   <212> PRT
   <213> Artificial Sequence
<220>
   <223> C4.4a binder
<400> 79
   Asp Asn Asn Lys Arg Pro Ser
   1 5
<210> 80
   <211> 12
   <212> PRT
   <213> Artificial Sequence
<220>
   <223> C4.4a binder
<400> 80
   Cys Ala Ala Tyr Asp Asp Ser Leu Ser Gly Pro Val
   1 5 10
<210> 81
   <211> 117
   <212> PRT
   <213> Artificial Sequence
<220>
   <223> C4.4a binder
<400> 81
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Tyr Trp Gly Gln Gly Thr Leu
               100                 105                 110
   Val Thr Val Ser Ser
           115
<210> 82
   <211> 113
   <212> PRT
   <213> Artificial Sequence
<220>
   <223> C4.4a binder
<400> 82
   Gln Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Lys Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Tyr Asp Asp Ser
                   85                  90                  95
   Leu Ser Gly Pro Val Phe Gly Gly Gly Thr Lys Leu Thr Val Leu Gly
               100                 105                 110
   Gln
<210> 83
   <211> 351
   <212> DNA
   <213> Artificial Sequence
<220>
   <223> C4.4a binder
<400> 83
   gaggtgcagc tgctggaatc cggcggaggc ctggtgcagc ctggcggatc tctgagactg 60
   tcctgcgccg ccagcggctt taccttctcc agcgcctgga tgtcctgggt ccgacaggcc 120
   cctggcaagg gactggaatg ggtgtcctac atctcctcct ccggctccac catctactac 180
   gccgactccg tgaagggccg gttcaccatc tcccgggaca actccaagaa caccctgtac 240
   ctgcagatga actccctgcg ggccgaggac accgccgtgt actactgcgc ccgagagggc 300
   ctgtgggcct tcgattattg gggccagggc accctggtca ccgtcagctc a 351
<210> 84
   <211> 339
   <212> DNA
   <213> Artificial Sequence
<220>
   <223> C4.4a binder
<400> 84
   cagtccgtgc tgacccagcc cccttctgtg tctggcgccc ctggccagag agtgaccatc       60
   tcttgcaccg gctcctccag caacatcggc gctggctacg tggtgcactg gtatcagcag      120
   ctgcccggca ccgcccccaa gctgctgatc tacgacaaca acaagcggcc ctccggcgtg      180
   cccgacagat tctccggctc caagtccggc acctccgcct ccctggccat ctccggcctg      240
   agatctgagg acgaggccga ctactactgc gccgcctacg acgactccct gtccggccct      300
   gtgttcggcg gaggcacaaa gttaaccgtg ctgggccag                             339
<210> 85
   <211> 9
   <212> PRT
   <213> Artificial Sequence
<220>
   <223> C4.4a binder
<400> 85
   Phe Ser Ser Ala Trp Met Ser Trp Val
   1               5
<210> 86
   <211> 20
   <212> PRT
   <213> Artificial Sequence
<220>
   <223> C4.4a binder
<400> 86
   Val Ser Tyr Ile Ser Ser Ser Gly Ser Ser Thr Tyr Tyr Ala Asp Ser
   1               5                   10                  15
   Val Lys Gly Arg
               20
<210> 87
   <211> 10
   <212> PRT
   <213> Artificial Sequence
<220>
   <223> C4.4a binder
<400> 87
   Ala Arg Glu Gly Leu Trp Ala Phe Asp Lys
   1               5                   10
<210> 88
   <211> 14
   <212> PRT
   <213> Artificial Sequence
<220>
   <223> C4.4a binder
<400> 88
   Ser Gly Ser Ser Ser Asn Ile Gly Ala Gly Tyr Val Val His
   1 5 10
<210> 89
   <211> 7
   <212> PRT
   <213> Artificial Sequence
<220>
   <223> C4.4a binder
<400> 89
   Asp Asn Asn Gln Arg Pro Ser
   1 5
<210> 90
   <211> 12
   <212> PRT
   <213> Artificial Sequence
<220>
   <223> C4.4a binder
<400> 90
   Cys Ala Ala Phe Asp Asp Arg Leu Ser Gly Pro Val
   1 5 10
<210> 91
   <211> 117
   <212> PRT
   <213> Artificial Sequence
<220>
   <223> C4.4a binder
<400> 91
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Lys Trp Gly Gln Gly Thr Leu
               100                 105                 110
   Val Thr Val Ser Ser
           115
<210> 92
   <211> 113
   <212> PRT
   <213> Artificial Sequence
<220>
   <223> C4.4a binder
<400> 92
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Gln Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Phe Asp Asp Arg
   85 90 95
   Leu Ser Gly Pro Val Phe Gly Gly Gly Thr Lys Leu Thr Val Leu Gly
   100 105 110
   Gln
<210> 93
   <211> 351
   <212> DNA
   <213> Artificial Sequence
<220>
   <223> C4.4a binder
<400> 93
   gaggtgcagc tgctggaaag cggcggaggc ctggtgcagc ctggcggaag cctgagactg 60
   agctgtgccg ccagcggctt caccttcagc agcgcctgga tgagctgggt ccgacaggcc 120
   cctggcaagg gcctggaatg ggtgtcctac atcagcagca gcggcagcag cacctactac 180
   gccgacagcg tgaagggccg gttcaccatc agccgggaca acagcaagaa caccctgtac 240
   ctgcagatga acagcctgcg ggccgaggac accgccgtgt actactgcgc cagagaaggc 300
   ctgtgggcct tcgataagtg gggccagggc accctggtca ccgtcagctc a 351
<210> 94
   <211> 339
   <212> DNA
   <213> Artificial Sequence
<220>
   <223> C4.4a binder
<400> 94
   cagagcgtgc tgacccagcc tcctagcgcc tctggcaccc ctggccagag agtgaccatc      60
   agctgcagcg gcagcagcag caacatcgga gccggctacg tggtgcactg gtatcagcag     120
   ctgcccggca ccgcccccaa gctgctgatc tacgacaaca accagcggcc cagcggcgtg     180
   cccgacagat tttccggcag caagagcggc accagcgcca gcctggccat cagcggcctg     240
   agaagcgagg acgaggccga ctactactgc gccgccttcg acgacagact gagcggccct     300
   gtgttcggcg gaggcacaaa gttaaccgtg ctgggccag                            339
<210> 95
   <211> 9
   <212> PRT
   <213> Artificial Sequence
<220>
   <223> C4.4a binder
<400> 95
   Phe Ser Asp Tyr Gln Met Thr Trp Ile
   1               5
<210> 96
   <211> 20
   <212> PRT
   <213> Artificial Sequence
<220>
   <223> C4.4a binder
<400> 96
   Val Ser Gly Val Ser Trp Asn Gly Ala Arg Thr His Tyr Ala Asp Ser
   1               5                   10                  15
   Val Lys Gly Arg
               20
<210> 97
   <211> 15
   <212> PRT
   <213> Artificial Sequence
<220>
   <223> C4.4a binder
<400> 97
   Ala Lys Gly Asp Tyr Leu Val Tyr Ser Ala Tyr Tyr Phe Asp Ser
   1               5                   10                  15
<210> 98
   <211> 13
   <212> PRT
   <213> Artificial Sequence
<220>
   <223> C4.4a binder
<400> 98
   Ser Gly Ser Ser Ser Asn Val Gly Ser Asn Pro Val Asn
   1               5                   10
<210> 99
   <211> 7
   <212> PRT
   <213> Artificial Sequence
<220>
   <223> C4.4a binder
<400> 99
   Arg Asn Asn Gln Arg Pro Ser
   1               5
<210> 100
   <211> 12
   <212> PRT
   <213> Artificial Sequence
<220>
   <223> C4.4a binder
<400> 100
   Cys Ala Ala Trp Asp Asp Arg Leu Asn Gly Trp Val
   1               5                   10
<210> 101
   <211> 122
   <212> PRT
   <213> Artificial Sequence
<220>
   <223> C4.4a binder
<400> 101
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Gln Met Thr Trp Ile Arg Gln Thr Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Ser Ala Tyr Tyr Phe Asp Ser Trp
               100                 105                 110
   Gly Gln Gly Thr Leu Val Thr Val Ser Ser
           115                 120
<210> 102
   <211> 112
   <212> PRT
   <213> Artificial Sequence
<220>
   <223> C4.4a binder
<400> 102
   Glu Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
   85 90 95
   Asn Gly Trp Gly Phe Gly Gly Gly Thr Lys Leu Thr Val Leu Gly Gln
   100 105 110
<210> 103
   <211> 366
   <212> DNA
   <213> Artificial Sequence
<220>
   <223> C4.4a binder
<400> 103
   gaggtgcagc tgctggaaag cggcggagga ctggtgcagc ctggaggcag cctgagactg 60
   tcttgcgccg ccagcggctt caccttcagc gactaccaga tgacctggat ccgacagacc 120
   cctggcaagg gcctggaatg ggtgtccggc atcagctgga acggaggcag cacccactac 180
   gccgacagcg tgaagggccg gttcaccatc agccgggaca acagcaagaa caccctgtac 240
   ctgcagatga acagcctgcg ggccgaggac accgccgtgt actactgcgc caagggcgac 300
   tacctggtgt acagcgccta ctacttcgac agctggggcc agggcaccct ggtcaccgtg 360
   tctagc 366
<210> 104
   <211> 336
   <212> DNA
   <213> Artificial Sequence
<220>
   <223> C4.4a binder
<400> 104
   gagagcgtgc tgacccagcc tcctagcgcc tctggcaccc ctggccagag agtgaccatc 60
   agctgctctg gcagcagcag caacatcgga agcaaccccg tgaactggta tcagcagctg 120
   cccggcaccg cccccaagct gctgatctac cggaacaacc agcggcctag cggcgtgccc 180
   gacagattca gcggcagcaa gagcggcacc agcgccagcc tggccatcag cggcctgaga 240
   agcgaggacg aggccgacta ctactgcgcc gcctgggacg acagactgaa cggctggggc 300
   ttcggcggag gcaccaagct gaccgtgctg ggacag 336
<210> 105
   <211> 9
   <212> PRT
   <213> Artificial Sequence
<220>
   <223> C4.4a binder
<400> 105
   Phe Ser Asp Tyr Gln Met Thr Trp Ile
   1 5
<210> 106
   <211> 20
   <212> PRT
   <213> Artificial Sequence
<220>
   <223> C4.4a binder
<400> 106
   Val Ser Gly Ile Ser Trp Asn Gly Gly Ser Thr His Tyr Ala Asp Ser
   1 5 10 15
   Val Lys Gly Arg
   20
<210> 107
   <211> 15
   <212> PRT
   <213> Artificial Sequence
<220>
   <223> C4.4a binder
<400> 107
   Ala Lys Gly Asp Tyr Leu Val Tyr Ser Ser Tyr Tyr Phe Lys Ser
   1 5 10 15
<210> 108
   <211> 13
   <212> PRT
   <213> Artificial Sequence
<220>
   <223> C4.4a binder
<400> 108
   Ser Gly Ser Ser Ser Asn Ile Gly Ser Asn Pro Val Asn
   1 5 10
<210> 109
   <211> 7
   <212> PRT
   <213> Artificial Sequence
<220>
   <223> C4.4a binder
<400> 109
   Arg Asn Asn Gln Arg Pro Ser
   1 5
<210> 110
   <211> 12
   <212> PRT
   <213> Artificial Sequence
<220>
   <223> C4.4a binder
<400> 110
   Cys Ala Ala Trp Asp Asp Arg Leu Ser Gly Trp Ala
   1 5 10
<210> 111
   <211> 122
   <212> PRT
   <213> Artificial Sequence
<220>
   <223> C4.4a binder
<400> 111
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asp Tyr
   20 25 30
   Gln Met Thr Trp Ile Arg Gln Thr Pro Gly Lys Gly Leu Glu Trp Val
   35 40 45
   Ser Gly Ile Ser Trp Asn Gly Gly Ser Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Asp Tyr Leu Val Tyr Ser Ser Tyr Tyr Phe Lys Ser Trp
   100 105 110
   Gly Gln Gly Thr Leu Val Thr Val Ser Ser
   115 120
<210> 112
   <211> 112
   <212> PRT
   <213> Artificial Sequence
<220>
   <223> C4.4a binder
<400> 112
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
                   85                  90                  95
   Ser Gly Trp Ala Phe Gly Gly Gly Thr Lys Leu Thr Val Leu Gly Gln
               100                 105                 110
<210> 113
   <211> 366
   <212> DNA
   <213> Artificial Sequence
<220>
   <223> C4.4a binder
<400> 113
   gaggtgcagc tgctggaatc cggcggaggc ctggtgcagc ctggcggatc tctgagactg 60
   tcctgcgccg cctccggctt caccttctcc gactaccaga tgacctggat cagacagacc 120
   cccggcaagg gcctggaatg ggtgtccggc atctcctgga acggcggctc cacccactac 180
   gccgactctg tgaagggccg gttcaccatc tcccgggaca actccaagaa caccctgtac 240
   ctgcagatga actccctgcg ggccgaggac accgccgtgt actactgcgc caagggcgac 300
   tacctggtgt actcctccta ctacttcaag tcctggggcc agggcaccct ggtcaccgtc 360
   agctca 366
<210> 114
   <211> 336
   <212> DNA
   <213> Artificial Sequence
<220>
   <223> C4.4a binder
<400> 114
   cagtccgtgc tgacccagcc tccttccgcc tctggcaccc ctggccagag agtgaccatc 60
   tcctgctccg gctcctcctc caacatcggc tccaaccccg tgaactggta tcagcagctg 120
   cccggcaccg cccccaagct gctgatctac cggaacaacc agcggccctc cggcgtgccc 180
   gacagattct ccggctccaa gtccggcacc tccgcctccc tggccatctc cggcctgaga 240
   tctgaggacg aggccgacta ctactgcgcc gcctgggacg accggctgtc tggctgggct 300
   tttggcggcg gaacaaagtt aaccgtgctg ggccag 336
<210> 115
   <211> 9
   <212> PRT
   <213> Artificial Sequence
<220>
   <223> C4.4a binder
<400> 115
   Phe Ser Asp Tyr Gln Met Thr Trp Ile
   1               5
<210> 116
   <211> 20
   <212> PRT
   <213> Artificial Sequence
<220>
   <223> C4.4a binder
<400> 116
   Val Ser Gly Ile Ser Trp Asn Gly Gly Ser Thr His Tyr Ala Asp Ser
   1 5 10 15
   Val Lys Gly Arg
   20
<210> 117
   <211> 15
   <212> PRT
   <213> Artificial Sequence
<220>
   <223> C4.4a binder
<400> 117
   Ala Lys Gly Asp Tyr Leu Val Tyr Lys Ser Tyr Tyr Phe Lys Ser
   1 5 10 15
<210> 118
   <211> 13
   <212> PRT
   <213> Artificial Sequence
<220>
   <223> C4.4a binder
<400> 118
   Ser Gly Ser Ser Ser Asn Ile Gly Ser Asn Pro Val Asn
   1 5 10
<210> 119
   <211> 7
   <212> PRT
   <213> Artificial Sequence
<220>
   <223> C4.4a binder
<400> 119
   Arg Asn Asn Gln Arg Pro Ser
   1 5
<210> 120
   <211> 12
   <212> PRT
   <213> Artificial Sequence
<220>
   <223> C4.4a binder
<400> 120
   Cys Ala Ala Trp Asp Asp Ser Leu Ser Gly Trp Ala
   1 5 10
<210> 121
   <211> 122
   <212> PRT
   <213> Artificial Sequence
<220>
   <223> C4.4a binder
<400> 121
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asp Tyr
   20 25 30
   Gln Met Thr Trp Ile Arg Gln Thr Pro Gly Lys Gly Leu Glu Trp Val
   35 40 45
   Ser Gly Ile Ser Trp Asn Gly Gly Ser Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Asp Tyr Leu Val Tyr Lys Ser Tyr Tyr Phe Lys Ser Trp
   100 105 110
   Gly Gln Gly Thr Leu Val Thr Val Ser Ser
   115 120
<210> 122
   <211> 112
   <212> PRT
   <213> Artificial Sequence
<220>
   <223> C4.4a binder
<400> 122
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Ser Leu
                   85                  90                  95
   Ser Gly Trp Ala Phe Gly Gly Gly Thr Lys Leu Thr Val Leu Gly Gln
               100                 105                 110
<210> 123
   <211> 366
   <212> DNA
   <213> Artificial Sequence
<220>
   <223> C4.4a binder
<400> 123
   gaggtgcagc tgctggaatc cggcggaggc ctggtgcagc ctggcggatc tctgagactg      60
   tcctgcgccg cctccggctt caccttctcc gactaccaga tgacctggat cagacagacc     120
   cccggcaagg gcctggaatg ggtgtccggc atctcctgga acggcggctc cacccactac     180
   gccgactctg tgaagggccg gttcaccatc tcccgggaca actccaagaa caccctgtac     240
   ctgcagatga actccctgcg ggccgaggac accgccgtgt actactgcgc caagggcgac     300
   tacctggtgt acaagtccta ctacttcaag tcctggggcc agggcaccct ggtcaccgtc     360
   agctca                                                                366
<210> 124
   <211> 336
   <212> DNA
   <213> Artificial Sequence
<220>
   <223> C4.4a binder
<400> 124
   cagtccgtgc tgacccagcc tccttccgcc tctggcaccc ctggccagag agtgaccatc      60
   tcctgctccg gctcctcctc caacatcggc tccaaccccg tgaactggta tcagcagctg     120
   cccggcaccg cccccaagct gctgatctac cggaacaacc agcggccctc cggcgtgccc     180
   gacagattct ccggctccaa gtccggcacc tccgcctccc tggccatctc cggcctgaga     240
   tctgaggacg aggccgacta ctactgcgcc gcctgggacg actccctgtc tggctgggct     300
   tttggcggcg gaacaaagtt aaccgtgctg ggccag                               336
<210> 125
   <211> 9
   <212> PRT
   <213> Artificial Sequence
<220>
   <223> C4.4a binder
<400> 125
   Phe Ser Asp Tyr Gln Met Thr Trp Ile
   1               5
<210> 126
   <211> 20
   <212> PRT
   <213> Artificial Sequence
<220>
   <223> C4.4a binder
<400> 126
   Val Ser Gly Ile Ser Trp Asn Gly Gly Ser Thr His Tyr Ala Asp Ser
   1               5                   10                  15
   Val Lys Gly Arg
               20
<210> 127
   <211> 15
   <212> PRT
   <213> Artificial Sequence
<220>
   <223> C4.4a binder
<400> 127
   Ala Lys Gly Asp Tyr Leu Val Tyr Ser Ser Tyr Tyr Phe Lys Ser
   1               5                   10                  15
<210> 128
   <211> 13
   <212> PRT
   <213> Artificial Sequence
<220>
   <223> C4.4a binder
<400> 128
   Ser Gly Ser Ser Ser Asn Ile Gly Ser Asn Pro Val Asn
   1               5                   10
<210> 129
   <211> 7
   <212> PRT
   <213> Artificial Sequence
<220>
   <223> C4.4a binder
<400> 129
   Arg Asn Asn Gln Arg Pro Ser
   1               5
<210> 130
   <211> 12
   <212> PRT
   <213> Artificial Sequence
<220>
   <223> C4.4a binder
<400> 130
   Cys Ala Ala Trp Asp Asp Arg Leu Ser Gly Trp Gly
   1               5                   10
<210> 131
   <211> 122
   <212> PRT
   <213> Artificial Sequence
<220>
   <223> C4.4a binder
<400> 131
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Gln Met Thr Trp Ile Arg Gln Thr Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Ser Ser Tyr Tyr Phe Lys Ser Trp
               100                 105                 110
   Gly Gln Gly Thr Leu Val Thr Val Ser Ser
           115                 120
<210> 132
   <211> 112
   <212> PRT
   <213> Artificial Sequence
<220>
   <223> C4.4a binder
<400> 132
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
                   85                  90                  95
   Ser Gly Trp Gly Phe Gly Gly Gly Thr Lys Leu Thr Val Leu Gly Gln
               100                 105                 110
<210> 133
   <211> 366
   <212> DNA
   <213> Artificial Sequence
<220>
   <223> C4.4a binder
<400> 133
   gaggtgcagc tgctggaatc cggcggaggc ctggtgcagc ctggcggatc tctgagactg 60
   tcctgcgccg cctccggctt caccttctcc gactaccaga tgacctggat cagacagacc 120
   cccggcaagg gcctggaatg ggtgtccggc atctcctgga acggcggctc cacccactac 180
   gccgactctg tgaagggccg gttcaccatc tcccgggaca actccaagaa caccctgtac 240
   ctgcagatga actccctgcg ggccgaggac accgccgtgt actactgcgc caagggcgac 300
   tacctggtgt actcctccta ctacttcaag tcctggggcc agggcaccct ggtcaccgtc 360
   agctca 366
<210> 134
   <211> 336
   <212> DNA
   <213> Artificial Sequence
<220>
   <223> C4.4a binder
<400> 134
   cagtccgtgc tgacccagcc tccttccgcc tctggcaccc ctggccagag agtgaccatc      60
   tcctgctccg gctcctcctc caacatcggc tccaaccccg tgaactggta tcagcagctg     120
   cccggcaccg cccccaagct gctgatctac cggaacaacc agcggccctc cggcgtgccc     180
   gacagattct ccggctccaa gtccggcacc tccgcctccc tggccatctc cggcctgaga     240
   tctgaggacg aggccgacta ctactgcgcc gcctgggacg accggctgtc tggctgggga     300
   tttggcggcg gaacaaagtt aaccgtgctg ggccag                               336
<210> 135
   <211> 9
   <212> PRT
   <213> Artificial Sequence
<220>
   <223> C4.4a binder
<400> 135
   Phe Ser Ser Tyr Gln Met Thr Trp Ile
   1               5
<210> 136
   <211> 20
   <212> PRT
   <213> Artificial Sequence
<220>
   <223> C4.4a binder
<400> 136
   Val Ser Gly Ile Ser Trp Asn Gly Gly Ser Thr His Tyr Ala Asp Ser
   1               5                   10                  15
   Val Lys Gly Arg
               20
<210> 137
   <211> 15
   <212> PRT
   <213> Artificial Sequence
<220>
   <223> C4.4a binder
<400> 137
   Ala Lys Gly Asp Tyr Leu Val Tyr Lys Ser Tyr Tyr Phe Lys Ser
   1               5                   10                  15
<210> 138
   <211> 13
   <212> PRT
   <213> Artificial Sequence
<220>
   <223> C4.4a binder
<400> 138
   Ser Gly Ser Ser Ser Asn Ile Gly Ser Asn Pro Val Asn
   1               5                   10
<210> 139
   <211> 7
   <212> PRT
   <213> Artificial Sequence
<220>
   <223> C4.4a binder
<400> 139
   Arg Asn Asn Gln Arg Pro Ser
   1               5
<210> 140
   <211> 12
   <212> PRT
   <213> Artificial Sequence
<220>
   <223> C4.4a binder
<400> 140
   Cys Ala Ala Trp Asp Asp Ser Leu Ser Gly Trp Ala
   1               5                   10
<210> 141
   <211> 122
   <212> PRT
   <213> Artificial Sequence
<220>
   <223> C4.4a binder
<400> 141
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gln Met Thr Trp Ile Arg Gln Ala Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Lys Ser Tyr Tyr Phe Lys Ser Trp
               100                 105                 110
   Gly Gln Gly Thr Leu Val Thr Val Ser Ser
           115                 120
<210> 142
   <211> 112
   <212> PRT
   <213> Artificial Sequence
<220>
   <223> C4.4a binder
<400> 142
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Ser Leu
                   85                  90                  95
   Ser Gly Trp Ala Phe Gly Gly Gly Thr Lys Leu Thr Val Leu Gly Gln
               100                 105                 110
<210> 143
   <211> 366
   <212> DNA
   <213> Artificial Sequence
<220>
   <223> C4.4a binder
<400> 143
   gaggtgcagc tgctggaaag cggcggaggc ctggtgcagc ctggcggaag cctgagactg       60
   agctgtgccg ccagcggctt caccttcagc agctaccaga tgacctggat cagacaggcc      120
   cctggcaagg gcctggaatg ggtgtccggc atcagctgga acggcggcag cacccactac      180
   gccgacagcg tgaagggccg gttcaccatc agccgggaca acagcaagaa caccctgtac      240
   ctgcagatga acagcctgcg ggccgaggac accgccgtgt actactgcgc caagggcgac      300
   tacctggtgt acaagagcta ctacttcaag agctggggcc agggcacact ggtcaccgtc      360
   agctca                                                                 366
<210> 144
   <211> 336
   <212> DNA
   <213> Artificial Sequence
<220>
   <223> C4.4a binder
<400> 144
   cagagcgtgc tgacccagcc tcctagcgcc tctggcaccc ctggccagag agtgaccatc 60
   agctgcagcg gcagcagcag caacatcggc agcaaccccg tgaactggta tcagcagctg 120
   cccggcaccg cccccaagct gctgatctac cggaacaacc agcggcccag cggcgtgccc 180
   gacagatttt ccggcagcaa gagcggcacc agcgccagcc tggccatcag cggcctgaga 240
   agcgaggacg aggccgacta ctactgcgcc gcctgggacg atagcctgag cggctgggcc 300
   tttggcggcg gaacaaagtt aaccgtgctg ggccag 336
<210> 145
   <211> 9
   <212> PRT
   <213> Artificial Sequence
<220>
   <223> C4.4a binder
<400> 145
   Phe Ser Asn Ala Trp Met Ser Trp Val
   1 5
<210> 146
   <211> 20
   <212> PRT
   <213> Artificial Sequence
<220>
   <223> C4.4a binder
<400> 146
   Val Ser Tyr Ile Ser Ser Ser Gly Ser Thr Ile Tyr Tyr Ala Asp Ser
   1 5 10 15
   Val Lys Gly Arg
   20
<210> 147
   <211> 10
   <212> PRT
   <213> Artificial Sequence
<220>
   <223> C4.4a binder
<400> 147
   Ala Arg Glu Gly Leu Trp Ala Phe Asp Lys
   1 5 10
<210> 148
   <211> 14
   <212> PRT
   <213> Artificial Sequence
<220>
   <223> C4.4a binder
<400> 148
   Thr Gly Ser Ser Ser Asn Ile Gly Ala Gly Tyr Val Val His
   1               5                   10
<210> 149
   <211> 7
   <212> PRT
   <213> Artificial Sequence
<220>
   <223> C4.4a binder
<400> 149
   Asp Asn Asn Lys Arg Pro Ser
   1               5
<210> 150
   <211> 12
   <212> PRT
   <213> Artificial Sequence
<220>
   <223> C4.4a binder
<400> 150
   Cys Ala Ala Tyr Asp Asp Ser Leu Lys Gly Pro Val
   1               5                   10
<210> 151
   <211> 117
   <212> PRT
   <213> Artificial Sequence
<220>
   <223> C4.4a binder
<400> 151
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Lys Trp Gly Gln Gly Thr Leu
               100                 105                 110
   Val Thr Val Thr Ser
           115
<210> 152
   <211> 113
   <212> PRT
   <213> Artificial Sequence
<220>
   <223> C4.4a binder
<400> 152
   Gln Ser Val Leu Thr Gln Pro Pro Ser Ala Ser Gly Thr Pro Gly Gln
   1 5 10 15
   Arg Val Thr Ile Ser Cys Thr Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Lys Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Tyr Asp Asp Ser
   85 90 95
   Leu Lys Gly Pro Val Phe Gly Gly Gly Thr Lys Leu Thr Val Leu Gly
   100 105 110
   Gln
<210> 153
   <211> 9
   <212> PRT
   <213> Artificial Sequence
<220>
   <223> C4.4a binder
<400> 153
   Phe Ser Asn Ala Trp Met Ser Trp Val
   1 5
<210> 154
   <211> 20
   <212> PRT
   <213> Artificial Sequence
<220>
   <223> C4.4a binder
<400> 154
   Val Ser Tyr Ile Ser Ser Ser Gly Ser Thr Ile Tyr Tyr Ala Asp Ser
   1 5 10 15
   Val Lys Gly Arg
   20
<210> 155
   <211> 10
   <212> PRT
   <213> Artificial Sequence
<220>
   <223> C4.4a binder
<400> 155
   Ala Arg Glu Gly Leu Trp Ala Phe Asp Lys
   1 5 10
<210> 156
   <211> 14
   <212> PRT
   <213> Artificial Sequence
<220>
   <223> C4.4a binder
<400> 156
   Thr Gly Ser Ser Ser Asn Ile Gly Ala Gly Tyr Val Val His
   1 5 10
<210> 157
   <211> 7
   <212> PRT
   <213> Artificial Sequence
<220>
   <223> C4.4a binder
<400> 157
   Asp Asn Asn Lys Arg Pro Ser
   1 5
<210> 158
   <211> 12
   <212> PRT
   <213> Artificial Sequence
<220>
   <223> C4.4a binder
<400> 158
   Cys Ala Ala Phe Asp Asp Ser Leu Asn Gly Pro Val
   1 5 10
<210> 159
   <211> 117
   <212> PRT
   <213> Artificial Sequence
<220>
   <223> C4.4a binder
<400> 159
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Lys Trp Gly Gln Gly Thr Leu
               100                 105                 110
   Val Thr Val Thr Ser
           115
<210> 160
   <211> 113
   <212> PRT
   <213> Artificial Sequence
<220>
   <223> C4.4a binder
<400> 160
   Gln Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Lys Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Phe Asp Asp Ser
   85 90 95
   Leu Asn Gly Pro Val Phe Gly Gly Gly Thr Lys Leu Thr Val Leu Gly
   100 105 110
   Gln
<210> 161
   <211> 9
   <212> PRT
   <213> Artificial Sequence
<220>
   <223> C4.4a binder
<400> 161
   Phe Ser Asn Ala Trp Met Ser Trp Val
   1 5
<210> 162
   <211> 20
   <212> PRT
   <213> Artificial Sequence
<220>
   <223> C4.4a binder
<400> 162
   Val Ser Tyr Ile Ser Ser Ser Gly Ser Thr Ile Tyr Tyr Ala Asp Ser
   1 5 10 15
   Val Lys Gly Arg
   20
<210> 163
   <211> 10
   <212> PRT
   <213> Artificial Sequence
<220>
   <223> C4.4a binder
<400> 163
   Ala Arg Glu Gly Leu Trp Ala Phe Asp Lys
   1               5                   10
<210> 164
   <211> 14
   <212> PRT
   <213> Artificial Sequence
<220>
   <223> C4.4a binder
<400> 164
   Thr Gly Ser Ser Ser Asn Ile Gly Ala Gly Tyr Val Val His
   1               5                   10
<210> 165
   <211> 7
   <212> PRT
   <213> Artificial Sequence
<220>
   <223> C4.4a binder
<400> 165
   Asp Asn Asn Lys Arg Pro Ser
   1               5
<210> 166
   <211> 12
   <212> PRT
   <213> Artificial Sequence
<220>
   <223> C4.4a binder
<400> 166
   Cys Ala Ala Tyr Asp Asp Ser Leu Ser Gly Pro Val
   1               5                   10
<210> 167
   <211> 117
   <212> PRT
   <213> Artificial Sequence
<220>
   <223> C4.4a binder
<400> 167
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Lys Trp Gly Gln Gly Thr Leu
               100                 105                 110
   Val Thr Val Thr Ser
           115
<210> 168
   <211> 113
   <212> PRT
   <213> Artificial Sequence
<220>
   <223> C4.4a binder
<400> 168
   Gln Ser Val Leu Thr Gln Pro Pro Ser Val Ser Gly Ala Pro Gly Gln
   1 5 10 15
   Arg Val Thr Ile Ser Cys Thr Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Lys Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Phe Asp Asp Ser
   85 90 95
   Leu Asn Gly Pro Val Phe Gly Gly Gly Thr Lys Leu Thr Val Leu Gly
   100 105 110
   Gln
<210> 169
   <211> 9
   <212> PRT
   <213> Artificial Sequence
<220>
   <223> C4.4a binder
<400> 169
   Phe Ser Ser Ala Trp Met Ser Trp Val
   1 5
<210> 170
   <211> 20
   <212> PRT
   <213> Artificial Sequence
<220>
   <223> C4.4a binder
<400> 170
   Val Ser Tyr Ile Ser Ser Ser Gly Ser Ser Thr Tyr Tyr Ala Asp Ser
   1 5 10 15
   Val Lys Gly Arg
   20
<210> 171
   <211> 10
   <212> PRT
   <213> Artificial Sequence
<220>
   <223> C4.4a binder
<400> 171
   Ala Arg Glu Gly Leu Trp Ala Phe Asp Trp
   1 5 10
<210> 172
   <211> 14
   <212> PRT
   <213> Artificial Sequence
<220>
   <223> C4.4a binder
<400> 172
   Ser Gly Ser Ser Ser Asn Ile Gly Ala Gly Tyr Val Val His
   1 5 10
<210> 173
   <211> 7
   <212> PRT
   <213> Artificial Sequence
<220>
   <223> C4.4a binder
<400> 173
   Asp Asn Asn Gln Arg Pro Ser
   1 5
<210> 174
   <211> 12
   <212> PRT
   <213> Artificial Sequence
<220>
   <223> C4.4a binder
<400> 174
   Cys Ala Ala Tyr Asp Asp Ser Leu Ser Gly Pro Val
   1 5 10
<210> 175
   <211> 117
   <212> PRT
   <213> Artificial Sequence
<220>
   <223> C4.4a binder
<400> 175
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Trp Trp Gly Gln Gly Thr Leu
               100                 105                 110
   Val Thr Val Thr Ser
           115
<210> 176
   <211> 113
   <212> PRT
   <213> Artificial Sequence
<220>
   <223> C4.4a binder
<400> 176
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Gln Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Tyr Asp Asp Ser
                   85                  90                  95
   Leu Ser Gly Pro Val Phe Gly Gly Gly Thr Lys Leu Thr Val Leu Gly
               100                 105                 110
   Gln
<210> 177
   <211> 9
   <212> PRT
   <213> Artificial Sequence
<220>
   <223> C4.4a binder
<400> 177
   Phe Ser Ser Ala Trp Met Ser Trp Val
   1               5
<210> 178
   <211> 20
   <212> PRT
   <213> Artificial Sequence
<220>
   <223> C4.4a binder
<400> 178
   Val Ser Tyr Ile Ser Ser Ser Gly Ser Ser Thr Tyr Tyr Ala Asp Ser
   1 5 10 15
   Val Lys Gly Arg
   20
<210> 179
   <211> 10
   <212> PRT
   <213> Artificial Sequence
<220>
   <223> C4.4a binder
<400> 179
   Ala Arg Glu Gly Leu Trp Ala Phe Asp Asn
   1 5 10
<210> 180
   <211> 14
   <212> PRT
   <213> Artificial Sequence
<220>
   <223> C4.4a binder
<400> 180
   Ser Gly Ser Ser Ser Asn Ile Gly Ala Gly Tyr Val Val His
   1 5 10
<210> 181
   <211> 7
   <212> PRT
   <213> Artificial Sequence
<220>
   <223> C4.4a binder
<400> 181
   Asp Asn Asn Gln Arg Pro Ser
   1 5
<210> 182
   <211> 12
   <212> PRT
   <213> Artificial Sequence
<220>
   <223> C4.4a binder
<400> 182
   Cys Ala Ala Tyr Asp Asp Ser Leu Asn Gly Pro Val
   1 5 10
<210> 183
   <211> 117
   <212> PRT
   <213> Artificial Sequence
<220>
   <223> C4.4a binder
<400> 183
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Tyr Trp Gly Gln Gly Thr Leu
               100                 105                 110
   Val Thr Val Thr Ser
           115
<210> 184
   <211> 113
   <212> PRT
   <213> Artificial Sequence
<220>
   <223> C4.4a binder
<400> 184
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Gln Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Tyr Asp Asp Ser
   85 90 95
   Leu Asn Gly Pro Val Phe Gly Gly Gly Thr Lys Leu Thr Val Leu Gly
   100 105 110
   Gln
<210> 185
   <211> 9
   <212> PRT
   <213> Artificial Sequence
<220>
   <223> C4.4a binder
<400> 185
   Phe Ser Ser Ala Trp Met Ser Trp Val
   1 5
<210> 186
   <211> 20
   <212> PRT
   <213> Artificial Sequence
<220>
   <223> C4.4a binder
<400> 186
   Val Ser Tyr Ile Ser Ser Ser Gly Ser Ser Thr Tyr Tyr Ala Asp Ser
   1 5 10 15
   Val Lys Gly Arg
   20
<210> 187
   <211> 10
   <212> PRT
   <213> Artificial Sequence
<220>
   <223> C4.4a binder
<400> 187
   Ala Arg Glu Gly Leu Trp Ala Phe Asp Tyr
   1               5                   10
<210> 188
   <211> 14
   <212> PRT
   <213> Artificial Sequence
<220>
   <223> C4.4a binder
<400> 188
   Ser Gly Ser Ser Ser Asn Ile Gly Ala Gly Tyr Val Val His
   1               5                   10
<210> 189
   <211> 7
   <212> PRT
   <213> Artificial Sequence
<220>
   <223> C4.4a binder
<400> 189
   Asp Asn Asn Gln Arg Pro Ser
   1               5
<210> 190
   <211> 12
   <212> PRT
   <213> Artificial Sequence
<220>
   <223> C4.4a binder
<400> 190
   Cys Ala Ala Trp Asp Asp Arg Leu Asn Gly Pro Val
   1               5                   10
<210> 191
   <211> 117
   <212> PRT
   <213> Artificial Sequence
<220>
   <223> C4.4a binder
<400> 191
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Tyr Trp Gly Gln Gly Thr Leu
               100                 105                 110
   Val Thr Val Ser Ser
           115
<210> 192
   <211> 113
   <212> PRT
   <213> Artificial Sequence
<220>
   <223> C4.4a binder
<400> 192
   Glu Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Gln Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Trp Asp Asp Arg
   85 90 95
   Leu Asn Gly Pro Val Phe Gly Gly Gly Thr Lys Leu Thr Val Leu Gly
   100 105 110
   Gln
<210> 193
   <211> 11
   <212> PRT
   <213> Artificial Sequence
<220>
   <223> C4.4a binder
<400> 193
   Phe Thr Phe Ser Asn Ala Trp Met Ser Trp Val
   1 5 10
<210> 194
   <211> 20
   <212> PRT
   <213> Artificial Sequence
<220>
   <223> C4.4a binder
<400> 194
   Val Ser Tyr Ile Ser Ser Ser Gly Ser Thr Ile Tyr Tyr Ala Asp Ser
   1 5 10 15
   Val Lys Gly Arg
   20
<210> 195
   <211> 10
   <212> PRT
   <213> Artificial Sequence
<220>
   <223> C4.4a binder
<400> 195
   Ala Arg Glu Gly Leu Trp Ala Phe Asp Tyr
   1 5 10
<210> 196
   <211> 14
   <212> PRT
   <213> Artificial Sequence
<220>
   <223> C4.4a binder
<400> 196
   Thr Gly Ser Ser Ser Asn Ile Gly Ala Gly Tyr Val Val His
   1 5 10
<210> 197
   <211> 7
   <212> PRT
   <213> Artificial Sequence
<220>
   <223> C4.4a binder
<400> 197
   Asp Asn Asn Lys Arg Pro Ser
   1 5
<210> 198
   <211> 12
   <212> PRT
   <213> Artificial Sequence
<220>
   <223> C4.4a binder
<400> 198
   Cys Ala Ala Tyr Asp Asp Arg Leu Asn Gly Pro Val
   1 5 10
<210> 199
   <211> 117
   <212> PRT
   <213> Artificial Sequence
<220>
   <223> C4.4a binder
<400> 199
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Tyr Trp Gly Gln Gly Thr Leu
               100                 105                 110
   Val Thr Val Ser Ser
           115
<210> 200
   <211> 113
   <212> PRT
   <213> Artificial Sequence
<220>
   <223> C4.4a binder
<400> 200
   Glu Ser Val Leu Thr Gln Pro Pro Ser Val Ser Gly Ala Pro Gly Gln
   1 5 10 15
   Arg Val Thr Ile Ser Cys Thr Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Lys Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Tyr Asp Asp Arg
   85 90 95
   Leu Asn Gly Pro Val Phe Gly Gly Gly Thr Lys Leu Thr Val Leu Gly
   100 105 110
   Gln
<210> 201
   <211> 11
   <212> PRT
   <213> Artificial Sequence
<220>
   <223> C4.4a binder
<400> 201
   Phe Thr Phe Ser Ser Ala Trp Met Ser Trp Val
   1 5 10
<210> 202
   <211> 20
   <212> PRT
   <213> Artificial Sequence
<220>
   <223> C4.4a binder
<400> 202
   Val Ser Tyr Ile Ser Ser Ser Gly Ser Ser Thr Tyr Tyr Ala Asp Ser
   1 5 10 15
   Val Lys Gly Arg
   20
<210> 203
   <211> 10
   <212> PRT
   <213> Artificial Sequence
<220>
   <223> C4.4a binder
<400> 203
   Ala Arg Glu Gly Leu Trp Ala Phe Asp Gly
   1 5 10
<210> 204
   <211> 14
   <212> PRT
   <213> Artificial Sequence
<220>
   <223> C4.4a binder
<400> 204
   Ser Gly Ser Ser Ser Asn Ile Gly Ala Gly Tyr Val Val His
   1 5 10
<210> 205
   <211> 7
   <212> PRT
   <213> Artificial Sequence
<220>
   <223> C4.4a binder
<400> 205
   Asp Asn Asn Gln Arg Pro Ser
   1 5
<210> 206
   <211> 12
   <212> PRT
   <213> Artificial Sequence
<220>
   <223> C4.4a binder
<400> 206
   Cys Ala Ala Tyr Asp Asp Ser Leu Asn Arg Pro Val
   1 5 10
<210> 207
   <211> 117
   <212> PRT
   <213> Artificial Sequence
<220>
   <223> C4.4a binder
<400> 207
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Gly Trp Gly Gln Gly Thr Leu
               100                 105                 110
   Val Thr Val Ser Ser
           115
<210> 208
   <211> 113
   <212> PRT
   <213> Artificial Sequence
<220>
   <223> C4.4a binder
<400> 208
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Gln Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Tyr Asp Asp Ser
   85 90 95
   Leu Asn Arg Pro Val Phe Gly Gly Gly Thr Lys Leu Thr Val Leu Gly
   100 105 110
   Gln
<210> 209
   <211> 11
   <212> PRT
   <213> Artificial Sequence
<220>
   <223> C4.4a binder
<400> 209
   Phe Thr Phe Ser Asn Ala Trp Met Ser Trp Val
   1 5 10
<210> 210
   <211> 20
   <212> PRT
   <213> Artificial Sequence
<220>
   <223> C4.4a binder
<400> 210
   Val Ser Tyr Ile Ser Ser Ser Gly Ser Thr Ile Tyr Tyr Ala Asp Ser
   1 5 10 15
   Val Lys Gly Arg
   20
<210> 211
   <211> 10
   <212> PRT
   <213> Artificial Sequence
<220>
   <223> C4.4a binder
<400> 211
   Ala Arg Glu Gly Leu Trp Ala Phe Asp Tyr
   1               5                   10
<210> 212
   <211> 14
   <212> PRT
   <213> Artificial Sequence
<220>
   <223> C4.4a binder
<400> 212
   Thr Gly Ser Ser Ser Asn Ile Gly Ala Gly Tyr Val Val His
   1               5                   10
<210> 213
   <211> 7
   <212> PRT
   <213> Artificial Sequence
<220>
   <223> C4.4a binder
<400> 213
   Asp Asn Asn Lys Arg Pro Ser
   1               5
<210> 214
   <211> 12
   <212> PRT
   <213> Artificial Sequence
<220>
   <223> C4.4a binder
<400> 214
   Cys Ala Ala Phe Asp Asp Ser Leu Asn Gly Pro Val
   1               5                   10
<210> 215
   <211> 117
   <212> PRT
   <213> Artificial Sequence
<220>
   <223> C4.4a binder
<400> 215
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Tyr Trp Gly Gln Gly Thr Leu
               100                 105                 110
   Val Thr Val Ser Ser
           115
<210> 216
   <211> 113
   <212> PRT
   <213> Artificial Sequence
<220>
   <223> C4.4a binder
<400> 216
   Gln Ser Val Leu Thr Gln Pro Pro Ser Val Ser Gly Ala Pro Gly Gln
   1 5 10 15
   Arg Val Thr Ile Ser Cys Thr Gly Ser Ser Ser Asn Ile Gly Ala Gly
   20 25 30
   Tyr Val Val His Trp Tyr Gln Gln Leu Pro Gly Thr Ala Pro Lys Leu
   35 40 45
   Leu Ile Tyr Asp Asn Asn Lys Arg Pro Ser Gly Val Pro Asp Arg Phe
   50 55 60
   Ser Gly Ser Lys Ser Gly Thr Ser Ala Ser Leu Ala Ile Ser Gly Leu
   65 70 75 80
   Arg Ser Glu Asp Glu Ala Asp Tyr Tyr Cys Ala Ala Phe Asp Asp Ser
   85 90 95
   Leu Asn Gly Pro Val Phe Gly Gly Gly Thr Lys Leu Thr Val Leu Gly
   100 105 110
   Gln
<210> 217
   <211> 9
   <212> PRT
   <213> Artificial Sequence
<220>
   <223> C4.4a binder
<400> 217
   Phe Ser Ser Ala Trp Met Ser Trp Val
   1 5
<210> 218
   <211> 20
   <212> PRT
   <213> Artificial Sequence
<220>
   <223> C4.4a binder
<400> 218
   Val Ser Tyr Ile Ser Ser Ser Gly Ser Ser Thr Tyr Tyr Ala Asp Ser
   1 5 10 15
   Val Lys Gly Arg
   20
<210> 219
   <211> 10
   <212> PRT
   <213> Artificial Sequence
<220>
   <223> C4.4a binder
<400> 219
   Ala Arg Glu Gly Leu Trp Ala Phe Asp Lys
   1 5 10
<210> 220
   <211> 14
   <212> PRT
   <213> Artificial Sequence
<220>
   <223> C4.4a binder
<400> 220
   Thr Gly Ser Ser Ser Asn Ile Gly Ala Gly Tyr Val Val His
   1 5 10
<210> 221
   <211> 7
   <212> PRT
   <213> Artificial Sequence
<220>
   <223> C4.4a binder
<400> 221
   Asp Asn Asn Lys Arg Pro Ser
   1 5
<210> 222
   <211> 12
   <212> PRT
   <213> Artificial Sequence
<220>
   <223> C4.4a binder
<400> 222
   Cys Ala Ala Phe Asp Asp Ser Leu Asn Gly Pro Val
   1 5 10
<210> 223
   <211> 117
   <212> PRT
   <213> Artificial Sequence
<220>
   <223> C4.4a binder
<400> 223
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Lys Trp Gly Gln Gly Thr Leu
               100                 105                 110
   Val Thr Val Thr Ser
           115
<210> 224
   <211> 113
   <212> PRT
   <213> Artificial Sequence
<220>
   <223> C4.4a binder
<400> 224
   Gln Ser Val Leu Thr Gln Pro Pro Ser Ala Ser Gly Thr Pro Gly Gln
   1               5                   10                  15
   Arg Val Thr Ile Ser Cys Thr Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Lys Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Phe Asp Asp Ser
                   85                  90                  95
   Leu Asn Gly Pro Val Phe Gly Gly Gly Thr Lys Leu Thr Val Leu Gly
               100                 105                 110
   Gln
<210> 225
   <211> 11
   <212> PRT
   <213> Artificial Sequence
<220>
   <223> C4.4a binder
<400> 225
   Phe Thr Phe Ser Ser Ala Trp Met Ser Trp Val
   1               5                   10
<210> 226
   <211> 20
   <212> PRT
   <213> Artificial Sequence
<220>
   <223> C4.4a binder
<400> 226
   Val Ser Tyr Ile Ser Ser Ser Gly Ser Ser Thr Tyr Tyr Ala Asp Ser
   1               5                   10                  15
   Val Lys Gly Arg
               20
<210> 227
   <211> 10
   <212> PRT
   <213> Artificial Sequence
<220>
   <223> C4.4a binder
<400> 227
   Ala Arg Glu Gly Leu Trp Ala Phe Asp Lys
   1 5 10
<210> 228
   <211> 14
   <212> PRT
   <213> Artificial Sequence
<220>
   <223> C4.4a binder
<400> 228
   Ser Gly Ser Ser Ser Asn Ile Gly Ala Gly Tyr Val Val His
   1 5 10
<210> 229
   <211> 7
   <212> PRT
   <213> Artificial Sequence
<220>
   <223> C4.4a binder
<400> 229
   Asp Asn Asn Gln Arg Pro Ser
   1 5
<210> 230
   <211> 12
   <212> PRT
   <213> Artificial Sequence
<220>
   <223> C4.4a binder
<400> 230
   Cys Ala Ala Phe Asp Asp Ser Leu Asn Gly Pro Val
   1 5 10
<210> 231
   <211> 117
   <212> PRT
   <213> Artificial Sequence
<220>
   <223> C4.4a binder
<400> 231
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ala
               20                  25                  30
   Trp Met Ser Trp Val Arg Gln Ala Pro Gly Lys Gly Leu Glu Trp Val
           35                  40                  45
   Ser Tyr Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Leu Trp Ala Phe Asp Lys Trp Gly Gln Gly Thr Leu
               100                 105                 110
   Val Thr Val Ser Ser
           115
<210> 232
   <211> 113
   <212> PRT
   <213> Artificial Sequence
<220>
   <223> C4.4a binder
<400> 232
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Gln Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Phe Asp Asp Ser
                   85                  90                  95
   Leu Asn Gly Pro Val Phe Gly Gly Gly Thr Lys Leu Thr Val Leu Gly
               100                 105                 110
   Gln
<210> 233
   <211> 11
   <212> PRT
   <213> Artificial Sequence
<220>
   <223> C4.4a binder
<400> 233
   Phe Thr Phe Ser Ser Ala Trp Met Ser Trp Val
   1               5                   10
<210> 234
   <211> 20
   <212> PRT
   <213> Artificial Sequence
<220>
   <223> C4.4a binder
<400> 234
   Val Ser Tyr Ile Ser Ser Ser Gly Ser Ser Thr Tyr Tyr Ala Asp Ser
   1               5                   10                  15
   Val Lys Gly Arg
               20
<210> 235
   <211> 10
   <212> PRT
   <213> Artificial Sequence
<220>
   <223> C4.4a binder
<400> 235
   Ala Arg Glu Gly Leu Trp Ala Phe Asp Lys
   1               5                   10
<210> 236
   <211> 14
   <212> PRT
   <213> Artificial Sequence
<220>
   <223> C4.4a binder
<400> 236
   Ser Gly Ser Ser Ser Asn Ile Gly Ala Gly Tyr Val Val His
   1               5                   10
<210> 237
   <211> 7
   <212> PRT
   <213> Artificial Sequence
<220>
   <223> C4.4a binder
<400> 237
   Asp Asn Asn Gln Arg Pro Ser
   1               5
<210> 238
   <211> 12
   <212> PRT
   <213> Artificial Sequence
<220>
   <223> C4.4a binder
<400> 238
   Cys Ala Ala Phe Asp Asp Arg Leu Ser Gly Pro Val
   1               5                   10
<210> 239
   <211> 117
   <212> PRT
   <213> Artificial Sequence
<220>
   <223> C4.4a binder
<400> 239
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Ala
   20 25 30
   Trp Met Ser Trp Val Arg Gln Ala Pro Gly Lys Gly Leu Glu Trp Val
   35 40 45
   Ser Tyr Ile Ser Ser Ser Gly Ser Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Glu Gly Leu Trp Ala Phe Asp Lys Trp Gly Gln Gly Thr Leu
   100 105 110
   Val Thr Val Ser Ser
   115
<210> 240
   <211> 113
   <212> PRT
   <213> Artificial Sequence
<220>
   <223> C4.4a binder
<400> 240
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ala Gly
               20                  25                  30
   Tyr Val Val His Trp Tyr Gln Gln Leu Pro Gly Thr Ala Pro Lys Leu
           35                  40                  45
   Leu Ile Tyr Asp Asn Asn Gln Arg Pro Ser Gly Val Pro Asp Arg Phe
       50                  55                  60
   Ser Gly Ser Lys Ser Gly Thr Ser Ala Ser Leu Ala Ile Ser Gly Leu
   65                  70                  75                  80
   Arg Ser Glu Asp Glu Ala Asp Tyr Tyr Cys Ala Ala Phe Asp Asp Arg
                   85                  90                  95
   Leu Ser Gly Pro Val Phe Gly Gly Gly Thr Lys Leu Thr Val Leu Gly
               100                 105                 110
   Gln
<210> 241
   <211> 9
   <212> PRT
   <213> Artificial Sequence
<220>
   <223> C4.4a binder
<400> 241
   Phe Ser Asp Tyr Gln Met Thr Trp Ile
   1               5
<210> 242
   <211> 20
   <212> PRT
   <213> Artificial Sequence
<220>
   <223> C4.4a binder
<400> 242
   Val Ser Gly Val Ser Trp Asn Gly Ala Arg Thr His Tyr Ala Asp Ser
   1               5                   10                  15
   Val Lys Gly Arg
               20
<210> 243
   <211> 15
   <212> PRT
   <213> Artificial Sequence
<220>
   <223> C4.4a binder
<400> 243
   Ala Lys Gly Asp Tyr Leu Val Tyr Ser Ser Tyr Tyr Phe Lys Ser
   1 5 10 15
<210> 244
   <211> 13
   <212> PRT
   <213> Artificial Sequence
<220>
   <223> C4.4a binder
<400> 244
   Ser Gly Ser Ser Ser Asn Val Gly Ser Asn Pro Val Asn
   1 5 10
<210> 245
   <211> 7
   <212> PRT
   <213> Artificial Sequence
<220>
   <223> C4.4a binder
<400> 245
   Arg Asn Asn Gln Arg Pro Ser
   1 5
<210> 246
   <211> 13
   <212> PRT
   <213> Artificial Sequence
<220>
   <223> C4.4a binder
<400> 246
   Cys Ala Ala Trp Asp Asp Arg Leu Asn Gly Trp Thr Gly
   1 5 10
<210> 247
   <211> 122
   <212> PRT
   <213> Artificial Sequence
<220>
   <223> C4.4a binder
<400> 247
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Gln Met Thr Trp Ile Arg Gln Thr Pro Gly Lys Gly Leu Glu Trp Val
           35                  40                  45
   Ser Gly Val Ser Trp Asn Gly Ala Arg Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Ser Ser Tyr Tyr Phe Lys Ser Trp
               100                 105                 110
   Gly Gln Gly Thr Leu Val Thr Val Thr Ser
           115                 120
<210> 248
   <211> 112
   <212> PRT
   <213> Artificial Sequence
<220>
   <223> C4.4a binder
<400> 248
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Val Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
                   85                  90                  95
   Asn Gly Trp Gly Phe Gly Gly Gly Thr Lys Leu Thr Val Leu Gly Gln
               100                 105                 110
<210> 249
   <211> 11
   <212> PRT
   <213> Artificial Sequence
<220>
   <223> C4.4a binder
<400> 249
   Phe Thr Phe Ser Ser Tyr Gln Met Thr Trp Ile
   1               5                   10
<210> 250
   <211> 20
   <212> PRT
   <213> Artificial Sequence
<220>
   <223> C4.4a binder
<400> 250
   Val Ser Gly Ile Ser Trp Asn Gly Gly Ser Thr His Tyr Ala Asp Ser
   1               5                   10                  15
   Val Lys Gly Arg
               20
<210> 251
   <211> 15
   <212> PRT
   <213> Artificial Sequence
<220>
   <223> C4.4a binder
<400> 251
   Ala Lys Gly Asp Tyr Leu Val Tyr Ser Ala Tyr Tyr Phe Asp Ser
   1 5 10 15
<210> 252
   <211> 13
   <212> PRT
   <213> Artificial Sequence
<220>
   <223> C4.4a binder
<400> 252
   Ser Gly Ser Ser Ser Asn Ile Gly Ser Asn Pro Val Asn
   1 5 10
<210> 253
   <211> 7
   <212> PRT
   <213> Artificial Sequence
<220>
   <223> C4.4a binder
<400> 253
   Arg Asn Asn Gln Arg Pro Ser
   1 5
<210> 254
   <211> 12
   <212> PRT
   <213> Artificial Sequence
<220>
   <223> C4.4a binder
<400> 254
   Cys Ala Ala Trp Asp Asp Arg Leu Asn Gly Trp Gly
   1 5 10
<210> 255
   <211> 122
   <212> PRT
   <213> Artificial Sequence
<220>
   <223> C4.4a binder
<400> 255
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gln Met Thr Trp Ile Arg Gln Thr Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Ser Ala Tyr Tyr Phe Asp Ser Trp
               100                 105                 110
   Gly Gln Gly Thr Leu Val Thr Val Ser Ser
           115                 120
<210> 256
   <211> 112
   <212> PRT
   <213> Artificial Sequence
<220>
   <223> C4.4a binder
<400> 256
   Glu Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
                   85                  90                  95
   Asn Gly Trp Gly Phe Gly Gly Gly Thr Lys Leu Thr Val Leu Gly Gln
               100                 105                 110
<210> 257
   <211> 11
   <212> PRT
   <213> Artificial Sequence
<220>
   <223> C4.4a binder
<400> 257
   Phe Thr Phe Ser Asp Tyr Gln Met Thr Trp Ile
   1               5                   10
<210> 258
   <211> 20
   <212> PRT
   <213> Artificial Sequence
<220>
   <223> C4.4a binder
<400> 258
   Val Ser Gly Ile Ser Trp Asn Gly Gly Ser Thr His Tyr Ala Asp Ser
   1               5                   10                  15
   Val Lys Gly Arg
               20
<210> 259
   <211> 15
   <212> PRT
   <213> Artificial Sequence
<220>
   <223> C4.4a binder
<400> 259
   Ala Lys Gly Asp Tyr Leu Val Tyr Ser Ser Tyr Tyr Phe Lys Tyr
   1               5                   10                  15
<210> 260
   <211> 13
   <212> PRT
   <213> Artificial Sequence
<220>
   <223> C4.4a binder
<400> 260
   Ser Gly Ser Ser Ser Asn Ile Gly Ser Asn Pro Val Asn
   1               5                   10
<210> 261
   <211> 7
   <212> PRT
   <213> Artificial Sequence
<220>
   <223> C4.4a binder
<400> 261
   Arg Asn Asn Gln Arg Pro Ser
   1               5
<210> 262
   <211> 12
   <212> PRT
   <213> Artificial Sequence
<220>
   <223> C4.4a binder
<400> 262
   Cys Ala Ala Trp Asp Asp Arg Leu Asn Gly Trp Ala
   1               5                   10
<210> 263
   <211> 122
   <212> PRT
   <213> Artificial Sequence
<220>
   <223> C4.4a binder
<400> 263
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Gln Met Thr Trp Ile Arg Gln Thr Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Ser Ser Tyr Tyr Phe Lys Tyr Trp
               100                 105                 110
   Gly Gln Gly Thr Leu Val Thr Val Ser Ser
           115                 120
<210> 264
   <211> 112
   <212> PRT
   <213> Artificial Sequence
<220>
   <223> C4.4a binder
<400> 264
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
                   85                  90                  95
   Asn Gly Trp Ala Phe Gly Gly Gly Thr Lys Leu Thr Val Leu Gly Gln
               100                 105                 110
<210> 265
   <211> 11
   <212> PRT
   <213> Artificial Sequence
<220>
   <223> C4.4a binder
<400> 265
   Phe Thr Phe Ser Asp Tyr Gln Met Thr Trp Ile
   1               5                   10
<210> 266
   <211> 20
   <212> PRT
   <213> Artificial Sequence
<220>
   <223> C4.4a binder
<400> 266
   Val Ser Gly Ile Ser Trp Asn Gly Gly Ser Thr His Tyr Ala Asp Ser
   1               5                   10                  15
   Val Lys Gly Arg
               20
<210> 267
   <211> 15
   <212> PRT
   <213> Artificial Sequence
<220>
   <223> C4.4a binder
<400> 267
   Ala Lys Gly Asp Tyr Leu Val Tyr Ser Ser Tyr Tyr Phe Lys Ser
   1               5                   10                  15
<210> 268
   <211> 13
   <212> PRT
   <213> Artificial Sequence
<220>
   <223> C4.4a binder
<400> 268
   Ser Gly Ser Ser Ser Asn Ile Gly Ser Asn Pro Val Asn
   1               5                   10
<210> 269
   <211> 7
   <212> PRT
   <213> Artificial Sequence
<220>
   <223> C4.4a binder
<400> 269
   Arg Asn Asn Gln Arg Pro Ser
   1               5
<210> 270
   <211> 12
   <212> PRT
   <213> Artificial Sequence
<220>
   <223> C4.4a binder
<400> 270
   Cys Ala Ala Trp Asp Asp Ser Leu Asn Gly Trp Gly
   1               5                   10
<210> 271
   <211> 122
   <212> PRT
   <213> Artificial Sequence
<220>
   <223> C4.4a binder
<400> 271
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Gln Met Thr Trp Ile Arg Gln Thr Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Ser Ser Tyr Tyr Phe Lys Ser Trp
               100                 105                 110
   Gly Gln Gly Thr Leu Val Thr Val Ser Ser
           115                 120
<210> 272
   <211> 112
   <212> PRT
   <213> Artificial Sequence
<220>
   <223> C4.4a binder
<400> 272
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Pro Val Asn Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Asn Gly Trp Gly Phe Gly Gly Gly Thr Lys Leu Thr Val Leu Gly Gln
   100 105 110
<210> 273
   <211> 11
   <212> PRT
   <213> Artificial Sequence
<220>
   <223> C4.4a binder
<400> 273
   Phe Thr Phe Ser Asp Tyr Gln Met Thr Trp Ile
   1 5 10
<210> 274
   <211> 20
   <212> PRT
   <213> Artificial Sequence
<220>
   <223> C4.4a binder
<400> 274
   Val Ser Gly Ile Ser Trp Asn Gly Gly Ser Thr His Tyr Ala Asp Ser
   1 5 10 15
   Val Lys Gly Arg
   20
<210> 275
   <211> 15
   <212> PRT
   <213> Artificial Sequence
<220>
   <223> C4.4a binder
<400> 275
   Ala Lys Gly Asp Tyr Leu Val Tyr Lys Ser Tyr Tyr Phe Lys Ser
   1               5                   10                  15
<210> 276
   <211> 13
   <212> PRT
   <213> Artificial Sequence
<220>
   <223> C4.4a binder
<400> 276
   Ser Gly Ser Ser Ser Asn Ile Gly Ser Asn Pro Val Asn
   1               5                   10
<210> 277
   <211> 7
   <212> PRT
   <213> Artificial Sequence
<220>
   <223> C4.4a binder
<400> 277
   Arg Asn Asn Gln Arg Pro Ser
   1               5
<210> 278
   <211> 12
   <212> PRT
   <213> Artificial Sequence
<220>
   <223> C4.4a binder
<400> 278
   Cys Ala Ala Trp Asp Asp Arg Leu Ser Gly Trp Gly
   1               5                   10
<210> 279
   <211> 122
   <212> PRT
   <213> Artificial Sequence
<220>
   <223> C4.4a binder
<400> 279
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Gln Met Thr Trp Ile Arg Gln Thr Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Lys Ser Tyr Tyr Phe Lys Ser Trp
               100                 105                 110
   Gly Gln Gly Thr Leu Val Thr Val Ser Ser
           115                 120
<210> 280
   <211> 112
   <212> PRT
   <213> Artificial Sequence
<220>
   <223> C4.4a binder
<400> 280
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
           35                  40                  45
   Ile Tyr Arg Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Ala Ala Trp Asp Asp Arg Leu
                   85                  90                  95
   Ser Gly Trp Gly Phe Gly Gly Gly Thr Lys Leu Thr Val Leu Gly Gln
               100                 105                 110
<210> 281
   <211> 11
   <212> PRT
   <213> Artificial Sequence
<220>
   <223> C4.4a binder
<400> 281
   Phe Thr Phe Ser Asp Tyr Gln Met Thr Trp Ile
   1               5                   10
<210> 282
   <211> 20
   <212> PRT
   <213> Artificial Sequence
<220>
   <223> C4.4a binder
<400> 282
   Val Ser Gly Ile Ser Trp Asn Gly Gly Ser Thr His Tyr Ala Asp Ser
   1               5                   10                  15
   Val Lys Gly Arg
               20
<210> 283
   <211> 15
   <212> PRT
   <213> Artificial Sequence
<220>
   <223> C4.4a binder
<400> 283
   Ala Lys Gly Asp Tyr Leu Val Tyr Ser Ser Tyr Tyr Phe Lys Ser
   1 5 10 15
<210> 284
   <211> 13
   <212> PRT
   <213> Artificial Sequence
<220>
   <223> C4.4a binder
<400> 284
   Ser Gly Ser Ser Ser Asn Ile Gly Ser Asn Pro Val Asn
   1 5 10
<210> 285
   <211> 7
   <212> PRT
   <213> Artificial Sequence
<220>
   <223> C4.4a binder
<400> 285
   Arg Asn Asn Gln Arg Pro Ser
   1 5
<210> 286
   <211> 12
   <212> PRT
   <213> Artificial Sequence
<220>
   <223> C4.4a binder
<400> 286
   Cys Ala Ala Trp Asp Asp Arg Leu Ser Gly Trp Gly
   1 5 10
<210> 287
   <211> 122
   <212> PRT
   <213> Artificial Sequence
<220>
   <223> C4.4a binder
<400> 287
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Tyr
               20                  25                  30
   Gln Met Thr Trp Ile Arg Gln Thr Pro Gly Lys Gly Leu Glu Trp Val
           35                  40                  45
   Ser Gly Ile Ser Trp Asn Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Asp Tyr Leu Val Tyr Ser Ser Tyr Tyr Phe Lys Ser Trp
               100                 105                 110
   Gly Gln Gly Thr Leu Val Thr Val Ser Ser
           115                 120
<210> 288
   <211> 112
   <212> PRT
   <213> Artificial Sequence
<220>
   <223> C4.4a binder
<400> 288
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Arg Leu
   85 90 95
   Ser Gly Trp Gly Phe Gly Gly Gly Thr Lys Leu Thr Val Leu Gly Gln
   100 105 110
<210> 289
   <211> 11
   <212> PRT
   <213> Artificial Sequence
<220>
   <223> C4.4a binder
<400> 289
   Phe Thr Phe Ser Asp Tyr Gln Met Thr Trp Ile
   1 5 10
<210> 290
   <211> 20
   <212> PRT
   <213> Artificial Sequence
<220>
   <223> C4.4a binder
<400> 290
   Val Ser Gly Ile Ser Trp Asn Gly Gly Ser Thr His Tyr Ala Asp Ser
   1 5 10 15
   Val Lys Gly Arg
   20
<210> 291
   <211> 15
   <212> PRT
   <213> Artificial Sequence
<220>
   <223> C4.4a binder
<400> 291
   Ala Lys Gly Asp Tyr Leu Val Tyr Lys Ser Tyr Tyr Phe Lys Ser
   1 5 10 15
<210> 292
   <211> 13
   <212> PRT
   <213> Artificial Sequence
<220>
   <223> C4.4a binder
<400> 292
   Ser Gly Ser Ser Ser Asn Ile Gly Ser Asn Pro Val Asn
   1 5 10
<210> 293
   <211> 7
   <212> PRT
   <213> Artificial Sequence
<220>
   <223> C4.4a binder
<400> 293
   Arg Asn Asn Gln Arg Pro Ser
   1 5
<210> 294
   <211> 12
   <212> PRT
   <213> Artificial Sequence
<220>
   <223> C4.4a binder
<400> 294
   Cys Ala Ala Trp Asp Asp Ser Leu Asn Gly Trp Gly
   1 5 10
<210> 295
   <211> 122
   <212> PRT
   <213> Artificial Sequence
<220>
   <223> C4.4a binder
<400> 295
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asp Tyr
   20 25 30
   Gln Met Thr Trp Ile Arg Gln Thr Pro Gly Lys Gly Leu Glu Trp Val
   35 40 45
   Ser Gly Ile Ser Trp Asn Gly Gly Ser Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Asp Tyr Leu Val Tyr Lys Ser Tyr Tyr Phe Lys Ser Trp
   100 105 110
   Gly Gln Gly Thr Leu Val Thr Val Ser Ser
   115 120
<210> 296
   <211> 112
   <212> PRT
   <213> Artificial Sequence
<220>
   <223> C4.4a binder
<400> 296
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Pro Val Asn Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Asn Gly Trp Gly Phe Gly Gly Gly Thr Lys Leu Thr Val Leu Gly Gln
   100 105 110
<210> 297
   <211> 9
   <212> PRT
   <213> Artificial Sequence
<220>
   <223> consensus sequence for M20 D02 S-A derived CDR H1
<220>
   <221> VARIANT
   <222> (3)..(3)
   <223> X = D or S
<400> 297
   Phe Ser Xaa Tyr Gln Met Thr Trp Ile
   1 5
<210> 298
   <211> 20
   <212> PRT
   <213> Artificial Sequence
<220>
   <223> consensus sequence for M20 D02 S-A derived CDRH2
<220>
   <221> VARIANT
   <222> (4)..(4)
   <223> X = V or I
<220>
   <221> VARIANT
   <222> (9) .. (9)
   <223> X = A or G
<220>
   <221> VARIANT
   <222> (10)..(10)
   <223> X = R or S
<400> 298
   Val Ser Gly Xaa Ser Trp Asn Gly Xaa Xaa Thr His Tyr Ala Asp Ser
   1 5 10 15
   Val Lys Gly Arg
   20
<210> 299
   <211> 15
   <212> PRT
   <213> Artificial Sequence
<220>
   <223> consensus for M20 D02 S-A derived CDRH3
<220>
   <221> VARIANT
   <222> (9) .. (9)
   <223> X = S, K, or R
<220>
   <221> VARIANT
   <222> (10)..(10)
   <223> X = A, S or R
<220>
   <221> VARIANT
   <222> (14)..(14)
   <223> X = D, K, E or R
<220>
   <221> VARIANT
   <222> (15)..(15)
   <223> X = S or Y
<400> 299
   Ala Lys Gly Asp Tyr Leu Val Tyr Xaa Xaa Tyr Tyr Phe Xaa Xaa
   1 5 10 15
<210> 300
   <211> 13
   <212> PRT
   <213> Artificial Sequence
<220>
   <223> consensus seqeunce derived from M20 D02 S-A CDRL1
<220>
   <221> VARIANT
   <222> (7)..(7)
   <223> X = V or I
<400> 300
   Ser Gly Ser Ser Ser Asn Xaa Gly Ser Asn Pro Val Asn
   1               5                   10
<210> 301
   <211> 12
   <212> PRT
   <213> Artificial Sequence
<220>
   <223> consensus sequence derived from M20 D02 S-A derived CDR L3
<220>
   <221> VARIANT
   <222> (3)..(3)
   <223> X = A, Q or R
<220>
   <221> VARIANT
   <222> (5)..(5)
   <223> X = D or G
<220>
   <221> VARIANT
   <222> (7)..(7)
   <223> X = R or S
<220>
   <221> VARIANT
   <222> (9) .. (9)
   <223> X = N, W or S
<220>
   <221> VARIANT
   <222> (12)..(12)
   <223> X = V, A or G
<400> 301
   Cys Ala Xaa Trp Xaa Asp Xaa Leu Xaa Gly Trp Xaa
   1               5                   10
<210> 302
   <211> 9
   <212> PRT
   <213> Artificial Sequence
<220>
   <223> consensus sequence derived from M31 B01 CDR H1
<220>
   <221> VARIANT
   <222> (3)..(3)
   <223> X = N or S
<220>
   <221> VARIANT
   <222> (4)..(4)
   <223> X = A or Y
<400> 302
   Phe Ser Xaa Xaa Trp Met Ser Trp Val
   1               5
<210> 303
   <211> 20
   <212> PRT
   <213> Artificial Sequence
<220>
   <223> consensus sequence derived from M31 B01 CDR H2
<220>
   <221> VARIANT
   <222> (10)..(10)
   <223> X = T or S
<220>
   <221> VARIANT
   <222> (11)..(11)
   <223> X = I or T
<400> 303
   Val Ser Tyr Ile Ser Ser Ser Gly Ser Xaa Xaa Tyr Tyr Ala Asp Ser
   1               5                   10                  15
   Val Lys Gly Arg
               20
<210> 304
   <211> 10
   <212> PRT
   <213> Artificial Sequence
<220>
   <223> consensus sequence derived from M31 B01 CDR H3
<220>
   <221> VARIANT
   <222> (10)..(10)
   <223> X = Y, K, G, W or N
<400> 304
   Ala Arg Glu Gly Leu Trp Ala Phe Asp Xaa
   1               5                   10
<210> 305
   <211> 14
   <212> PRT
   <213> Artificial Sequence
<220>
   <223> consensus sequence derived from M31 B01 CDR L1
<220>
   <221> VARIANT
   <222> (1) .. (1)
   <223> X = T or S
<400> 305
   Xaa Gly Ser Ser Ser Asn Ile Gly Ala Gly Tyr Val Val His
   1               5                   10
<210> 306
   <211> 7
   <212> PRT
   <213> Artificial Sequence
<220>
   <223> consensus sequence derived from M31 B01 CDR L2
<220>
   <221> VARIANT
   <222> (4)..(4)
   <223> X = K or Q
<400> 306
   Asp Asn Asn Xaa Arg Pro Ser
   1               5
<210> 307
   <211> 12
   <212> PRT
   <213> Artificial Sequence
<220>
   <223> consensus sequence derived from M31 B01 CDR L3
<220>
   <221> VARIANT
   <222> (4)..(4)
   <223> X = W, E, F or Y
<220>
   <221> VARIANT
   <222> (7)..(7)
   <223> X = R, S or M
<220>
   <221> VARIANT
   <222> (9) .. (9)
   <223> X = N, K or S
<220>
   <221> VARIANT
   <222> (10)..(10)
   <223> X = G or R
<220>
   <221> VARIANT
   <222> (11)..(11)
   <223> X = P or A
<400> 307
   Cys Ala Ala Xaa Asp Asp Xaa Leu Xaa Xaa Xaa Val
   1 5 10
<210> 308
   <211> 351
   <212> DNA
   <213> Artificial Sequence
<220>
   <223> C4.4a binder
<400> 308
   gaggtgcagc tgctggagag cggggggggg ctggtgcagc cgggggggag cctgcgcctg 60
   agctgcgcgg cgagcgggtt tacctttagc aacgcgtgga tgagctgggt gcgccaggcg 120
   ccggggaaag ggctggagtg ggtgagctat attagcagca gcgggagcac catttattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgataaatg gggcaggggg accctggtga ccgtgaccag c 351
<210> 309
   <211> 339
   <212> DNA
   <213> Artificial Sequence
<220>
   <223> C4.4a binder
<400> 309
   cagagcgtgc tgacccagcc gccgagcgcg agcgggaccc cggggcagcg cgtgaccatt 60
   agctgcaccg ggagcagcag caacattggg gcggggtatg tggtgcattg gtatcagcag 120
   ctgccgggga ccgcgccgaa actgctgatt tatgataaca acaaacgccc gagcggggtg 180
   ccggatcgct ttagcgggag caaaagcggg accagcgcga gcctggcgat tagcgggctg 240
   cgcagcgagg atgaggcgga ttattattgc gcggcgtatg atgatagcct gaaagggccg 300
   gtgtttgggg gggggaccaa actgaccgtg ctggggcag 339
<210> 310
   <211> 351
   <212> DNA
   <213> Artificial Sequence
<220>
   <223> C4.4a binder
<400> 310
   gaggtgcagc tgctggagag cggggggggg ctggtgcagc cgggggggag cctgcgcctg 60
   agctgcgcgg cgagcgggtt tacctttagc aacgcgtgga tgagctgggt gcgccaggcg 120
   ccggggaaag ggctggagtg ggtgagctat attagcagca gcgggagcac catttattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgataaatg ggggcagggg accctggtga ccgtgaccag c 351
<210> 311
   <211> 339
   <212> DNA
   <213> Artificial Sequence
<220>
   <223> C4.4a binder
<400> 311
   cagagcgtgc tgacccagcc gccgagcgtg agcggggcgc cggggcagcg cgtgaccatt 60
   agctgcaccg ggagcagcag caacattggg gcggggtatg tggtgcattg gtatcagcag 120
   ctgccgggga ccgcgccgaa actgctgatt tatgataaca acaaacgccc gagcggggtg 180
   ccggatcgct ttagcgggag caaaagcggg accagcgcga gcctggcgat tagcgggctg 240
   cgcagcgagg atgaggcgga ttattattgc gcggcgtttg atgatagcct gaacgggccg 300
   gtgtttgggg gggggaccaa actgaccgtg ctggggcag 339
<210> 312
   <211> 351
   <212> DNA
   <213> Artificial Sequence
<220>
   <223> C4.4a binder
<400> 312
   gaggtgcagc tgctggagag cggggggggg ctggtgcagc cgggggggag cctgcgcctg 60
   agctgcgcgg cgagcgggtt tacctttagc aacgcgtgga tgagctgggt gcgccaggcg 120
   ccggggaaag ggctggagtg ggtgagctat attagcagca gcgggagcac catttattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgataaatg ggggcagggg accctggtga ccgtgaccag c 351
<210> 313
   <211> 339
   <212> DNA
   <213> Artificial Sequence
<220>
   <223> C4.4a binder
<400> 313
   cagagcgtgc tgacccagcc gccgagcgtg agcggggcgc cggggcagcg cgtgaccatt 60
   agctgcaccg ggagcagcag caacattggg gcggggtatg tggtgcattg gtatcagcag 120
   ctgccgggga ccgcgccgaa actgctgatt tatgataaca acaaacgccc gagcggggtg 180
   ccggatcgct ttagcgggag caaaagcggg accagcgcga gcctggcgat tagcgggctg 240
   cgcagcgagg atgaggcgga ttattattgc gcggcgtttg atgatagcct gaacgggccg 300
   gtgtttgggg gggggaccaa actgaccgtg ctggggcag 339
<210> 314
   <211> 351
   <212> DNA
   <213> Artificial Sequence
<220>
   <223> C4.4a binder
<400> 314
   gaggtgcagc tgctggagag cggggggggg ctggtgcagc cgggggggag cctgcgcctg 60
   agctgcgcgg cgagcgggtt tacctttagc agcgcgtgga tgagctgggt gcgccaggcg 120
   ccggggaaag ggctggagtg ggtgagctat attagcagca gcgggagcag cacctattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgattggtg ggggcagggg accctggtga ccgtgaccag c 351
<210> 315
   <211> 339
   <212> DNA
   <213> Artificial Sequence
<220>
   <223> C4.4a binder
<400> 315
   cagagcgtgc tgacccagcc gccgagcgcg agcgggaccc cggggcagcg cgtgaccatt      60
   agctgcagcg ggagcagcag caacattggg gcggggtatg tggtgcattg gtatcagcag     120
   ctgccgggga ccgcgccgaa actgctgatt tatgataaca accagcgccc gagcggggtg     180
   ccggatcgct ttagcgggag caaaagcggg accagcgcga gcctggcgat tagcgggctg     240
   cgcagcgagg atgaggcgga ttattattgc gcggcgtatg atgatagcct gagcgggccg     300
   gtgtttgggg gggggaccaa actgaccgtg ctggggcag                            339
<210> 316
   <211> 351
   <212> DNA
   <213> Artificial Sequence
<220>
   <223> C4.4a binder
<400> 316
   gaggtgcagc tgctggagag cgggggcggc ctggtgcagc cggggggcag cctgcgcctg      60
   agctgcgcgg cgagcgggtt tacctttagc aacgcgtgga tgagctgggt gcgccaggcg     120
   ccggggaaag ggctggagtg ggtgagctat attagcagca gcgggagcac catttattat     180
   gcggatagcg tgaaagggcg ctttaccatt agccgcgata acagcaaaaa caccctgtat     240
   ctgcagatga acagcctgcg cgcggaggat accgcggtgt attattgcgc gcgcgagggg     300
   ctgtgggcgt ttgattattg gggcaggggg accctggtga ccgtgaccag c              351
<210> 317
   <211> 339
   <212> DNA
   <213> Artificial Sequence
<220>
   <223> C4.4a binder
<400> 317
   cagagcgtgc tgacccagcc gccgagcgcg agcgggaccc cggggcagcg cgtgaccatt        60
   agctgcagcg ggagcagcag caacattggg gcggggtatg tggtgcattg gtatcagcag       120
   ctgccgggga ccgcgccgaa actgctgatt tatgataaca accagcgccc gagcggggtg       180
   ccggatcgct ttagcgggag caaaagcggg accagcgcga gcctggcgat tagcgggctg       240
   cgcagcgagg atgaggcgga ttattattgc gcggcgtatg atgatagcct gaacgggccg       300
   gtgtttgggg gggggaccaa actgaccgtg ctggggcag                              339
<210> 318
   <211> 351
   <212> DNA
   <213> Artificial Sequence
<220>
   <223> C4.4a binder
<400> 318
   gaggtgcagc tgctggagag cggggggggg ctggtgcagc cgggggggag cctgcgcctg        60
   agctgcgcgg cgagcgggtt tacctttagc agcgcgtgga tgagctgggt gcgccaggcg       120
   ccggggaaag ggctggagtg ggtgagctat attagcagca gcgggagcag cacctattat       180
   gcggatagcg tgaaagggcg ctttaccatt agccgcgata acagcaaaaa caccctgtat       240
   ctgcagatga acagcctgcg cgcggaggat accgcggtgt attattgcgc gcgcgagggg       300
   ctgtgggcgt ttgattattg gggcaggggg accctggtga ccgtgagcag c                351
<210> 319
   <211> 339
   <212> DNA
   <213> Artificial Sequence
<220>
   <223> C4.4a binder
<400> 319
   gagagcgtgc tgacccagcc gccgagcgcg agcgggaccc cggggcagcg cgtgaccatt        60
   agctgcagcg ggagcagcag caacattggg gcggggtatg tggtgcattg gtatcagcag       120
   ctgccgggga ccgcgccgaa actgctgatt tatgataaca accagcgccc gagcggggtg       180
   ccggatcgct ttagcgggag caaaagcggg accagcgcga gcctggcgat tagcgggctg       240
   cgcagcgagg atgaggcgga ttattattgc gcggcgtggg atgatcgcct gaacgggccg       300
   gtgtttgggg gggggaccaa actgaccgtg ctggggcag                              339
<210> 320
   <211> 351
   <212> DNA
   <213> Artificial Sequence
<220>
   <223> C4.4a binder
<400> 320
   gaggtgcagc tgctggagag cggggggggg ctggtgcagc cgggggggag cctgcgcctg        60
   agctgcgcgg cgagcgggtt tacctttagc aacgcgtgga tgagctgggt gcgccaggcg       120
   ccggggaaag ggctggagtg ggtgagctat attagcagca gcgggagcac catttattat       180
   gcggatagcg tgaaagggcg ctttaccatt agccgcgata acagcaaaaa caccctgtat       240
   ctgcagatga acagcctgcg cgcggaggat accgcggtgt attattgcgc gcgcgagggg       300
   ctgtgggcgt ttgattattg gggcaggggg accctggtga ccgtgagcag c                351
<210> 321
   <211> 339
   <212> DNA
   <213> Artificial Sequence
<220>
   <223> C4.4a binder
<400> 321
   gagagcgtgc tgacccagcc gccgagcgtg agcggggcgc cggggcagcg cgtgaccatt        60
   agctgcaccg ggagcagcag caacattggg gcggggtatg tggtgcattg gtatcagcag       120
   ctgccgggga ccgcgccgaa actgctgatt tatgataaca acaaacgccc gagcggggtg       180
   ccggatcgct ttagcgggag caaaagcggg accagcgcga gcctggcgat tagcgggctg       240
   cgcagcgagg atgaggcgga ttattattgc gcggcgtatg atgatcgcct gaacgggccg       300
   gtgtttgggg gggggaccaa actgaccgtg ctggggcag                              339
<210> 322
   <211> 351
   <212> DNA
   <213> Artificial Sequence
<220>
   <223> C4.4a binder
<400> 322
   gaggtgcagc tgctggagag cggggggggg ctggtgcagc cgggggggag cctgcgcctg 60
   agctgcgcgg cgagcgggtt tacctttagc agcgcgtgga tgagctgggt gcgccaggcg 120
   ccggggaaag ggctggagtg ggtgagctat attagcagca gcgggagcag cacctattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgatgggtg ggggcagggg accctggtga ccgtgagcag c 351
<210> 323
   <211> 339
   <212> DNA
   <213> Artificial Sequence
<220>
   <223> C4.4a binder
<400> 323
   cagagcgtgc tgacccagcc gccgagcgcg agcgggaccc cggggcagcg cgtgaccatt 60
   agctgcagcg ggagcagcag caacattggg gcggggtatg tggtgcattg gtatcagcag 120
   ctgccgggga ccgcgccgaa actgctgatt tatgataaca accagcgccc gagcggggtg 180
   ccggatcgct ttagcgggag caaaagcggg accagcgcga gcctggcgat tagcgggctg 240
   cgcagcgagg atgaggcgga ttattattgc gcggcgtatg atgatagcct gaaccgcccg 300
   gtgtttgggg gggggaccaa actgaccgtg ctggggcag 339
<210> 324
   <211> 351
   <212> DNA
   <213> Artificial Sequence
<220>
   <223> C4.4a binder
<400> 324
   gaggtgcagc tgctggagag cggggggggg ctggtgcagc cgggggggag cctgcgcctg 60
   agctgcgcgg cgagcgggtt tacctttagc aacgcgtgga tgagctgggt gcgccaggcg 120
   ccggggaaag ggctggagtg ggtgagctat attagcagca gcgggagcac catttattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgattattg ggggcagggg accctggtga ccgtgagcag c 351
<210> 325
   <211> 339
   <212> DNA
   <213> Artificial Sequence
<220>
   <223> C4.4a binder
<400> 325
   cagagcgtgc tgacccagcc gccgagcgtg agcggggcgc cggggcagcg cgtgaccatt 60
   agctgcaccg ggagcagcag caacattggg gcggggtatg tggtgcattg gtatcagcag 120
   ctgccgggga ccgcgccgaa actgctgatt tatgataaca acaaacgccc gagcggggtg 180
   ccggatcgct ttagcgggag caaaagcggg accagcgcga gcctggcgat tagcgggctg 240
   cgcagcgagg atgaggcgga ttattattgc gcggcgtttg atgatagcct gaacgggccg 300
   gtgtttgggg gggggaccaa actgaccgtg ctggggcag 339
<210> 326
   <211> 351
   <212> DNA
   <213> Artificial Sequence
<220>
   <223> C4.4a binder
<400> 326
   gaggtgcagc tgctggagag cggggggggg ctggtgcagc cgggggggag cctgcgcctg 60
   agctgcgcgg cgagcgggtt tacctttagc agcgcgtgga tgagctgggt gcgccaggcg 120
   ccggggaaag ggctggagtg ggtgagctat attagcagca gcgggagcag cacctattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgataaatg ggggcagggg accctggtga ccgtgaccag c 351
<210> 327
   <211> 339
   <212> DNA
   <213> Artificial Sequence
<220>
   <223> C4.4a binder
<400> 327
   cagagcgtgc tgacccagcc gccgagcgcg agcgggaccc cggggcagcg cgtgaccatt 60
   agctgcaccg ggagcagcag caacattggg gcggggtatg tggtgcattg gtatcagcag 120
   ctgccgggga ccgcgccgaa actgctgatt tatgataaca acaaacgccc gagcggggtg 180
   ccggatcgct ttagcgggag caaaagcggg accagcgcga gcctggcgat tagcgggctg 240
   cgcagcgagg atgaggcgga ttattattgc gcggcgtttg atgatagcct gaacgggccg 300
   gtgtttgggg gggggaccaa actgaccgtg ctggggcag 339
<210> 328
   <211> 351
   <212> DNA
   <213> Artificial Sequence
<220>
   <223> C4.4a binder
<400> 328
   gaggtgcagc tgctggagag cggggggggg ctggtgcagc cgggggggag cctgcgcctg 60
   agctgcgcgg cgagcgggtt tacctttagc agcgcgtgga tgagctgggt gcgccaggcg 120
   ccggggaaag ggctggagtg ggtgagctat attagcagca gcgggagcag cacctattat 180
   gcggatagcg tgaaagggcg ctttaccatt agccgcgata acagcaaaaa caccctgtat 240
   ctgcagatga acagcctgcg cgcggaggat accgcggtgt attattgcgc gcgcgagggg 300
   ctgtgggcgt ttgataaatg gggcaggggg accctggtga ccgtgagcag c 351
<210> 329
   <211> 339
   <212> DNA
   <213> Artificial Sequence
<220>
   <223> C4.4a
<400> 329
   cagagcgtgc tgacccagcc gccgagcgcg agcgggaccc cggggcagcg cgtgaccatt 60
   agctgcagcg ggagcagcag caacattggg gcggggtatg tggtgcattg gtatcagcag 120
   ctgccgggga ccgcgccgaa actgctgatt tatgataaca accagcgccc gagcggggtg 180
   ccggatcgct ttagcgggag caaaagcggg accagcgcga gcctggcgat tagcgggctg 240
   cgcagcgagg atgaggcgga ttattattgc gcggcgtttg atgatagcct gaacgggccg 300
   gtgtttgggg gggggaccaa actgaccgtg ctggggcag 339
<210> 330
   <211> 351
   <212> DNA
   <213> Artificial Sequence
<220>
   <223> C4.4a binder
<400> 330
   gaggtgcagc tgctggagag cggggggggg ctggtgcagc cgggggggag cctgcgcctg      60
   agctgcgcgg cgagcgggtt tacctttagc agcgcgtgga tgagctgggt gcgccaggcg      120
   ccggggaaag ggctggagtg ggtgagctat attagcagca gcgggagcag cacctattat      180
   gcggatagcg tgaaagggcg ctttaccatt agccgcgata acagcaaaaa caccctgtat      240
   ctgcagatga acagcctgcg cgcggaggat accgcggtgt attattgcgc gcgcgagggg      300
   ctgtgggcgt ttgataaatg ggggcagggg accctggtga ccgtgagcag c               351
<210> 331
   <211> 339
   <212> DNA
   <213> Artificial Sequence
<220>
   <223> C4.4a binder
<400> 331
   cagagcgtgc tgacccagcc gccgagcgcg agcgggaccc cggggcagcg cgtgaccatt      60
   agctgcagcg ggagcagcag caacattggg gcggggtatg tggtgcattg gtatcagcag      120
   ctgccgggga ccgcgccgaa actgctgatt tatgataaca accagcgccc gagcggggtg      180
   ccggatcgct ttagcgggag caaaagcggg accagcgcga gcctggcgat tagcgggctg      240
   cgcagcgagg atgaggcgga ttattattgc gcggcgtttg atgatcgcct gagcgggccg      300
   gtgtttgggg gggggaccaa actgaccgtg ctggggcag                             339
<210> 332
   <211> 366
   <212> DNA
   <213> Artificial Sequence
<220>
   <223> C4.4a binder
<400> 332
   gaggtgcagc tgctggagag cggggggggg ctggtgcagc cgggggggag cctgcgcctg 60
   agctgcgcgg cgagcgggtt tacctttagc gattatcaga tgacctggat tcgccagacc 120
   ccggggaaag ggctggagtg ggtgagcggg gtgagctgga acggggcgcg cacccattat 180
   gcggatagcg tgaaagggcg ctttaccatt agccgcgata acagcaaaaa caccctgtat 240
   ctgcagatga acagcctgcg cgcggaggat accgcggtgt attattgcgc gaaaggggat 300
   tatctggtgt atagcagcta ttattttaaa agctgggggc aggggaccct ggtgaccgtg 360
   accagc 366
<210> 333
   <211> 336
   <212> DNA
   <213> Artificial Sequence
<220>
   <223> C4.4a binder
<400> 333
   cagagcgtgc tgacccagcc gccgagcgcg agcgggaccc cggggcagcg cgtgaccatt 60
   agctgcagcg ggagcagcag caacgtgggg agcaacccgg tgaactggta tcagcagctg 120
   ccggggaccg cgccgaaact gctgatttat cgcaacaacc agcgcccgag cggggtgccg 180
   gatcgcttta gcgggagcaa aagcgggacc agcgcgagcc tggcgattag cgggctgcgc 240
   agcgaggatg aggcggatta ttattgcgcg gcgtgggatg atcgcctgaa cgggtggggg 300
   tttggggggg ggaccaaact gaccgtgctg gggcag 336
<210> 334
   <211> 363
   <212> DNA
   <213> Artificial Sequence
<220>
   <223> C4.4a binder
<400> 334
   gagcagctgc tggagagcgg gggggggctg gtgcagccgg gggggagcct gcgcctgagc 60
   tgcgcggcga gcgggtttac ctttagcagc tatcagatga cctggattcg ccagaccccg 120
   gggaaagggc tggagtgggt gagcgggatt agctggaacg gggggagcac ccattatgcg 180
   gatagcgtga aagggcgctt taccattagc cgcgataaca gcaaaaacac cctgtatctg 240
   cagatgaaca gcctgcgcgc ggaggatacc gcggtgtatt attgcgcgaa aggggattat 300
   ctggtgtata gcgcgtatta ttttgatagc tgggggcagg ggaccctggt gaccgtgagc 360
   agc 363
<210> 335
   <211> 333
   <212> DNA
   <213> Artificial Sequence
<220>
   <223> C4.4a binder
<400> 335
   caggtgctga cccagccgcc gagcgcgagc gggaccccgg ggcagcgcgt gaccattagc 60
   tgcagcggga gcagcagcaa cattgggagc aacccggtga actggtatca gcagctgccg 120
   gggaccgcgc cgaaactgct gatttatcgc aacaaccagc gcccgagcgg ggtgccggat 180
   cgctttagcg ggagcaaaag cgggaccagc gcgagcctgg cgattagcgg gctgcgcagc 240
   gaggatgagg cggattatta ttgcgcggcg tgggatgatc gcctgaacgg gtgggggttt 300
   ggggggggga ccaaactgac cgtgctgggg cag 333
<210> 336
   <211> 366
   <212> DNA
   <213> Artificial Sequence
<220>
   <223> C4.4a binder
<400> 336
   gaggtgcagc tgctggagag cggggggggg ctggtgcagc cgggggggag cctgcgcctg 60
   agctgcgcgg cgagcgggtt tacctttagc gattatcaga tgacctggat tcgccagacc 120
   ccggggaaag ggctggagtg ggtgagcggg attagctgga acggggggag cacccattat 180
   gcggatagcg tgaaagggcg ctttaccatt agccgcgata acagcaaaaa caccctgtat 240
   ctgcagatga acagcctgcg cgcggaggat accgcggtgt attattgcgc gaaaggggat 300
   tatctggtgt atagcagcta ttattttaaa tattgggggc aggggaccct ggtgaccgtg 360
   agcagc 366
<210> 337
   <211> 336
   <212> DNA
   <213> Artificial Sequence
<220>
   <223> C4.4a binder
<400> 337
   cagagcgtgc tgacccagcc gccgagcgcg agcgggaccc cggggcagcg cgtgaccatt 60
   agctgcagcg ggagcagcag caacattggg agcaacccgg tgaactggta tcagcagctg 120
   ccggggaccg cgccgaaact gctgatttat cgcaacaacc agcgcccgag cggggtgccg 180
   gatcgcttta gcgggagcaa aagcgggacc agcgcgagcc tggcgattag cgggctgcgc 240
   agcgaggatg aggcggatta ttattgcgcg gcgtgggatg atcgcctgaa cgggtgggcg 300
   tttggggggg ggaccaaact gaccgtgctg gggcag 336
<210> 338
   <211> 366
   <212> DNA
   <213> Artificial Sequence
<220>
   <223> C4.4a binder
<400> 338
   gaggtgcagc tgctggagag cggggggggg ctggtgcagc cgggggggag cctgcgcctg 60
   agctgcgcgg cgagcgggtt tacctttagc gattatcaga tgacctggat tcgccagacc 120
   ccggggaaag ggctggagtg ggtgagcggg attagctgga acggggggag cacccattat 180
   gcggatagcg tgaaagggcg ctttaccatt agccgcgata acagcaaaaa caccctgtat 240
   ctgcagatga acagcctgcg cgcggaggat accgcggtgt attattgcgc gaaaggggat 300
   tatctggtgt atagcagcta ttattttaaa agctgggggc aggggaccct ggtgaccgtg 360
   agcagc 366
<210> 339
   <211> 336
   <212> DNA
   <213> Artificial Sequence
<220>
   <223> C4.4a binder
<400> 339
   cagagcgtgc tgacccagcc gccgagcgcg agcgggaccc cggggcagcg cgtgaccatt 60
   agctgcagcg ggagcagcag caacattggg agcaacccgg tgaactggta tcagcagctg 120
   ccggggaccg cgccgaaact gctgatttat cgcaacaacc agcgcccgag cggggtgccg 180
   gatcgcttta gcgggagcaa aagcgggacc agcgcgagcc tggcgattag cgggctgcgc 240
   agcgaggatg aggcggatta ttattgcgcg gcgtgggatg atagcctgaa cgggtggggg 300
   tttggggggg ggaccaaact gaccgtgctg gggcag 336
<210> 340
   <211> 366
   <212> DNA
   <213> Artificial Sequence
<220>
   <223> C4.4a binder
<400> 340
   gaggtgcagc tgctggagag cgggggcggg ctggtgcagc cggggggcag cctgcgcctg 60
   agctgcgcgg cgagcgggtt tacctttagc gattatcaga tgacctggat tcgccagacc 120
   ccggggaaag ggctggagtg ggtgagcggg attagctgga acgggggcag cacccattat 180
   gcggatagcg tgaaagggcg ctttaccatt agccgcgata acagcaaaaa caccctgtat 240
   ctgcagatga acagcctgcg cgcggaggat accgcggtgt attattgcgc gaaaggggat 300
   tatctggtgt ataaaagcta ttattttaaa agctgggggc aggggaccct ggtgaccgtg 360
   agcagc 366
<210> 341
   <211> 336
   <212> DNA
   <213> Artificial Sequence
<220>
   <223> C4.4a binder
<400> 341
   cagagcgtgc tgacccagcc gccgagcgcg agcgggaccc cggggcagcg cgtgaccatt      60
   agctgcagcg ggagcagcag caacattggg agcaacccgg tgaactggta tcagcagctg     120
   ccggggaccg cgccgaaact gctgatttat cgcaacaacc agcgcccgag cggggtgccg     180
   gatcgcttta gcgggagcaa aagcgggacc agcgcgagcc tggcgattag cgggctgcgc     240
   agcgaggatg aggcggatta ttattgcgcg gcgtgggatg atcgcctgag cgggtggggg     300
   tttggggggg ggaccaaact gaccgtgctg gggcag                                336
<210> 342
   <211> 366
   <212> DNA
   <213> Artificial Sequence
<220>
   <223> C4.4a binder
<400> 342
   gaggtgcagc tgctggagag cggggggggg ctggtgcagc cgggggggag cctgcgcctg      60
   agctgcgcgg cgagcgggtt tacctttagc gattatcaga tgacctggat tcgccagacc     120
   ccggggaaag ggctggagtg ggtgagcggg attagctgga acggggggag cacccattat     180
   gcggatagcg tgaaagggcg ctttaccatt agccgcgata acagcaaaaa caccctgtat     240
   ctgcagatga acagcctgcg cgcggaggat accgcggtgt attattgcgc gaaaggggat     300
   tatctggtgt atagcagcta ttattttaaa agctgggggc aggggaccct ggtgaccgtg     360
   agcagc                                                                366
<210> 343
   <211> 336
   <212> DNA
   <213> Artificial Sequence
<220>
   <223> C4.4a binder
<400> 343
   cagagcgtgc tgacccagcc gccgagcgcg agcgggaccc cggggcagcg cgtgaccatt 60
   agctgcagcg ggagcagcag caacattggg agcaacccgg tgaactggta tcagcagctg 120
   ccggggaccg cgccgaaact gctgatttat cgcaacaacc agcgcccgag cggggtgccg 180
   gatcgcttta gcgggagcaa aagcgggacc agcgcgagcc tggcgattag cgggctgcgc 240
   agcgaggatg aggcggatta ttattgcgcg gcgtgggatg atcgcctgag cgggtggggg 300
   tttggggggg ggaccaaact gaccgtgctg gggcag 336
<210> 344
   <211> 366
   <212> DNA
   <213> Artificial Sequence
<220>
   <223> C4.4a binder
<400> 344
   gaggtgcagc tgctggagag cggggggggg ctggtgcagc cgggggggag cctgcgcctg 60
   agctgcgcgg cgagcgggtt tacctttagc gattatcaga tgacctggat tcgccagacc 120
   ccggggaaag ggctggagtg ggtgagcggg attagctgga acggggggag cacccattat 180
   gcggatagcg tgaaagggcg ctttaccatt agccgcgata acagcaaaaa caccctgtat 240
   ctgcagatga acagcctgcg cgcggaggat accgcggtgt attattgcgc gaaaggggat 300
   tatctggtgt ataaaagcta ttattttaaa agctgggggc aggggaccct ggtgaccgtg 360
   agcagc 366
<210> 345
   <211> 336
   <212> DNA
   <213> Artificial Sequence
<220>
   <223> C4.4a binder
<400> 345
   cagagcgtgc tgacccagcc gccgagcgcg agcgggaccc cggggcagcg cgtgaccatt 60
   agctgcagcg ggagcagcag caacattggg agcaacccgg tgaactggta tcagcagctg 120
   ccggggaccg cgccgaaact gctgatttat cgcaacaacc agcgcccgag cggggtgccg 180
   gatcgcttta gcgggagcaa aagcgggacc agcgcgagcc tggcgattag cgggctgcgc 240
   agcgaggatg aggcggatta ttattgcgcg gcgtgggatg atagcctgaa cgggtggggg 300
   tttggggggg ggaccaaact gaccgtgctg gggcag 336

## Description

The present invention provides recombinant antigen-binding regions and antibodies and functional fragments containing such antigen-binding regions that are specific for the membrane-anchored, 29 kDa polypeptide named C4.4a , which is over expressed in tumors.
Furthermore, it has a high abundance in metastases of these cancer types. The antibodies, accordingly, can be used to treat these and other disorders and conditions. Antibodies of the invention also can be used in the diagnostics field, as well as for further investigating the role of C4.4a in the progression of disorders associated with cancer. The invention also provides nucleic acid sequences encoding the foregoing antibodies, vectors containing the same, pharmaceutical compositions and kits with instructions for use.

### BACKGROUND OF THE INVENTION

Antibody-based therapy is proving very effective in the treatment of various cancers, including solid tumors. For example, HERCEPTIN® has been used successfully to treat breast cancer and RITUXAN® is effective in B-cell related cancer types. Central to the development of a successful antibody-based therapy is isolation of antibodies against cell-surface proteins found to be preferentially expressed on tumor cells. The C4.4a (gene name: LYPD3) polypeptide is a glycophosphatidylinositol (GPI)-anchored, highly glycosylated cell surface protein. Rat C4.4a was first described as a metastasis-associated, cell surface protein in metastasizing rat pancreatic tumor cells (Rösel M. et al., Oncogene 1998,17(15):1989-2002). Human C4.4a was cloned form a placental cDNA library (Würfel, J. et. al. Gene 2001,262:35-41). C4.4a displays structural homology to the uPAR receptor and contains two LY6 domains, exhibiting the typical three finger protein fold (Jacobsen B. & Ploug M., Current Medicinal Chemistry 2008, 15:2559-2573). The protein is highly glycosylated and contains 6 predicted N-glycosylation sites and several O-glycosylation sites. Furthermore C4.4a contains in total 9 disulfide bridges located in the two Ly6 domains (Hansen L. et al., Biochem J. 2004, 380:845-857). C4.4a shows a strong expression in tumor cells like lung cancer, colorectal cancer, breast cancer, Cervix cancer, pancreatic cancer, renal cancer, Head and Neck cancer and melanomas. Northern blot analysis demonstrated C4.4a expression in ~ 50 % of primary lung tumors and ∼75 % of lung tumor metastases, while expression in non-diseased lung tissue was undetectable (Würfel J. et. al., Gene 2001, 262:35-41). In non-small cell lung cancer C4.4a can be used as a prognostic marker. Here clinical data clearly show that high C4.4a expression correlates with poor prognosis (Hansen L. et al., Lung Cancer 2007, 58:260-266). In melanoma detailed expression analysis revealed that C4.4a is not expressed in melanocytes and nevi but is expressed in ~ 60 % of primary malignant melanomas and in 100 % of lymph node and skin metastases (Seiter S. et al., J Invest Dermatol. 2001, 116(2):344-347). Furthermore up regulation of C4.4a gene expression was found in breast cancer tissue compared to matched adjacent normal breast tissue (Fletcher G.C., Br. J. Cancer 2003, 88(4):579-585), in various breast cancer cell lines and in urothelial cancer compared to normal urothelium (Smith B. A. et al., Cancer Res 2001, 61(4):1678-1685). C4.4a expression was demonstrated by FACS with a polyclonal antibody in various tumor cell lines of colorectal cancer, pancreatic cancer, breast cancer and prostate cancer. In IHC studies of colorectal cancer, pancreatic cancer and breast cancer samples variable glycosylation of C4.4a on human tumor cell lines interferes with binding of these antibodies. Therefore, C4.4a has to be at least partially deglycosylated to allow for binding of these polyclonal antibodies. In colorectal cancer patients C4.4a expression is highly prevalent and C4.4a is shed from the cell surface, making it a prognostic serum tumor marker. Expression of C4.4a at invasive front is a novel prognostic marker for disease recurrence of colorectal cancer (K. Konishi et al., Cancer Science 2010) Diagnostic antibodies against soluble serum C4.4a have not been described (Paret C. et al., British Journal of Cancer 2007, 97:1146-1156). In normal tissue C4.4a expression is limited to skin keratinocytes, esophagus endothelial cells and placental cells (Würfel J. et. al., Gene 2001, 262:35-41), making it an ideal target for tumor therapy. WO01/23553 suggests the use of a C4.4a inhibitor (e.g. an anti-C4.4a antibody) which decreases or inhibits C4.4a expression or activity for the treatment of cancer.

The exact function of C4.a is unknown; however it is up regulated in migrating keratinocytes in wound healing (Hansen L. et al., Biochem J. 2004, 380:845-857). In light of metastasis association and structural homology to uPAR it is proposed that this molecule is involved in tumor cell invasion probably through interaction with the extra cellular matrix (Rösel M. et al., Oncogene 1998, 17(15):1989-2002; Paret C. et al., British Journal of Cancer 2007, 97:1146-1156). Potential ligands are Laminin1 and 5, Galectin 3 (Paret C., Int. J. Cancer 2005, 115:724-733) as well as agr2 and agr3 (Fletcher GC., Brit. J. Cancer 2003, 88:579-585).

The predictive value of xenograft murine cancer models for clinical outcome of immunotoxin cancer therapy is often limited by a lack of cross-reactivity of the therapeutic antibodies with their murine orthologues, which leads to reduced unspecific binding to normal tissue. On the other hand, neutralizing anti-mouse Fv antibodies which are formed in patients being treated with murine or chimeric antibodies may result in either dose-limiting toxicity or diminished therapeutic potency. Thus, to fully exploit the potential of specific C4.4a expression in cancer therapy, targeting antibodies are required which combine the advantages of high affinity C4.4a binding with a fully human or humanized antibody format, and with murine cross-reactivity.

A further necessary feature of novel antibodies is high affinity binding to different cancer cell lines expressing C4.4a on their surface. C4.4a is differently glycosylated on tumor cells (Paret C. et al., British Journal of Cancer 2007 97:1146-1156). Thus, effective anti-C4.4a antibodies must bind to an epitope presented by tumor cells from different patients, independently of individual variance including, but not restricted to, variances in glycosylation patterns, which leads to the expression of different forms of C4.4a.

Provided herein are antibodies, antigen-binding antibody fragments thereof, or variants thereof, that bind to C4.4a with high affinity, internalize efficiently, and that are preferably cross-reactive to C4.4a from another species. Also provided are antibody-based therapies for cancer, in particular for C4.4a expressing tumors, such as cancers of the breast, respiratory tract, brain, reproductive organs, digestive tract, urinary tract, eye, liver, skin, head and neck, thyroid, parathyroid, and their distant metastases and also including lymphomas, sarcomas and leukemias. These Therapies are using antibodies, antigen-binding antibody fragments thereof, or variants thereof, that facilitate delivery of therapeutically active agents to cancer cells.

### SUMMARY OF THE INVENTION

It is an object of the invention to provide antibodies, or antigen-binding antibody fragments thereof, or variants thereof, that are highly selective for the C4.4a polypeptide either GPI-anchored onto the cell surface or soluble, by removal of the GPI-portion, in patients' serum and which may be employed in methods for detection of C4.4a expression, which is associated with disease states such as cancer of the lung, colon, breast, cervix, pancreas, kidney, Head and Neck or melanomas, and in the treatment of such disease states. Toward these ends, it is an object of the invention to provide isolated human, humanized or chimeric antibodies, or antigen binding antibody fragments thereof, that specifically bind to a C4.4a epitope which is present in different forms of the mature human C4.4a polypeptide of 278 amino acids (SEQ ID 1), which is presented by C4.4a expressing cancer cell lines, and/or which is bound by these antibodies with high affinities. As used herein, different 'forms' of C4.4a include, but are not restricted to, different glycoforms, different isoforms or C4.4a polypeptides which undergo different translational and posttranslational modifications. It is another object of the invention to provide antibodies, or antigen-binding antibody fragments thereof, or variants thereof that are safe for human administration.

It is object of this disclosure to provide antibodies, or antigen-binding antibody fragments thereof, or variants thereof, which bind to human C4.4a and are cross-reactive to C4.4a of another species. Preferably said other species is a rodent, such as for example mouse or rat. Most preferably the antibodies, or antigen-binding antibody fragments thereof, or variants thereof bind to human C4.4a and are cross-reactive to murine C4.4a.

It is another object of this disclosure to provide antibodies, or antigen-binding antibody fragments thereof, or variants thereof, which bind to a broad range of different C4.4a-expressing cell lines. Disclosed are antibodies or variants thereof, which bind to different C4.4a-expressing cancer cells or tumor cells and elicit immune effector activity (e.g. ADCC or CDC) against C4.4a-expressing cancer cells, by using one or more antibodies or variants thereof, of the invention.

It is an object of the invention to provide antibodies, or antigen-binding antibody fragments thereof, or variants thereof, which are internalized efficiently following binding to a C4.4a expressing cell. An antibody disclosed herein might be co-administered with known medicaments, and in some instances the antibody might itself be modified. For example, an antibody could be conjugated to a cytotoxic agent, immunotoxin, toxophore or radioisotope to potentially further increase efficacy.

It is another object of the invention to provide antibodies which constitute a tool for diagnosis of malignant or dysplastic conditions in which C4.4a expression is elevated compared to normal tissue or where C4.4a is shed from the cell surface and becoming detectable in serum. Provided are anti-C4.4a antibodies conjugated to a detectable marker. Preferred markers are a radiolabel, an enzyme, a chromophore or a fluorescer.

The invention is also related to polynucleotides encoding the antibodies of the invention, or antigen-binding fragments thereof, cells expressing the antibodies of the invention, or antigen-binding fragments thereof, methods for producing the antibodies of the invention, or antigen-binding fragments thereof, methods for inhibiting the growth of dysplastic cells using the antibodies of the invention, or antigen-binding fragments thereof, and methods for treating and detecting cancer using the antibodies of the invention, or antigen-binding fragments thereof.

The invention provides antibodies that are distinguished from existing C4.4a antibodies described by Paret et al. (Paret C. et al., British Journal of Cancer 2007 97:1146-1156) in that they a) bind to native, cell surface expressed and fully glycosylated C4.4a, to domain S1 of native, cell surface expressed and fully glycosylated C4.4a, b) are cross-reactive to murine C4.4a and c) internalize efficiently into C4.4a-expressing cells. Hansen et al. (Thrombosis and Haemostasis, Vol. 93, No. 4, 2005page A33) discloses in a a short meeting abstract two monoclonal antibodies binding to domain 1 (S1) of C4.4a. It is mentioned that the two antibodies recognize non overlapping epitopes. These antibodies are not described as being murine cross-reactive and no pharmacological features are provided. These antibodies are described as tools for research, only. Starting from Hansen et al. or Paret et. al this invention provides antibodies which are suitable for a treatment or a diognosig porpose.

These and other objects of the invention are more fully described herein.

This disclosure provides an isolated antibody or antigen-binding fragment thereof that contains an antigen-binding region that binds specifically to native, cell surface expressed and fully glycosylated C4.4a, preferably binds specifically to domain S1 (amino acids 1-85 of C4.4a; SEQ ID NO: 1) of native, cell surface expressed and fully glycosylated C4.4a polypeptide. In another embodiment the antibodies or antigen-binding fragments are internalized into a C4.4a expressing cell upon binding of the antibody or antigen-binding fragment to the aforementioned cell. In a further preferred embodiment of this disclosure the antibodies or antigen-binding fragments compete in binding to C4.4a with the antibodies M31-B01 or M20-D02 S-A. In a further preferred embodiment of this disclosure the antibodies or antigen-binding fragments compete in binding to human C4.4a with the antibodies M31-B01 or M20-D02 S-A. In a further preferred embodiment of this disclosure the antibodies or antigen-binding fragments compete in binding to human and rodent C4.4a with the antibodies M31-B01 or M20-D02 S-A, a further preferred embodiment is wherein the rodent C4.4a is mouse C4.4a.

An antibody of the invention or antigen-binding fragment thereof comprises a heavy chain antigen-binding region that comprises SEQ ID NO:5 (H-CDR1), SEQ ID NO:9 (H-CDR2) and SEQ ID NO: 13 (H-CDR3) and comprises a light chain antigen-binding region that comprises SEQ ID NO:17 (L-CDR1), SEQ ID NO:21 (L-CDR2) and SEQ ID NO:25 (L-CDR3).

In more preferred embodiment the antibody of the disclosure or antigen-binding fragment thereof comprises a heavy chain antigen-binding region that comprises SEQ ID NO:6 (H-CDR1), SEQ ID NO:10 (H-CDR2) and SEQ ID NO:14 (H-CDR3) and comprises a light chain antigen-binding region that comprises SEQ ID NO:18 (L-CDR1), SEQ ID NO:22 (L-CDR2) and SEQ ID NO:26 (L-CDR3).

In a more preferred embodiment the antibody of the disclosure or antigen-binding fragment thereof comprises a heavy chain antigen-binding region that comprises SEQ ID NO:7 (H-CDR1), SEQ ID NO:11 (H-CDR2) and SEQ ID NO:15 (H-CDR3) and comprises a light chain antigen-binding region that comprises SEQ ID NO:19 (L-CDR1), SEQ ID NO:23 (L-CDR2) and SEQ ID NO:27 (L-CDR3).

In a more preferred embodiment the antibody of the disclosure or antigen-binding fragment thereof comprises a heavy chain antigen-binding region that comprises SEQ ID NO:8 (H-CDR1), SEQ ID NO:12 (H-CDR2) and SEQ ID NO:16 (H-CDR3) and comprises a light chain antigen-binding region that comprises SEQ ID NO:20 (L-CDR1), SEQ ID NO:24 (L-CDR2) and SEQ ID NO:28 (L-CDR3).

An antibody of the invention or antigen-binding fragment thereof comprises a heavy chain antigen-binding region that comprises SEQ ID NO:45 (H-CDR1), SEQ ID NO:46 (H-CDR2) and SEQ ID NO:47 (H-CDR3) and comprises a light chain antigen-binding region that comprises SEQ ID NO:48 (L-CDR1), SEQ ID NO:49 (L-CDR2) and SEQ ID NO:50 (L-CDR3).

In a more preferred embodiment the antibody of the disclosure or antigen-binding fragment thereof comprises a heavy chain antigen-binding region that comprises SEQ ID NO:55 (H-CDR1), SEQ ID NO:56 (H-CDR2) and SEQ ID NO:57 (H-CDR3) and comprises a light chain antigen-binding region that comprises SEQ ID NO:58 (L-CDR1), SEQ ID NO:59 (L-CDR2) and SEQ ID NO:60 (L-CDR3).

In a more preferred embodiment the antibody of the disclosure or antigen-binding fragment thereof comprises a heavy chain antigen-binding region that comprises SEQ ID NO:65 (H-CDR1), SEQ ID NO:66 (H-CDR2) and SEQ ID NO:67 (H-CDR3) and comprises a light chain antigen-binding region that comprises SEQ ID NO:68 (L-CDR1), SEQ ID NO:69 (L-CDR2) and SEQ ID NO:70 (L-CDR3).

In a further more preferred embodiment the antibody of the disclosure or antigen-binding fragment thereof comprises a heavy chain antigen-binding region that comprises SEQ ID NO:75 (H-CDR1), SEQ ID NO:76 (H-CDR2) and SEQ ID NO:77 (H-CDR3) and comprises a light chain antigen-binding region that comprises SEQ ID NO:78 (L-CDR1), SEQ ID NO:79 (L-CDR2) and SEQ ID NO:80 (L-CDR3).

In a more preferred embodiment the antibody of the disclosure or antigen-binding fragment thereof comprises a heavy chain antigen-binding region that comprises SEQ ID NO:85 (H-CDR1), SEQ ID NO:86 (H-CDR2) and SEQ ID NO:87 (H-CDR3) and comprises a light chain antigen-binding region that comprises SEQ ID NO:88 (L-CDR1), SEQ ID NO:89 (L-CDR2) and SEQ ID NO:90 (L-CDR3).

In a more preferred embodiment the antibody of the disclosure or antigen-binding fragment thereof comprises a heavy chain antigen-binding region that comprises SEQ ID NO:95 (H-CDR1), SEQ ID NO:96 (H-CDR2) and SEQ ID NO:97 (H-CDR3) and comprises a light chain antigen-binding region that comprises SEQ ID NO:98 (L-CDR1), SEQ ID NO:99 (L-CDR2) and SEQ ID NO:100 (L-CDR3).

In a more preferred embodiment the antibody of the disclosure or antigen-binding fragment thereof comprises a heavy chain antigen-binding region that comprises SEQ ID NO:105 (H-CDR1), SEQ ID NO:106 (H-CDR2) and SEQ ID NO:107 (H-CDR3) and comprises a light chain antigen-binding region that comprises SEQ ID NO:108 (L-CDR1), SEQ ID NO:109 (L-CDR2) and SEQ ID NO:110 (L-CDR3).

In a more preferred embodiment the antibody of the disclosure or antigen-binding fragment thereof comprises a heavy chain antigen-binding region that comprises SEQ ID NO:115 (H-CDR1), SEQ ID NO:116 (H-CDR2) and SEQ ID NO: 117 (H-CDR3) and comprises a light chain antigen-binding region that comprises SEQ ID NO:118 (L-CDR1), SEQ ID NO:119 (L-CDR2) and SEQ ID NO:120 (L-CDR3).

In a more preferred embodiment the antibody of the disclosure or antigen-binding fragment thereof comprises a heavy chain antigen-binding region that comprises SEQ ID NO:125 (H-CDR1), SEQ ID NO:126 (H-CDR2) and SEQ ID NO: 127 (H-CDR3) and comprises a light chain antigen-binding region that comprises SEQ ID NO:128 (L-CDR1), SEQ ID NO:129 (L-CDR2) and SEQ ID NO:130 (L-CDR3).

In a more preferred embodiment the antibody of the disclosure or antigen-binding fragment thereof comprises a heavy chain antigen-binding region that comprises SEQ ID NO:135 (H-CDR1), SEQ ID NO:136 (H-CDR2) and SEQ ID NO:137 (H-CDR3) and comprises a light chain antigen-binding region that comprises SEQ ID NO:138 (L-CDR1), SEQ ID NO:139 (L-CDR2) and SEQ ID NO:140 (L-CDR3).

An antibody of the invention may be an IgG (e.g., IgG₁ IgG₂, IgG₃,IgG₄), while an antibody fragment may be a Fab, Fab', F(ab')₂ or scFv, for example. An inventive antibody fragment, accordingly, may be, or may contain, an antigen-binding region that behaves in one or more ways as described herein.

The invention also is related to isolated nucleic acid sequences, each of which can encode an aforementioned antibody or antigen-binding fragment thereof that is specific for an epitope of C4.4a. Nucleic acids of the invention are suitable for recombinant production of antibodies or antigen-binding antibody fragments. Thus, the invention also relates to vectors and host cells containing a nucleic acid sequence of the invention.

Compositions of the invention may be used for therapeutic or prophylactic applications. The invention, therefore, includes a pharmaceutical composition comprising an inventive antibody (or antigen-binding fragment thereof) and a pharmaceutically acceptable carrier or excipient therefore. In a related aspect, the invention provides a method for treating a disorder or condition associated with the undesired presence of C4.4a expressing cells. In a preferred embodiment the aforementioned disorder is cancer. Such method contains the steps of administering to a subject in need thereof an effective amount of the pharmaceutical composition that contains an inventive antibody as described or contemplated herein.

The disclosure also provides instructions for using an antibody library to isolate one or more members of such library that binds specifically to C4.4a.

### DESCRIPTION OF THE FIGURES

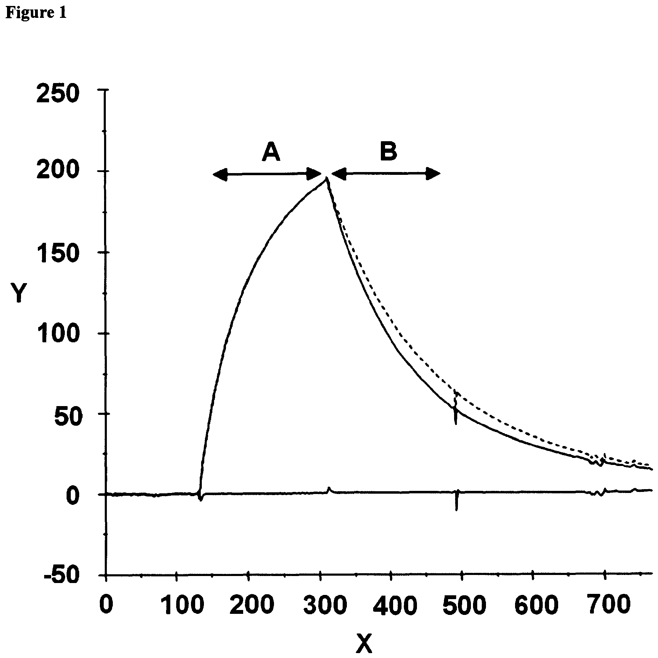

Figure 1 shows the result of an Epitope grouping experiment performed using sandwich surface plasmon resonance analysis. Y is resonance units X is time in seconds. One of the antibodies of the disclosure was coated to the chip. A indicates the time C4.4a was added. B indicates the time the other antibody of the disclosure was added. Both antibodies were unable to bind simultaneously to C4.4a indicating an at least overlapping epitope.

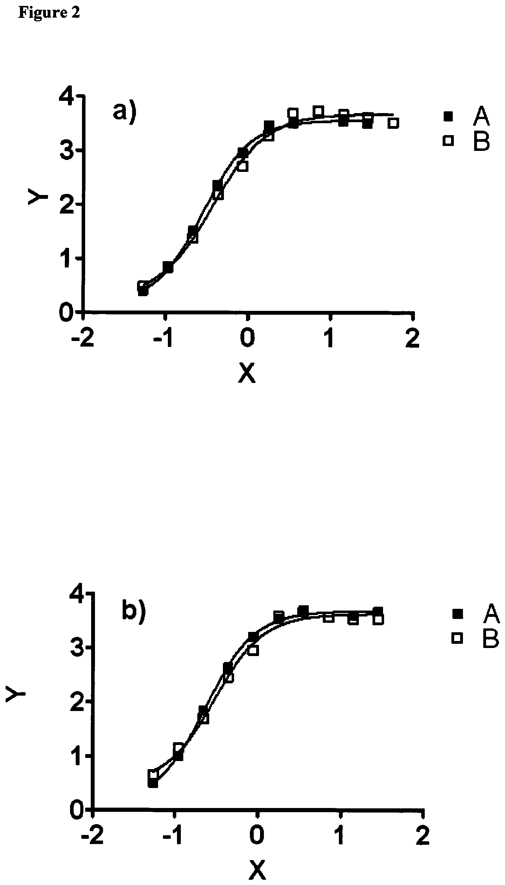

Figure 2 provides data on binding of anti C4.4a antibodies of the disclosure to recombinant human (a) and mouse (b) C4.4a in human IgG1 format. EC₅₀ values were determined by ELISA as described in Example 4. M31-B01 (A) is binding with an EC₅₀ of 0.24 and 0.29 nM to human and murine C4.4a respectively. M20 B02 S-A (B) is binding with an EC₅₀ of 0.3 and 0.38 nM to human and murine C4.4a respectively . X is log nM ; Y is Extinction at 360 nm.

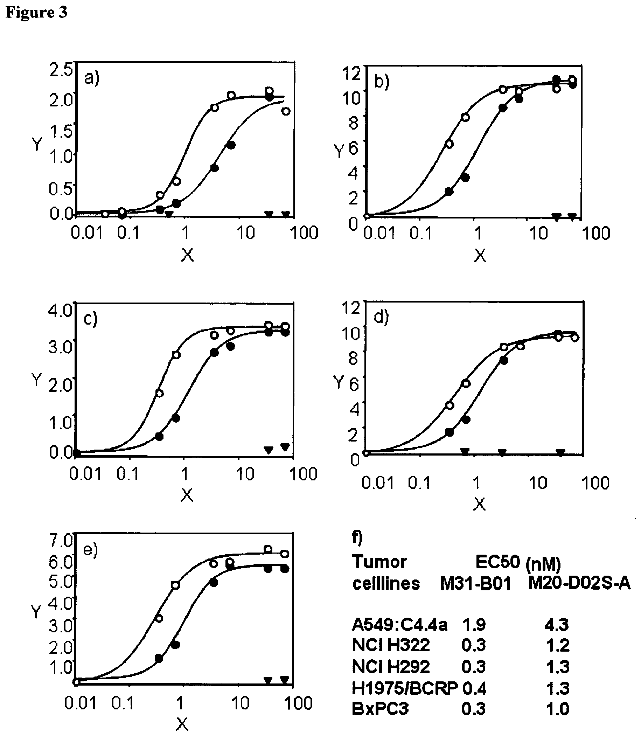

Figure 3 provides data on specific binding of antibodies of the disclosure to different tumor cell lines, either transfected with hC4.4a like A549:hC4.4a (a) or natively C4.4a expressing tumor cell lines as, NCI H322 (b) NCI H292 (c), H1975/BCRP (d) or BxPC3 (e). EC₅₀ values for binding of M31-B01 (open circles) and M20-D02 S-A (closed circles) are summarized in (f). The IgG1 isotype control (closed triangles) did not show any binding to the cells. All data was generated by FACS titration. X is concentration (log nM) and Y is geomean fluorescence (x10³).

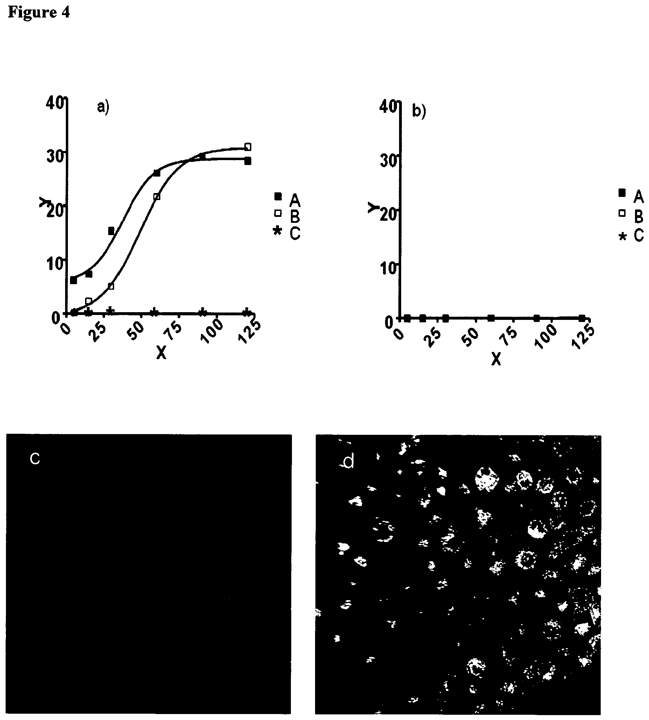

Figure 4(a) provides data on specific internalization of Fluorophor labeled-anti-C4.4a antibodies into A549:hC4.4a cells. Figure 4 (b) provides data on non transfected A549 cells as negative control. A is M31-B01 (closed squares); B is M20-D02 S-A (open squares); C is an isotype control for hIgG1 (stars); X is time in minutes and Y is the granule count/cell [*10³]. Both C4.4a antibodies are internalized specifically, efficiently and rapidly into C4.4a bearing tumor cells.
Fig 4 (c) and (d) provide data on internalization of M31-B01 into A549:hC4.4a cells. After 5 minutes (c) only a weak staining of the cell surface can be observed. After 90 min (d) intensively coloured endosomes containing the internalized M31-B01 antibody, which is carrying the pH dependent fluorescence dye, are clearly visible and indicate effective internalization.

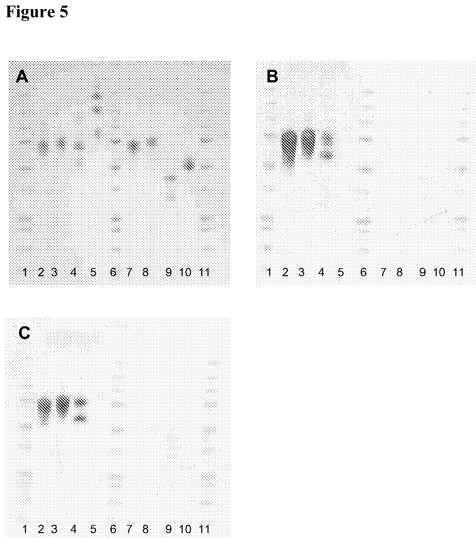

Figure 5 provides data on the epitope M31-B01 and M20-D02 S-A are binding to as determined by western blotting. A) shows a Coomassie 250 stained SDS-PAGE of different C4.4a species (lanes 2+7 hC4.4a , lanes 3+8 mC4.4a, lanes 4+9 hC4.4a domain S1-Fc-his6, lanes 5+10 hC4.4a domain S2-Fc-his6; lanes 1, 6 and 11 contain molecular weight markers; lanes 2-5 contain non reduced samples; lanes 6-10 contain reduced samples) B) and C) show the respective western blots with the identical sample load as in A). B) was incubated with M31-B01 and C) was incubated with M20-D02 S-A as detection antibody. Both antibodies show specific reduction dependent staining indicating a conformational epitope. However both bind to human and mouse full length C4.4a and to human C4.4a domain S1 but not to human C4.4a domain S2.

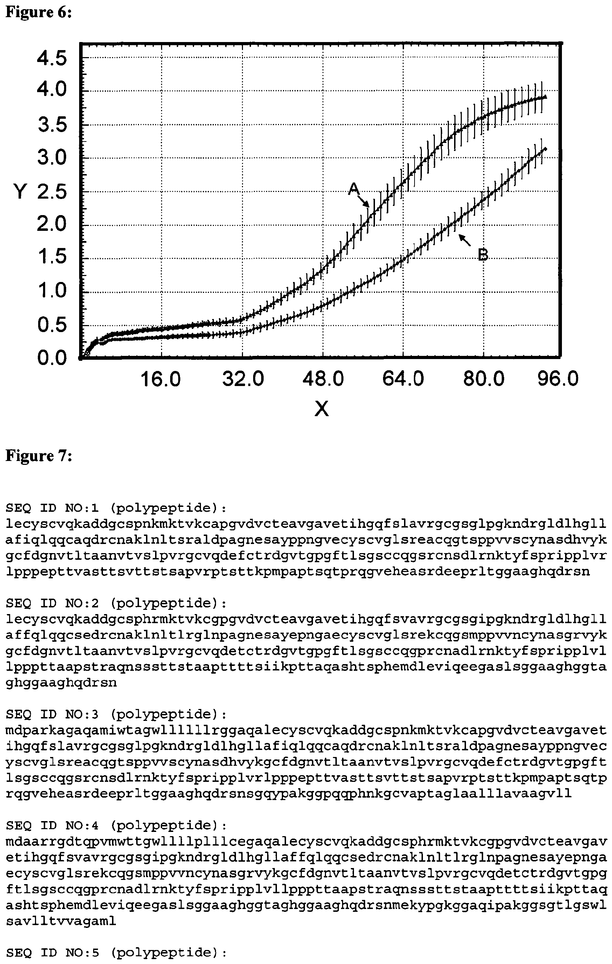

Figure 6 provides data on the inhibition of tumor cell proliferation in vitro by an antibody of the invention (B01-3) determined by measurement with an xCELLigence analyzer. A549:hC4.4a cells were either co-incubated with 100 nM of an non-binding hIgG1 isotype control antibody (A) or 100 nM of B01-3 (B) in single wells of an E-plate for the time indicated under conditions described in example 15. X is time in hours, Y is the relative rate of cell proliferation. A549:hC4.4a cells incubated with B01-3 show decreased cell proliferation relative to the control IgG1.

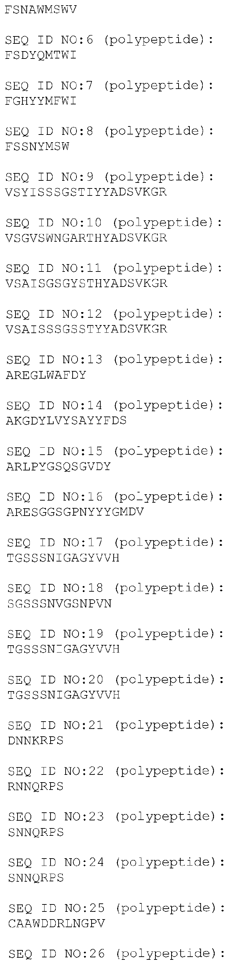

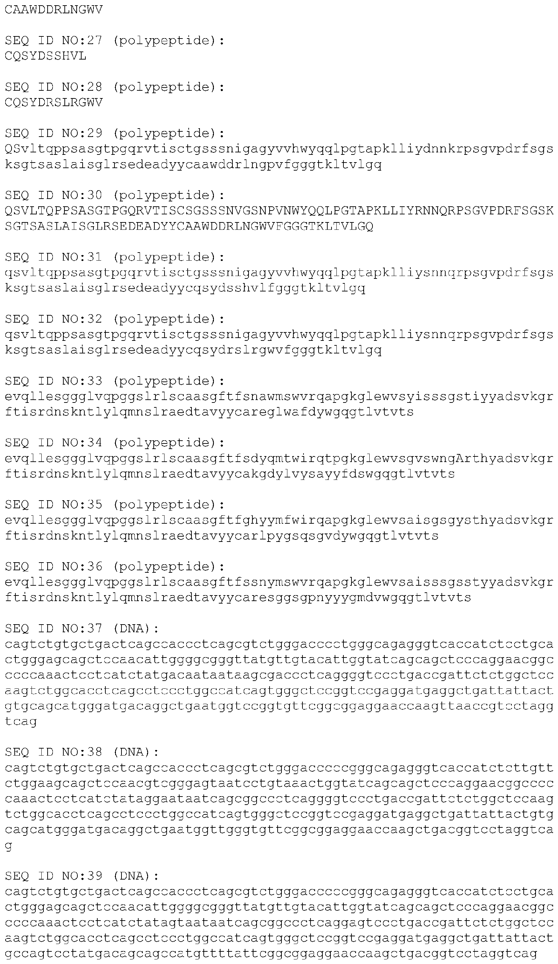

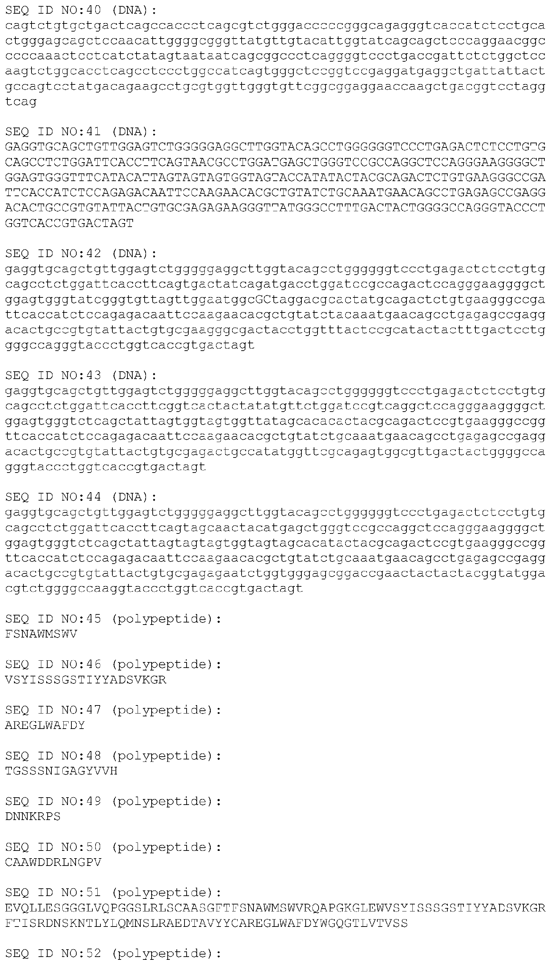

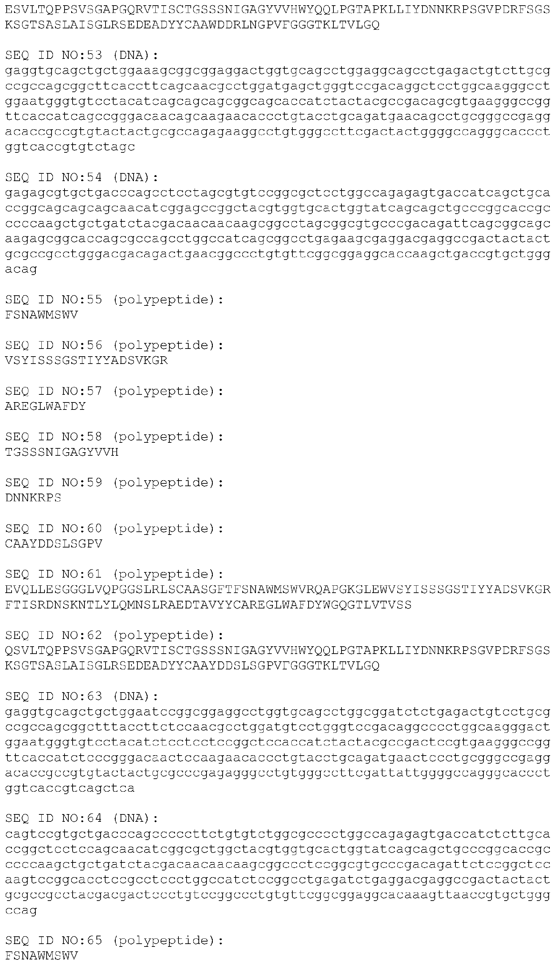

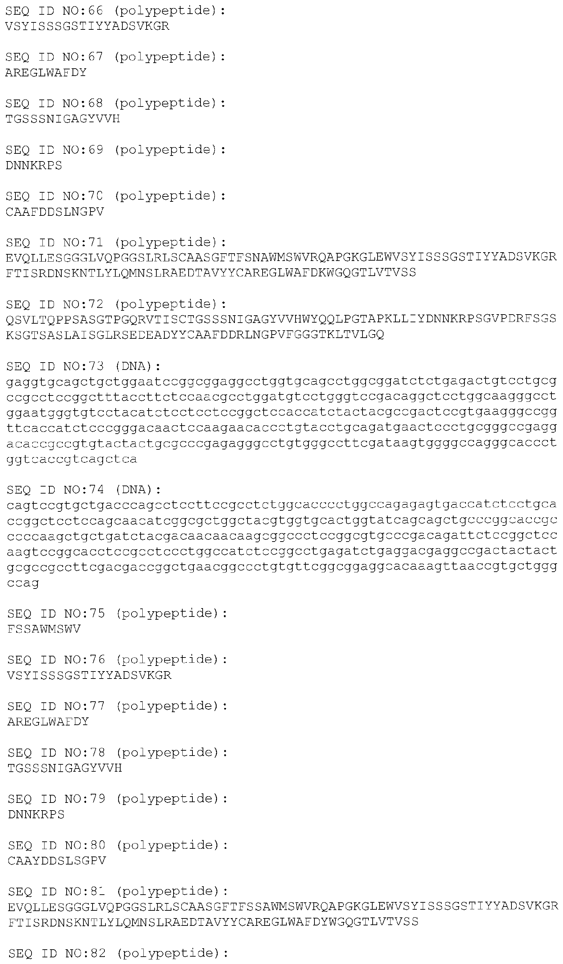

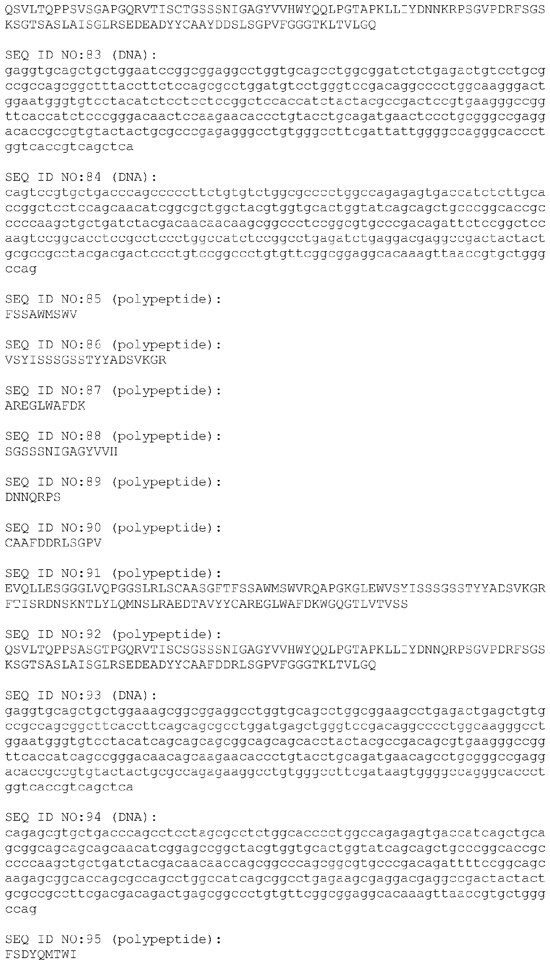

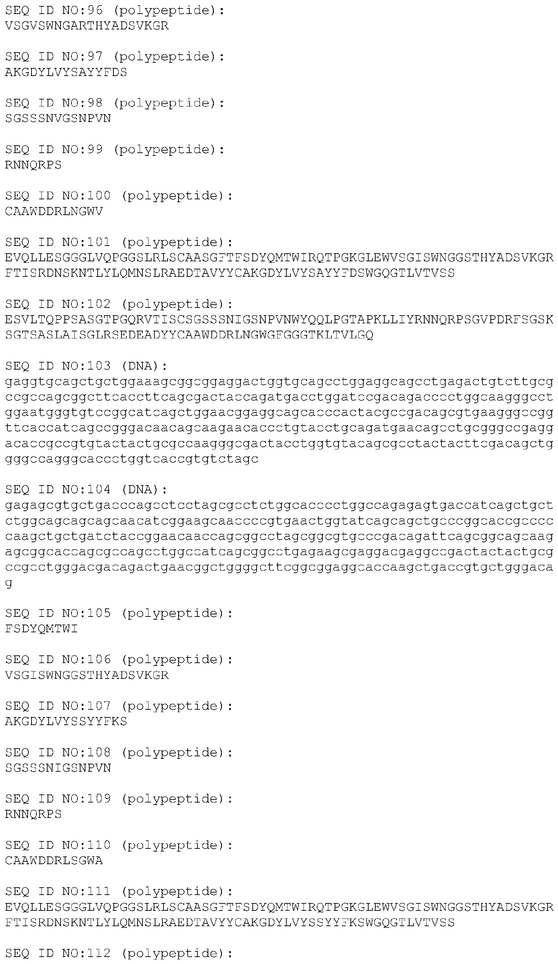

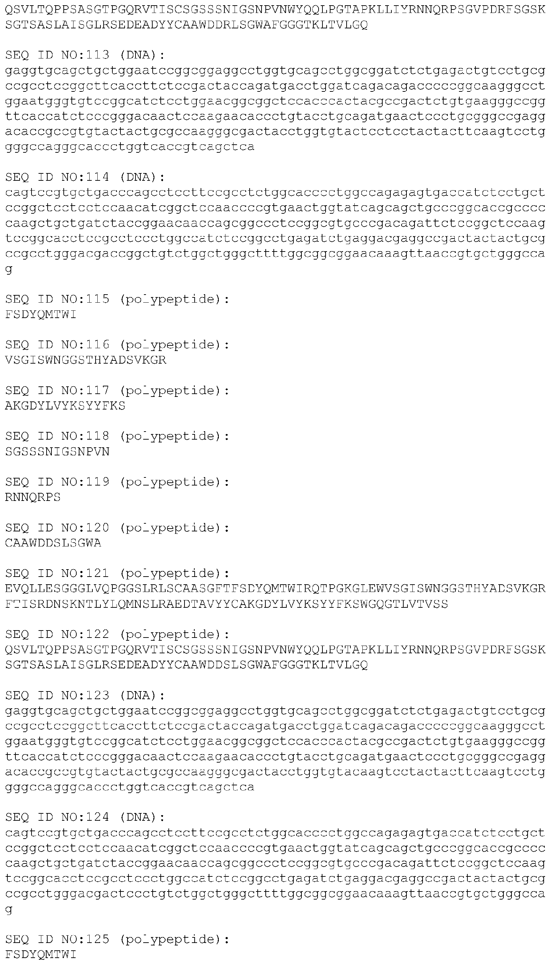

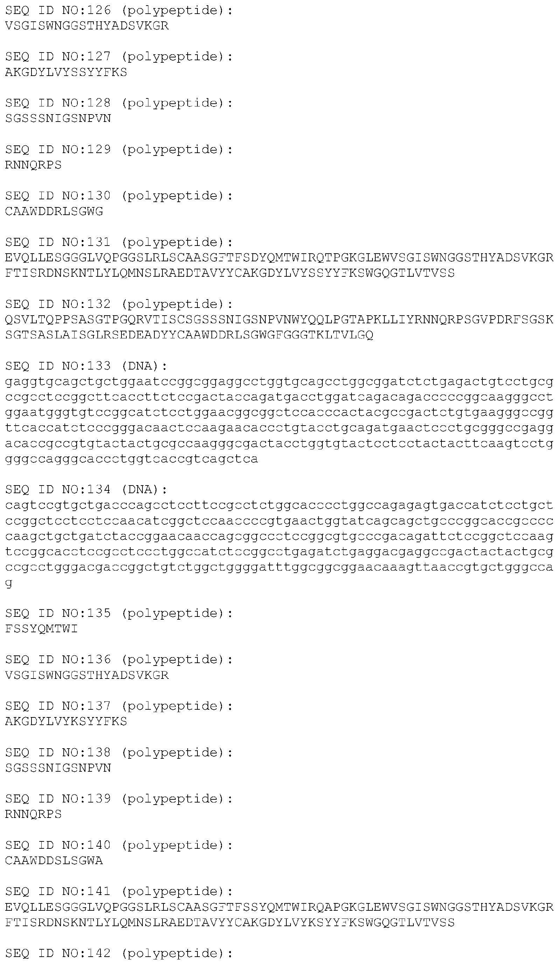

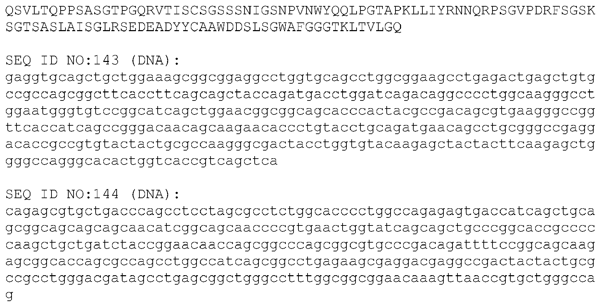

Figure 7 shows sequences of the disclosure.

### DETAILED DESCRIPTION OF THE INVENTION

The present invention is based on the discovery of novel antibodies that are specific to or have a high affinity for C4.4a and can deliver a therapeutic benefit to a subject. The antibodies of the invention, which may be human, humanized or chimeric, can be used in many contexts, which are more fully described herein.

### Definitions

A "human" antibody or antigen-binding fragment thereof is hereby defined as one that is not chimeric (*e.g.*, not "humanized") and not from (either in whole or in part) a non-human species. A human antibody or antigen-binding fragment thereof can be derived from a human or can be a synthetic human antibody. A "synthetic human antibody" is defined herein as an antibody having a sequence derived, in whole or in part, *in silico* from synthetic sequences that are based on the analysis of known human antibody sequences. *In silico* design of a human antibody sequence or fragment thereof can be achieved, for example, by analyzing a database of human antibody or antibody fragment sequences and devising a polypeptide sequence utilizing the data obtained there from. Another example of a human antibody or antigen-binding fragment thereof is one that is encoded by a nucleic acid isolated from a library of antibody sequences of human origin (*e.g.*., such library being based on antibodies taken from a human natural source). Examples of human antibodies include antibodies as described in Söderlind et al., Nature Biotech. 2000, 18:853-856.

A "humanized antibody" or humanized antigen-binding fragment thereof is defined herein as one that is (i) derived from a non-human source (*e.g.,* a transgenic mouse which bears a heterologous immune system), which antibody is based on a human germline sequence; (ii) where amino acids of the framework regions of a non human antibody are partially exchanged to human amino acid sequences by genetic engineering or (iii) CDR-grafted, wherein the CDRs of the variable domain are from a non-human origin, while one or more frameworks of the variable domain are of human origin and the constant domain (if any) is of human origin.

A "chimeric antibody" or antigen-binding fragment thereof is defined herein as one, wherein the variable domains are derived from a non-human origin and some or all constant domains are derived from a human origin.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. The term monoclonal antibody specifically includes chimeric, humanized and human antibodies.

As used herein, an antibody "binds specifically to", is "specific to/for" or "specifically recognizes" an antigen of interest, e.g. a tumor-associated polypeptide antigen target (here, C4.4a), is one that binds the antigen with sufficient affinity such that the antibody is useful as a therapeutic agent in targeting a cell or tissue expressing the antigen, and does not significantly cross-react with other proteins or does not significantly cross-react with proteins other than orthologs and variants (e.g. mutant forms, splice variants, or proteolytically truncated forms) of the aforementioned antigen target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent K_{D} for the antigen of less than about 10⁻⁴ M, alternatively less than about 10⁻⁵ M, alternatively less than about 10⁻⁶ M, alternatively less than about 10⁻⁷ M, alternatively less than about 10⁻⁸ M, alternatively less than about 10⁻⁹ M, alternatively less than about 10⁻¹⁰ M, alternatively less than about 10⁻¹¹ M, alternatively less than about 10⁻¹² M, or less. An antibody "binds specifically to," is "specific to/for" or "specifically recognizes" an antigen if such antibody is able to discriminate between such antigen and one or more reference antigen(s). In its most general form, "specific binding". "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to Western blots, ELISA-, RIA-, ECL-, IRMA-tests and peptide scans. For example, a standard ELISA assay can be carried out. The scoring may be carried out by standard color development (e.g. secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

"Binding affinity" refers to the strength of the sum total of noncovalent interactions between a single binding site of a molecule and its binding partner. Unless indicated otherwise, as used herein, "binding affinity" refers to intrinsic binding affinity which reflects a 1 : 1 interaction between members of a binding pair (e.g. an antibody and an antigen). The dissociation constant "K_{D}" is commonly used to describe the affinity between a molecule (such as an antibody) and its binding partner (such as an antigen) i.e. how tightly a ligand binds to a particular protein. Ligand-protein affinities are influenced by non-covalent intermolecular interactions between the two molecules Affinity can be measured by common methods known in the art, including those described herein. In one embodiment, the "K_{D}" or "K_{D} value" according to this invention is measured by using surface plasmon resonance assays using a Biacore T100 instrument (GE Healthcare Biacore, Inc.) according to Example 3. In brief, antibodies were immobilized onto a CM5 sensor chip through an indirect capturing reagent, anti-human IgG Fc. Reagents from the "Human Antibody Capture Kit" (BR-1008-39, GE Healthcare Biacore, Inc.) were used as described by the manufacturer. Approximately 5000 resonance units (RU) monoclonal mouse anti-human IgG (Fc) antibody were immobilized per cell. Anti C4.4 antibodies were injected to reach a capturing level of approximately 200 to 600 RU. Various concentrations of human or murine C4.4a were injected over immobilized anti-C4.4a antibodies. Sensograms were generated after in-line reference cell correction followed by buffer sample subtraction. The dissociation equilibrium constant (K_{D}) was calculated based on the ratio of association (kₒₙ) and dissociation rated (k_{off}) constants, obtained by fitting sensograms with a first order 1:1 binding model using Biacore Evaluation Software. Other suitable devices are BIACORE(R)-2000, a BIACORE (R)-3000 (BIAcore, Inc., Piscataway, NJ), or ProteOn XPR36 instrument (Bio-Rad Laboratories, Inc.).

The term "antibody", as used herein, is intended to refer to immunglobulin molecules, preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g. three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs. arranged from amino terminus to carboxy-terminus e.g. in the following order: FR1, CDR1, FR2, CDR2, FR3, CDR3, FR4.
As used herein, the term "Complementarity Determining Regions (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immulological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26- 32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop. Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA, and IgA2. The heavy-chain constant domains that correspond to the different classes of antibodies are called [alpha], [delta], [epsilon], [gamma], and [mu], respectively. The subunit structures and three-dimensional configurations of different classes of immunglobulins are well known. As used herein antibodies are conventionally known antibodies and functional fragments thereof.
A "functional fragment" or "antigen-binding antibody fragment" of an antibody/immunoglobulin hereby is defined as a fragment of an antibody/immunoglobulin (e.g., a variable region of an IgG) that retains the antigen-binding region. An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320). A preferred class of immunoglobulins for use in the present invention is IgG.
"Functional fragments" or "antigen-binding antibody fragments" of the invention include Fab, Fab', F(ab')₂, and Fv fragments; diabodies; single domain antibodies (DAbs), linear antibodies; single-chain antibody molecules (scFv); and multispecific, such as bi- and tri-specific, antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). An antibody other than a "multi-specific" or "multi-functional" antibody is understood to have each of its binding sites identical. The F(ab')₂ or Fab may be engineered to minimize or completely remove the intermolecular disulphide interactions that occur between the C_{H1} and C_{L} domains.

An antibody of the invention may be derived from a recombinant antibody library that is based on amino acid sequences that have been isolated from the antibodies of a large number of healthy volunteers. Using the n-CoDeR^{®} technology the fully human CDRs are recombined into new antibody molecules. The unique recombination process allows the library to contain a wider variety of antibodies than could have been created naturally by the human immune system.

As used herein, different 'forms' of antigen, e.g. C4.4a are hereby defined as different protein molecules resulting from different translational and posttranslational modifications, such as, but not limited to, differences in splicing of the primary C4.4a transcript, differences in glycosylation, and differences in posttranslational proteolytic cleavage.
As used herein, the term 'epitope' includes any protein determinant capable of specific binding to an immunoglobulin or T-cell receptors. Epitopic determinants usually consist of chemically active surface groupings of molecules such as amino acids or sugar side chains, or combinations thereof and usually have specific three dimensional structural characteristics, as well as specific charge characteristics. Two antibodies are said to 'bind the same epitope' if one antibody is shown to compete with the second antibody in a competitive binding assay, by any of the methods well known to those of skill in the art.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or nonproteinaceous solutes. In preferred embodiments, the antibody is purified (1) to greater than 95% by weight of antibody as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or nonreducing conditions using Coomassie blue or, preferably, silver stain. Isolated naturally occurring antibody includes the antibody in situ within recombinant cells since at least one component of the antibody's natural environment will not be present. Ordinarily, however, isolated antibody will be prepared by at least one purification step.

"Antibody-dependent cell-mediated cytotoxicity" or "ADCC" refers to a form of cytotoxicity in which secreted Ig bound onto Fc gamma receptors (FcγRs) present on certain cytotoxic cells (e.g. NK cells, neutrophils, and macrophages) enable these cytotoxic effector cells to bind specifically to an antigen-bearing target cell and subsequently kill the target cell e.g. with cytotoxins. To assess ADCC activity of an antibody of interest, an in vitro ADCC assay, such as that described in US Patent No. 5,500,362 or 5,821,337 or U.S. Patent No. 6,737,056 (Presta), may be performed. Useful effector cells for such assays include PBMC and NK cells.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, e.g., as described in Gazzano-Santoro et al., J. Immunol. Methods 202: 163 (1996), may be performed. Polypeptide variants with altered Fc region amino acid sequences (polypeptides with a variant Fc region) and increased or decreased C1q binding are described, e.g., in US Patent No. 6,194,551 B1 and WO 1999/51642.

The term immunoconjugate (interchangeably referred to as "antibody-drug conjugate," or "ADC") refers to an antibody conjugated to one or more cytotoxic agents, such as a chemotherapeutic agent, a drug, a growth inhibitory agent, a toxin (e.g., a protein toxin, a enzymatically active toxin of bacterial, fungal, plant, or animal origin, or fragments thereof), or a radioactive isotope (i.e., a radioconjugate). Immunoconjugates have been used for the local delivery of cytotoxic agents, i.e., drugs that kill or inhibit the growth or proliferation of cells, in the treatment of cancer (e.g. Liu et al., Proc Natl. Acad. Sci. (1996), 93, 8618-8623)). Immunoconjugates allow for the targeted delivery of a drug moiety to a tumor, and intracellular accumulation therein, where systemic administration of unconjugated drugs may result in unacceptable levels of toxicity to normal cells and/or tissues. Toxins used in antibody-toxin conjugates include bacterial toxins such as diphtheria toxin, plant toxins such as ricin, small molecule toxins such as geldanamycin. The toxins may exert their cytotoxic effects by mechanisms including tubulin binding, DNA binding, or topoisomerase inhibition.

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are un-gapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

### Antibodies of the Invention

The present invention relates to methods to inhibit growth of C4.4a-positive cancer cells and the progression of neoplastic disease by providing anti-C4.4a antibodies. Provided are antibodies, antigen-binding antibody fragments thereof, and variants of the antibodies and fragments, that specifically bind to the 29 kDa, human C4.4a polypeptide, (SEQ ID NO: 1 or fragments thereof). The antibodies, antigen-binding fragments or variants thereof bind specifically to the extracellular domain S1 of the C4.4a polypeptide. The C4.4a polypeptide is named 'C4.4a' herein.

The antibodies or antigen-binding fragments thereof are internalized into a C4.4a expressing cell upon binding of the antibody or antigen-binding fragment thereof to the aforementioned cell. In a further preferred embodiment of this disclosure the antibodies or antigen-binding fragments thereof compete in binding to C4.4a with the antibodies M31-B01 or M20-D02 S-A. In a further preferred embodiment of this disclosure the antibodies or antigen-binding fragments thereof compete in binding to human C4.4a with the antibodies M31-B01 or M20-D02 S-A. In a further preferred embodiment of this disclosure the antibodies or antigen-binding fragments thereof compete in binding to human and rodent C4.4a with the antibodies M31-B01 or M20-D02 S-A, a further preferred embodiment is wherein the rodent C4.4a is mouse C4.4a.

In a further preferred embodiment of this disclosure the antibodies or antigen-binding fragments thereof comprise heavy or light chain CDR sequences which are at least 50%, 55%, 60% 70%, 80%, 90%, or 95% identical to at least one, preferably corresponding, CDR sequence as depicted in table 7, or which comprise variable heavy or light chain sequences which are at least 50%, 60%, 70%, 80%, 90%, 92% or 95% identical to a VH or VL sequence depicted in table 7, respectively.

In a further preferred embodiment of this disclosure the antibodies or antigen-binding fragments thereof comprise heavy and/or light chain CDR sequences which are at least 50%, 55%, 60% 70%, 80%, 90%, or 95% identical to at least one, preferably corresponding, CDR sequence of the antibodies M31-B01 or M20-D02 S-A, respectively.

In a further preferred embodiment of this disclosure the antibodies or antigen-binding fragments thereof comprise heavy and/or light chain CDR sequences which are at least 50%, 55%, 60% 70%, 80%, 90%, or 95% identical to the, preferably corresponding, heavy and/or light chain CDR sequences of the antibodies M31-B01 or M20-D02 S-A, respectively.

In a further preferred embodiment of this disclosure the antibodies or antigen-binding fragments thereof comprise heavy chain CDR2 and -3 sequences which are at least 50%, 55%, 60% 70%, 80%, 90%, or 95% identical to the heavy chain CDR2 and -3 sequences and light chain CDR1 and -3 sequences which are at least 50%, 55%, 60% 70%, 80%, 90%, or 95% identical to the light chain CDR1 and -3 sequences of the antibodies M31-B01. In a further preferred embodiment of this disclosure the antibodies or antigen-binding fragments thereof comprise heavy chain CDR2 and -3 sequences which are at least 50%, 55%, 60% 70%, 80%, 90%, or 95% identical to the heavy chain CDR2 and -3 sequences and light chain CDR1 and -3 sequences which are at least 50%, 55%, 60% 70%, 80%, 90%, or 95% identical to the light chain CDR1 and -3 sequences of the antibodies M20-D02 S-A.

In a further preferred embodiment of this disclosure the antibodies or antigen-binding fragments thereof comprise a variable heavy chain sequence which is at least 50%, 60%, 70%, 80%, 90%, 92% or 95% identical to a VH sequence disclosed in table 7 or table 4, preferably of the antibodies M31-B01 or M20-D02 S-A,. In a further preferred embodiment of this disclosure the antibodies or antigen-binding fragments thereof comprise a variable light chain sequence which is at least 50%, 60%, 70%, 80%, 90%, 92% or 95% identical to a VL sequence disclosed in table 7 or table 3, preferably of the antibodies M31-B01 or M20-D02 S-A.

In a further preferred embodiment of this disclosure the antibodies or antigen-binding fragments thereof comprise variable heavy and light chain sequences that are at least 50%, 60%, 70%, 80%, 90%, 92% or 95% identical to the VH and VL sequence of the antibodies M31-B01 or M20-D02 S-A, respectively.

In a further preferred embodiment of this disclosure the antibodies or antigen-binding fragments thereof comprise heavy and light chain CDR sequences which conform to the M31-B01 or M20-D02 S-A derived, preferably corresponding, CDR consensus sequences as depicted in table 15. A further preferred embodiment of this disclosure are antibodies or antigen-binding fragments thereof comprising heavy chain CDR sequences conforming to the corresponding heavy chain CDR sequences as represented by the consensus sequences SEQ ID NO: 297 (CDR H1), SEQ ID NO: 298 (CDR H2) and SEQ ID NO: 299 (CDR H3), and light chain CDR sequences conforming to the corresponding light chain CDR sequences as represented by the consensus sequences SEQ ID NO: 300 (CDR L1), SEQ ID NO: 22 (CDR L2) and SEQ ID NO: 301 (CDR L3), or comprising heavy chain CDR sequences conforming to the corresponding heavy chain CDR sequences as represented by the consensus sequences SEQ ID NO: 302 (CDR H1), SEQ ID NO: 303 (CDR H2) and SEQ ID NO: 304 (CDR H3), and light chain CDR sequences conforming to the corresponding light chain CDR sequences as represented by the consensus sequences SEQ ID NO: 305 (CDR L1), SEQ ID NO: 306 (CDR L2) and SEQ ID NO: 307 (CDR L3).

In a further preferred embodiment of this disclosure the antibodies or antigen-binding antibody fragments comprise at least one, preferably corresponding, heavy and/or light chain CDR sequence as disclosed in table 7 or table 3 and 4, or preferably of an antibody as depicted in table 7 or table 3 and 4. In a further preferred embodiment of this disclosure the antibodies or antigen-binding antibody fragments comprise at least one, two, three, four, five or six, preferably corresponding, heavy and light chain CDR sequences as disclosed in table 7 or table 3 and 4, or preferably of an antibody as depicted in table 7 or table 3 and 4. In a further preferred embodiment the antibodies or antigen-binding antibody fragments comprise the heavy or light chain CDR1, CDR2 or CDR3 sequences of an antibody as depicted in table 7 or table 3 and 4, the heavy or light chain CDR1 and CDR2 sequences of an antibody as depicted in table 7 or table 3 and 4, the heavy or light chain CDR1 and CDR3 sequences of an antibody as depicted in table 7 or table 3 and 4, the heavy or light chain CDR2 and CDR3 sequences of an antibody as depicted in table or table 3 and 4, the heavy or light chain CDR1, CDR2 and CDR3 sequences of an antibody as depicted in table or table 3 and 4. In a further preferred embodiment of this disclosure the antibodies or antigen-binding antibody fragments comprise the heavy chain CDR sequences CDR1 and CDR2 and the light chain CDR sequences CDR1, CDR2, CDR3 of an antibody as depicted in table 7 or table 3 and 4. In a further preferred embodiment of this disclosure the antibodies or antigen-binding antibody fragments comprise the heavy and light chain CDR1, CDR2 or CDR3 sequences of an antibody as depicted in table 7 or table 3 and 4, the heavy and light chain CDR1 and CDR2 sequences of an antibody as depicted in table or table 3 and 4, the heavy and light chain CDR1 and CDR3 sequences of an antibody as depicted in table 7 or table 3 and 4, the heavy and light chain CDR2 and CDR3 sequences of an antibody as depicted in table 7 or table 3 and 4, the heavy and light chain CDR1, CDR2 and CDR3 sequences of an antibody as depicted in table 7 or table 3 and 4. In a further preferred embodiment of this disclosure the antibodies or antigen-binding antibody fragments comprise the heavy and light chain CDR sequences of an antibody as depicted in table 7 or table 3 and 4.

In a further embodiment of this disclosure the antibodies or antigen-binding antibody fragments comprise a VH and/or VL sequence disclosed in table 7 or table 3 and 4,. In a further preferred embodiment of this disclosure the antibodies or antigen-binding antibody fragments comprise the VH and VL sequence of an antibody depicted in table 7 or table 3 and 4.

In a preferred embodiment the antibodies or antigen-binding antibody fragments of the invention are monoclonal. In a further preferred embodiment the antibodies or antigen-binding antibody fragments of the invention are human, humanized or chimeric.

Variants of the antibodies or antigen-binding antibody fragments contemplated in the disclosure are molecules in which the binding activity of the antibody or antigen-binding antibody fragment for C4.4a is maintained. In a preferred embodiment of this disclosure the variants compete in binding to C4.4a with an antibody depicted in table 7, preferably with antibody M31-B01 or M20-D02 S-A.

Throughout this document, reference is made to the following preferred antibodies: "M31-B01", "M20-D02 S-A", "M60-G03" and "M36-H02", "B01-3", "B01-5", "B01-7", "B01-10", "B01-12", "D02-4", "D02-6", "D02-7", "D02-11" and "D02-13".

M31-B01 represents an antibody comprising a variable heavy chain region corresponding to SEQ ID NO: 41 (DNA)/SEQ ID NO: 33 (protein) and a variable light chain region corresponding to SEQ ID NO: 37 (DNA)/SEQ ID NO: 29 (protein).

M20-D02 S-A represents an antibody comprising a variable heavy chain region corresponding to SEQ ID NO: 42 (DNA)/SEQ ID NO: 34 (protein) and a variable light chain region corresponding to SEQ ID NO: 38 (DNA)/SEQ ID NO: 30 (protein).

M60-DG03 represents an antibody comprising a variable heavy chain region corresponding to SEQ ID NO: 43 (DNA)/SEQ ID NO: 35 (protein) and a variable light chain region corresponding to SEQ ID NO: 39 (DNA)/SEQ ID NO: 31 (protein).

M36-H02 represents an antibody comprising a variable heavy chain region corresponding to SEQ ID NO: 44 (DNA)/SEQ ID NO: 36 (protein) and a variable light chain region corresponding to SEQ ID NO: 40 (DNA)/SEQ ID NO: 32 (protein).

B01-3 represents an antibody comprising a variable heavy chain region corresponding to SEQ ID NO: 53 (DNA)/SEQ ID NO: 51 (protein) and a variable light chain region corresponding to SEQ ID NO: 54 (DNA)/SEQ ID NO: 52 (protein).

B01-5 represents an antibody comprising a variable heavy chain region corresponding to SEQ ID NO: 63 (DNA)/SEQ ID NO: 61 (protein) and a variable light chain region corresponding to SEQ ID NO: 64 (DNA)/SEQ ID NO: 62 (protein).

B01-7 represents an antibody comprising a variable heavy chain region corresponding to SEQ ID NO: 73 (DNA)/SEQ ID NO: 71 (protein) and a variable light chain region corresponding to SEQ ID NO: 74 (DNA)/SEQ ID NO: 72 (protein).

B01-10 represents an antibody comprising a variable heavy chain region corresponding to SEQ ID NO: 83 (DNA)/SEQ ID NO: 81 (protein) and a variable light chain region corresponding to SEQ ID NO: 84 (DNA)/SEQ ID NO: 82 (protein).

B01-12 represents an antibody comprising a variable heavy chain region corresponding to SEQ ID NO: 93 (DNA)/SEQ ID NO: 91 (protein) and a variable light chain region corresponding to SEQ ID NO: 94 (DNA)/SEQ ID NO: 92 (protein).

D02-4 represents an antibody comprising a variable heavy chain region corresponding to SEQ ID NO: 103 (DNA)/SEQ ID NO: 101 (protein) and a variable light chain region corresponding to SEQ ID NO: 104 (DNA)/SEQ ID NO: 102 (protein).

D02-6 represents an antibody comprising a variable heavy chain region corresponding to SEQ ID NO: 113 (DNA)/SEQ ID NO: 111 (protein) and a variable light chain region corresponding to SEQ ID NO: 114 (DNA)/SEQ ID NO: 112 (protein).

D02-7 represents an antibody comprising a variable heavy chain region corresponding to SEQ ID NO: 123 (DNA)/SEQ ID NO: 121 (protein) and a variable light chain region corresponding to SEQ ID NO: 124 (DNA)/SEQ ID NO: 122 (protein).

D02-11 represents an antibody comprising a variable heavy chain region corresponding to SEQ ID NO: 133 (DNA)/SEQ ID NO: 131 (protein) and a variable light chain region corresponding to SEQ ID NO: 134 (DNA)/SEQ ID NO: 132 (protein).

D02-13 represents an antibody comprising a variable heavy chain region corresponding to SEQ ID NO: 143 (DNA)/SEQ ID NO: 141 (protein) and a variable light chain region corresponding to SEQ ID NO: 144 (DNA)/SEQ ID NO: 142 (protein).

In another aspect, the disclosure provides antibodies or antigen-binding fragments having an antigen-binding region that bind specifically to and/or has a high affinity for one or more regions of C4.4a, whose amino acid sequence is depicted by SEQ ID NO: 1. An antibody or antigen-binding fragment is said to have a "high affinity" for an antigen if the affinity measurement is less than 250 nM (monovalent affinity of the antibody or antigen-binding fragment). A disclosed antibody or antigen-binding region preferably can bind to human C4.4a with an affinity of less than 250 nM, preferably less than 100 nM, more preferably less than 25 nM and even more preferable with less than 11 nM determined as monovalent affinity to human C4.4a. For instance, the affinity of an antibody of the disclosure against C4.4a may be about 220.0 nM or 1 nM (monovalent affinity of the antibody or antigen-binding fragment).

Table 1 provides a summary of dissociation constants of representative antibodies of the disclosure, as determined by surface plasmon resonance (Biacore) on directly immobilized human or murine C4.4a.

**Table 1: Monovalent dissociation constants determined for anti-C4.4a IgG1 by surface plasmon resonance**

| | Human C4.4a | Mouse C4.4a |
|---|---|---|
| Antibody (IgG1) | K_{D} [M] | K_{D} [M] |
| M31-B01 | *7.0 x 10⁻⁸* | *1.3 x 10⁻⁷* |
| M20-D02 S-A | *2.2 x 10⁻⁷* | *1.8 x 10⁻⁷* |
| B01-3 | *6.0 x 10⁻⁸* | *1.2 x 10⁻⁷* |
| B01-5 | *4 x 10⁻⁹* | *1 x 10⁻⁸* |
| B01-7 | *7 x 10⁻⁹* | *9 x 10⁻⁹* |
| B01-10 | *4 x 10⁻⁹* | *1 x 10⁻⁹* |
| B01-12 | *1 x 10⁻⁹* | *2 x 10⁻⁹* |
| D02-4 | *2.9 x 10⁻⁸* | *5.6 x 10⁻⁸* |
| D02-6 | *6 x 10⁻⁹* | *2.1 x 10⁻⁸* |
| D02-7 | *9 x 10⁻⁹* | *2.2 x 10⁻⁸* |
| D02-11 | *1 x 10⁻⁸* | *2.4x 10⁻⁸* |
| D02-13 | *2 x 10⁻⁹* | *4.2 x 10⁻⁸* |

The IgG1 format was used for the cell-based affinity determination by fluorescence-activated cell sorting (FACS) combined with Scatchard analysis. figure 3 f) denotes the binding strength of representative IgG antibodies on transfected C4.4a-expressing A549 tumor cells and endogenously C4.4a expressing tumor cells. Table 8 provides a summary of the binding strength (EC₅₀) of representative IgG antibodies on transfected murine CHO-S:mC4.4a cells and endogenously C4.4a expressing NCI H292 tumor cells.

**Table 8: EC₅₀ values determined for anti-C4.4a IgG1 by FACS**

| | Mouse C4.4a:CHO | NCI H292 |
|---|---|---|
| Antibody (IgG1) | EC₅₀ [M] | EC₅₀ [M] |
| M31-B01 | *1.9 x 10⁻⁹* | *3 x 10⁻¹⁰* |
| M20-D02 S-A | *1.6 x 10⁻⁹* | *1.3 x 10⁻⁹* |
| B01-3 | *3.6 x 10⁻¹⁰* | *6 x 10⁻¹¹* |
| B01-5 | *1.2 x 10⁻⁹* | *5 x 10⁻¹¹* |
| B01-7 | *5.7 x 10⁻¹⁰* | *6 x 10⁻¹¹* |
| B01-10 | *4.3 x 10⁻¹⁰* | *1 x 10⁻¹⁰* |
| B01-12 | *1.4 x 10⁻⁹* | *Nd* |
| D02-6 | *1 x 10⁻⁹* | *2 x 10⁻¹¹* |
| D02-7 | *7.8 x 10⁻¹⁰* | *3 x 10⁻¹¹* |
| D02-11 | *1.7 x 10⁻⁹* | *1 x 10⁻¹¹* |
| D02-13 | *2 x 10⁻⁹* | *1.2 x 10⁻¹⁰* |

| | | |
|---|---|---|
| nd= not determined | | |

An IgG1 is said to have a "high affinity" for an antigen if the affinity measurement measured by FACS is less than 100 nM (apparent affinity of IgG). A disclosed bivalent antibody or antigen-binding fragment preferably can bind to human C4.4a with an affinity of less than 100 nM, more preferably less than 50 nM, and still more preferably less than 10 nM. Further preferred are bivalent antibodies that bind to C4.4a with an affinity of less than 5 nM, and more preferably less than 1 nM determined as aparent affinity of an IgG to human C4.4a. For instance, the apparent affinity of an antibody of the disclosure against C4.4a may be about 4.3 nM or 0.03 nM on different tumor cell lines as determined by FACS analysis as depicted in figure 3 f.

An antibody or antigen-binding fragment of the disclosure internalizes "efficiently" when its time of half maximal internalization (t ½) into C4.4a expressing tumor cells is shorter than 180 min or more preferably shorter than 120 min and still more preferably shorter than 90 min. Further preferred are antibodies or antigen-binding fragments with half maximal internalization times (t ½) of 60 minutes or less as determined by the protocol described in example 6, further preferred are less than 50 minutes, or less than 35 minutes. Table 9 provides a summary of internalization times of representative antibodies of the disclosure, as determined by the protocol described in example 6. Internalizable antibodies or antigen-binding fragments of the disclosure are suitable as targeting moiety of an antibody-drug conjugate (ADC). An antibody or antigen-binding fragment is suitable in an in vitro or in vivo method to deliver a compound, preferably a cytotoxic agent, into a C4.4a expressing cell.

**Table 9:**

| | interalization |
|---|---|
| Antibody (IgG1) | t ½ [min] |
| M31-B01 | 49 |
| M20-D02 S-A | 60 |
| B01-3 | *55* |
| B01-7 | *33* |
| B01-10 | *30* |
| D02-6 | *39* |
| D02-7 | *33* |
| D02-11 | *22* |

In some embodiments, the antibody, antigen-binding fragment thereof, or derivative thereof or nucleic acid encoding the same is isolated. An isolated biological component (such as a nucleic acid molecule or protein such as an antibody) is one that has been substantially separated or purified away from other biological components in the cell of the organism in which the component naturally occurs, e.g., other chromosomal and extra-chromosomal DNA and RNA, proteins and organelles. Nucleic acids and proteins that have been "isolated" include nucleic acids and proteins purified by standard purification methods Sambrook et al., 1989 (Sambrook, J., Fritsch, E. F. and Maniatis, T. (1989) Molecular Cloning: A laboratory manual, Cold Spring Harbor Laboratory Press, Cold Spring Harbor, USA) and Robert K. Scopes eat al 1994 Protein Purification, - Principles and Practice, Springer Science and Business Media LLC. The term also embraces nucleic acids and proteins prepared by recombinant expression in a host cell as well as chemically synthesized nucleic acids.

### Antibody Generation

A fully human N-CoDeR antibody phage display library was used to isolate high affinity, C4.4a-specific, human monoclonal antibodies by a combination of whole cell and protein panning and through the development of specific tools. These tools and methods include a human C4.4a-expressing recombinant cell-line, a murine C4.4a expressing cell line, recombinant human and murine C4.4a and the development of panning procedures and screening assays capable of identifying antibodies that preferentially bind to C4.4a displayed on the cell surface and that are cross-reactive to murine C4.4a.

Antibodies to the cancer cell-surface marker C4.4a were developed by a combination of three non-conventional approaches in phage-display technology (PDT). First, recombinant cell lines expressing the membrane-bound form of human and mouse C4.4a were constructed by stable transfection of CHO-S cells and A549 tumor cells with a plasmid encoding the full length GPI-anchored form of the human or mouse protein (SEQ ID NO:3) and (SEQ ID NO:4) respectively, to give the human CHO-S:hC4.4a, the murine CHO-S:mC4.4a and the human A549:hC4.4a cell lines, respectively. Second, cell-surface selections were performed with the latter recombinant cell lines and the breast cancer cell line MCF-7. Pre-adsorption with CHO-S cells or non transfected A549 cells was included to avoid the selection of Fab fragments binding to epitopes of the parental cells. Additional selections were performed with recombinant, soluble, purified human C4.4a, with recombinant, soluble, purified murine C4.4a. Third, screening methods were developed which allowed for successive screening of the phage outputs obtained in panning on whole A549:hC4.4a cells as well as CHO-S:hC4.4a cells. The combination of these specific methods allowed the isolation of the unique antibodies "M31-B01", "M20-D02 S-A", "M60-G03", and, "M36-H02".

These unique antibodies were further characterized by their binding affinity in ELISA's, by BIAcore binding to soluble C4.4a, by their ability to recognize different epitopes on soluble C4.4a and by their ability to cross react with murine C4.4a assessed by BIAcore and FACS, and their ability to be internalized in a cell based assay. The internalization assays quantitatively measured the internalization of fluorescently labeled anti-C4.4a antibodies in a time resolved manner.

### Peptide Variants

Antibodies or antigen-binding fragments of the disclosure are not limited to the specific peptide sequences provided herein. Rather, the disclosure also embodies variants of these polypeptides. With reference to the instant disclosure and conventionally available technologies and references, the skilled worker will be able to prepare, test and utilize functional variants of the antibodies disclosed herein.

A variant can include, for example, an antibody that has at least one altered complementary determining region (CDR) (hyper-variable) and/or framework (FR) (variable) domain/position, vis-à-vis a peptide sequence disclosed herein. To better illustrate this concept, a brief description of antibody structure follows.

An antibody is composed of two peptide chains, each containing one (light chain) or three (heavy chain) constant domains and a variable region (VL, VH), the latter of which is in each case made up of four FR regions and three interspaced CDRs. The antigen-binding site is formed by one or more CDRs, yet the FR regions provide the structural framework for the CDRs and, hence, play an important role in antigen binding. By altering one or more amino acid residues in a CDR or FR region, the skilled worker routinely can generate mutated or diversified antibody sequences, which can be screened against the antigen, for new or improved properties, for example.

Tables 3 (VL) and 4 (VH) delineate the CDR and FR regions for certain antibodies of the disclosure and compare amino acids at a given position to each other and to corresponding consensus sequences.

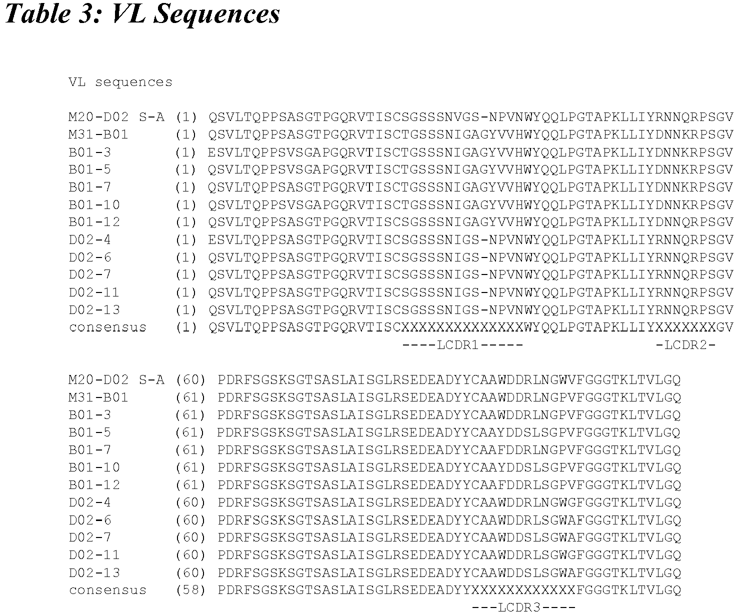

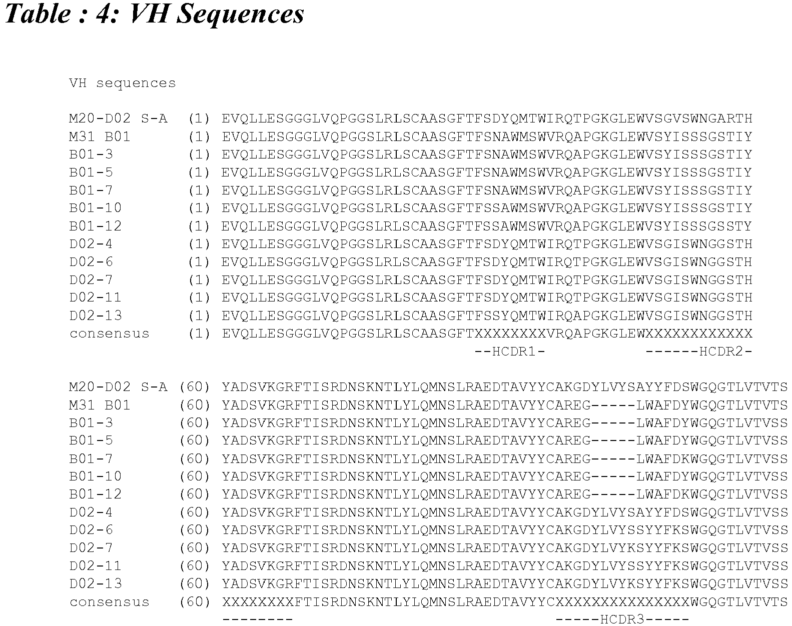

A further preferred embodiment of the disclosure is an antibody or antigen binding fragment thereof in which the CDR sequences are selected as shown in table 7.

A further preferred embodiment of the disclosure is an antibody or antigen-binding fragment in which the VH and VL sequences are selected as shown in table 7. The skilled worker can use the data in Tables 3, 4 and 7 to design peptide variants. It is preferred that variants are constructed by changing amino acids within one or more CDR regions; a variant might also have one or more altered framework regions. Alterations also may be made in the framework regions. For example, a peptide FR domain might be altered where there is a deviation in a residue compared to a germline sequence.

With reference to a comparison of the novel antibodies to the corresponding consensus sequences, which are listed in Tables 3 and 4 candidate residues that can be changed include e.g. residue 42 of the variable heavy chain of M20-D02 S-A compared to VHIII of Gene DP47. Alternatively, the skilled worker could make the same analysis by comparing the amino acid sequences disclosed herein to known sequences of the same class of such antibodies, using, for example, the procedure described by Knappik A., et al., JMB 2000, 296:57-86.

Furthermore, variants may be obtained by using one antibody as starting point for optimization by diversifying one or more amino acid residues in the antibody, preferably amino acid residues in one or more CDRs, and by screening the resulting collection of antibody variants for variants with improved properties. Particularly preferred is diversification of one or more amino acid residues in CDR3 of VL and/or VH.. Diversification can be done by synthesizing a collection of DNA molecules using trinucleotide mutagenesis (TRIM) technology (Virnekäs B. et al., Nucl. Acids Res. 1994, 22: 5600.). Antibodies or antigen-binding fragments thereof include molecules with modifications/variations including but not limited to e.g. modifications leading to altered half-life (e.g. modification of the Fc part or attachment of further molecules such as PEG) or altered ADCC or CDC activity.

### Conservative Amino Acid Variants

Polypeptide variants may be made that conserve the overall molecular structure of an antibody peptide sequence described herein. Given the properties of the individual amino acids, some rational substitutions will be recognized by the skilled worker. Amino acid substitutions, *i.e.,* "conservative substitutions," may be made, for instance, on the basis of similarity in polarity, charge, solubility, hydrophobicity, hydrophilicity, and/or the amphipathic nature of the residues involved.

For example, (a) nonpolar (hydrophobic) amino acids include alanine, leucine, isoleucine, valine, proline, phenylalanine, tryptophane, and methionine; (b) polar neutral amino acids include glycine, serine, threonine, cysteine, tyrosine, asparagine, and glutamine; (c) positively charged (basic) amino acids include arginine, lysine, and histidine; and (d) negatively charged (acidic) amino acids include aspartic acid and glutamic acid. Substitutions typically may be made within groups (a)-(d). In addition, glycine and proline may be substituted for one another based on their ability to disrupt α-helices. Similarly, certain amino acids, such as alanine, cysteine, leucine, methionine, glutamic acid, glutamine, histidine and lysine are more commonly found in α-helices, while valine, isoleucine, phenylalanine, tyrosine, tryptophan and threonine are more commonly found in β-pleated sheets. Glycine, serine, aspartic acid, asparagine, and proline are commonly found in turns. Some preferred substitutions may be made among the following groups: (i) S and T; (ii) P and G; and (iii) A, V, L and I. Given the known genetic code, and recombinant and synthetic DNA techniques, the skilled scientist readily can construct DNAs encoding the conservative amino acid variants. In one particular example, amino acid position 3 in SEQ ID NOS: 33 -36 can be changed from a Q to an E.

As used herein, "sequence identity" between two polypeptide sequences, indicates the percentage of amino acids that are identical between the sequences. "Sequence homology" indicates the percentage of amino acids that either is identical or that represent conservative amino acid substitutions.

### DNA molecules of the invention

The present invention also relates to the DNA molecules that encode an antibody of the invention or antigen-binding fragment thereof.

DNA molecules of the invention are not limited to the sequences disclosed herein, but also include variants thereof. DNA variants within the invention may be described by reference to their physical properties in hybridization. The skilled worker will recognize that DNA can be used to identify its complement and, since DNA is double stranded, its equivalent or homolog, using nucleic acid hybridization techniques. It also will be recognized that hybridization can occur with less than 100% complementarity. However, given appropriate choice of conditions, hybridization techniques can be used to differentiate among DNA sequences based on their structural relatedness to a particular probe. For guidance regarding such conditions see, Sambrook et al., 1989 *supra* and Ausubel et al., 1995 (Ausubel, F. M., Brent, R., Kingston, R. E., Moore, D. D., Sedman, J. G., Smith, J. A., & Struhl, K. eds. (1995). Current Protocols in Molecular Biology. New York: John Wiley and Sons).

Structural similarity between two polynucleotide sequences can be expressed as a function of "stringency" of the conditions under which the two sequences will hybridize with one another. As used herein, the term "stringency" refers to the extent that the conditions disfavor hybridization. Stringent conditions strongly disfavor hybridization, and only the most structurally related molecules will hybridize to one another under such conditions. Conversely, non-stringent conditions favor hybridization of molecules displaying a lesser degree of structural relatedness. Hybridization stringency, therefore, directly correlates with the structural relationships of two nucleic acid sequences. The following relationships are useful in correlating hybridization and relatedness (where Tₘ is the melting temperature of a nucleic acid duplex):
a. Tₘ = 69.3 + 0.41(G+C)%
b. The Tₘ of a duplex DNA decreases by 1°C with every increase of 1% in the number of mismatched base pairs.
c. (Tₘ)_{µ2} - (Tₘ) _{µ1} = 18.5 log₁₀µ2/µ1
   where µ1 and µ2 are the ionic strengths of two solutions.

Hybridization stringency is a function of many factors, including overall DNA concentration, ionic strength, temperature, probe size and the presence of agents which disrupt hydrogen bonding. Factors promoting hybridization include high DNA concentrations, high ionic strengths, low temperatures, longer probe size and the absence of agents that disrupt hydrogen bonding. Hybridization typically is performed in two phases: the "binding" phase and the "washing" phase.

First, in the binding phase, the probe is bound to the target under conditions favoring hybridization. Stringency is usually controlled at this stage by altering the temperature. For high stringency, the temperature is usually between 65°C and 70°C, unless short (< 20 nt) oligonucleotide probes are used. A representative hybridization solution comprises 6X SSC, 0.5% SDS, 5X Denhardt's solution and 100 µg of nonspecific carrier DNA. See Ausubel *et al.,* section 2.9, supplement 27 (1994). Of course, many different, yet functionally equivalent, buffer conditions are known. Where the degree of relatedness is lower, a lower temperature may be chosen. Low stringency binding temperatures are between about 25°C and 40°C. Medium stringency is between at least about 40°C to less than about 65°C. High stringency is at least about 65°C.

Second, the excess probe is removed by washing. It is at this phase that more stringent conditions usually are applied. Hence, it is this "washing" stage that is most important in determining relatedness via hybridization. Washing solutions typically contain lower salt concentrations. One exemplary medium stringency solution contains 2X SSC and 0.1% SDS. A high stringency wash solution contains the equivalent (in ionic strength) of less than about 0.2X SSC, with a preferred stringent solution containing about O.1X SSC. The temperatures associated with various stringencies are the same as discussed above for "binding." The washing solution also typically is replaced a number of times during washing. For example, typical high stringency washing conditions comprise washing twice for 30 minutes at 55° C. and three times for 15 minutes at 60° C.

An embodiment of the disclosure is an isolated nucleic acid sequence that encodes (i) the antibody or antigen-binding fragment of the disclosure, the CDR sequences as depicted in table 7, or (ii) the variable light and heavy chain sequences as depicted in table 7, or (iii) which comprises a nucleic acid sequence that encodes an antibody or antigen-binding fragment of the disclosure, the CDR sequences as depicted in table 7, or the variable light and heavy chain sequences as depicted in table 7.

### Functionally Equivalent Variants

Yet another class of DNA variants within the scope of the disclosure may be described with reference to the product they encode. These functionally equivalent polynucleotides are characterized by the fact that they encode the same peptide sequences found in SEQ ID NOS: 5-36, 45-50, 55-60, 65-70, 75-80, 85-90, 95-100, 105-110, 115-120, 125-130, 135-140, due to the degeneracy of the genetic code.

It is recognized that variants of DNA molecules provided herein can be constructed in several different ways. For example, they may be constructed as completely synthetic DNAs. Methods of efficiently synthesizing oligonucleotides in the range of 20 to about 150 nucleotides are widely available. *See* Ausubel *et al.,* section 2.11, Supplement 21 (1993). Overlapping oligonucleotides may be synthesized and assembled in a fashion first reported by Khorana et al., J. Mol. Biol. 72:209-217 (1971); *see also* Ausubel *et al., supra,* Section 8.2. Synthetic DNAs preferably are designed with convenient restriction sites engineered at the 5' and 3' ends of the gene to facilitate cloning into an appropriate vector.

As indicated, a method of generating variants is to start with one of the DNAs disclosed herein and then to conduct site-directed mutagenesis. *See* Ausubel et al., *supra,* chapter 8, Supplement 37 (1997). In a typical method, a target DNA is cloned into a single-stranded DNA bacteriophage vehicle. Single-stranded DNA is isolated and hybridized with an oligonucleotide containing the desired nucleotide alteration(s). The complementary strand is synthesized and the double stranded phage is introduced into a host. Some of the resulting progeny will contain the desired mutant, which can be confirmed using DNA sequencing. In addition, various methods are available that increase the probability that the progeny phage will be the desired mutant. These methods are well known to those in the field and kits are commercially available for generating such mutants.

### Recombinant DNA constructs and expression

The disclosure provides recombinant DNA constructs comprising one or more of the nucleotide sequences of the present invention. The recombinant constructs of the disclosire are used in connection with a vector, such as a plasmid, phagemid, phage or viral vector, into which a DNA molecule encoding an antibody of the invention or antigen-binding fragment thereof is inserted.

An antibody, antigen binding portion, or derivative thereof provided herein can be prepared by recombinant expression of nucleic acid sequences encoding light and heavy chains or portions thereof in a host cell. To express an antibody, antigen binding portion, or derivative thereof recombinantly, a host cell can be transfected with one or more recombinant expression vectors carrying DNA fragments encoding the light and/or heavy chains or portions thereof such that the light and heavy chains are expressed in the host cell. Standard recombinant DNA methodologies are used prepare and/or obtain nucleic acids encoding the heavy and light chains, incorporate these nucleic acids into recombinant expression vectors and introduce the vectors into host cells, such as those described in Sambrook, Fritsch and Maniatis (eds.), Molecular Cloning; A Laboratory Manual, Second Edition, Cold Spring Harbor, N.Y., (1989), Ausubel, F. M. et al. (eds.) Current Protocols in Molecular Biology, Greene Publishing Associates, (1989) and in U.S. Pat. No. 4,816,397 by Boss et al.

In addition, the nucleic acid sequences encoding variable regions of the heavy and/or light chains can be converted, for example, to nucleic acid sequences encoding full-length antibody chains, Fab fragments, or to scFv. The VL- or VH-encoding DNA fragment can be operatively linked, (such that the amino acid sequences encoded by the two DNA fragments are in-frame) to another DNA fragment encoding, for example, an antibody constant region or a flexible linker. The sequences of human heavy chain and light chain constant regions are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification.

To create a polynucleotide sequence that encodes a scFv, the VH- and VL-encoding nucleic acids can be operatively linked to another fragment encoding a flexible linker such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, antigen binding portions or derivatives thereof standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g. bacteria, examples for eukaryotic host cells are yeast, insect or mammalian cells. In some embodiments, the DNAs encoding the heavy and light chains are inserted into separate vectors. In other embodiments, the DNA encoding the heavy and light chains are inserted into the same vector. It is understood that the design of the expression vector, including the selection of regulatory sequences is affected by factors such as the choice of the host cell, the level of expression of protein desired and whether expression is constitutive or inducible.

### Bacterial Expression

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include *E. coli, Bacillus subtilis, Salmonella typhimurium* and various species within the genera Pseudomonas, Streptomyces, and Staphylococcus.

Bacterial vectors may be, for example, bacteriophage-, plasmid- or phagemid-based. These vectors can contain a selectable marker and bacterial origin of replication derived from commercially available plasmids typically containing elements of the well known cloning vector pBR322 (ATCC 37017). Following transformation of a suitable host strain and growth of the host strain to an appropriate cell density, the selected promoter is de-repressed/induced by appropriate means (*e.g*., temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced, for the generation of antibodies or to screen peptide libraries, for example, vectors which direct the expression of high levels of fusion protein products that are readily purified may be desirable. Antibodies of the present invention or antigen-binding fragment thereof include naturally purified products, products of chemical synthetic procedures, and products produced by recombinant techniques from a prokaryotic host, including, for example, *E. coli, Bacillus subtilis, Salmonella typhimurium* and various species within the genera Pseudomonas, Streptomyces, and Staphylococcus, preferably, from *E. coli* cells.

### Mammalian Expression & Purification

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. For further description of viral regulatory elements, and sequences thereof, see e.g., U.S. 5,168,062 by Stinski, U.S. 4,510,245 by Bell et al. and U.S. 4,968,615 by Schaffner et al. The recombinant expression vectors can also include origins of replication and selectable markers (see e.g., U.S. 4,399,216, 4,634,665 and U.S. 5,179,017, by Axel et al.). Suitable selectable markers include genes that confer resistance to drugs such as G418, hygromycin or methotrexate, on a host cell into which the vector has been introduced. For example, the dihydrofolate reductase (DHFR) gene confers resistance to methotrexate and the neo gene confers resistance to G418.

Transfection of the expression vector into a host cell can be carried out using standard techniques such as electroporation, calcium-phosphate precipitation, and DEAE-dextran, lipofection or polycation-mediated transfection.

Suitable mammalian host cells for expressing the antibodies, antigen binding portions, or derivatives thereof provided herein include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) Mol. Biol. 159:601-621, NSO myeloma cells, COS cells and SP2 cells. In some embodiments, the expression vector is designed such that the expressed protein is secreted into the culture medium in which the host cells are grown. Transient transfection/epression of antibodies can for example be achieved following the protocols by Durocher et al (2002) Nucl.Acids Res. Vol 30 e9. Stable transfection/expression of antibodies can for example be achieved following the protocols of the UCOE system (T. Benton et al. (2002) Cytotechnology 38: 43-46).

The antibodies, antigen binding portions, or derivatives thereof can be recovered from the culture medium using standard protein purification methods.

Antibodies of the invention or an antigen-binding fragment thereof can be recovered and purified from recombinant cell cultures by well-known methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference.

Antibodies of the disclosure or antigen-binding fragment thereof include naturally purified products, products of chemical synthetic procedures, and products produced by recombinant techniques from a eukaryotic host, including, for example, yeast, higher plant, insect and mammalian cells, preferably from mammalian cells. Depending upon the host employed in a recombinant production procedure, the antibody of the present invention can be glycosylated or can be non-glycosylated, with glycosylated preferred. Such methods are described in many standard laboratory manuals, such as Sambrook, supra, Sections 17.37-17.42; Ausubel, supra, Chapters 10, 12, 13, 16, 18 and 20.

### Therapeutic Methods

Therapeutic methods involve administering to a subject in need of treatment a therapeutically effective amount of an antibody or antigen-binding fragment thereof contemplated by the invention. A "therapeutically effective" amount hereby is defined as the amount of an antibody or antigen-binding fragment that is of sufficient quantity to deplete C4.4a-positive cells in a treated area of a subject - either as a single dose or according to a multiple dose regimen, alone or in combination with other agents, which leads to the alleviation of an adverse condition, yet which amount is toxicologically tolerable. The subject may be a human or non-human animal (*e.g*., rabbit, rat, mouse, dog, monkey or other lower-order primate).

An antibody of the invention or antigen-binding fragment thereof might be co-administered with known medicaments, and in some instances the antibody might itself be modified. For example, an antibody could be conjugated to a cytotoxic agent or radioisotope to potentially further increase efficacy.

Antibodies of the present invention may be administered as the sole pharmaceutical agent or in combination with one or more additional therapeutic agents where the combination causes no unacceptable adverse effects. This combination therapy includes administration of a single pharmaceutical dosage formulation which contains an antibody of the invention and one or more additional therapeutic agents, as well as administration of an antibody of the invention and each additional therapeutic agent in its own separate pharmaceutical dosage formulation. For example, an antibody of the invention and a therapeutic agent may be administered to the patient together in a single oral dosage composition such as a tablet or capsule, or each agent may be administered in separate dosage formulations.

Where separate dosage formulations are used, an antibody of the invention and one or more additional therapeutic agents may be administered at essentially the same time (*e.g.,* concurrently) or at separately staggered times (*e.g.,* sequentially).

In particular, an antibodies of the present invention may be used in fixed or separate combination with other anti-tumor agents such as alkylating agents, anti-metabolites, plant-derived anti-tumor agents, hormonal therapy agents, topoisomerase inhibitors, camptothecin derivatives, kinase inhibitors, targeted drugs, antibodies, interferons and/or biological response modifiers, anti-angiogenic compounds, and other anti-tumor drugs. In this regard, the following is a nonlimiting list of examples of secondary agents that may be used in combination with the antibodies of the present invention:
Alkylating agents include, but are not limited to, nitrogen mustard N-oxide, cyclophosphamide, ifosfamide, thiotepa, ranimustine, nimustine, temozolomide, altretamine, apaziquone, brostallicin, bendamustine, carmustine, estramustine, fotemustine, glufosfamide, mafosfamide, bendamustin, and mitolactol; platinum-coordinated alkylating compounds include, but are not limited to, cisplatin, carboplatin, eptaplatin, lobaplatin, nedaplatin, oxaliplatin, and satraplatin;
Anti-metabolites include, but are not limited to, methotrexate, 6-mercaptopurine riboside, mercaptopurine, 5-fluorouracil alone or in combination with leucovorin, tegafur, doxifluridine, carmofur, cytarabine, cytarabine ocfosfate, enocitabine, gemcitabine, fludarabin, 5-azacitidine, capecitabine, cladribine, clofarabine, decitabine, eflornithine, ethynylcytidine, cytosine arabinoside, hydroxyurea, melphalan, nelarabine, nolatrexed, ocfosfite, disodium premetrexed, pentostatin, pelitrexol, raltitrexed, triapine, trimetrexate, vidarabine, vincristine, and vinorelbine;
Hormonal therapy agents include, but are not limited to, exemestane, Lupron, anastrozole, doxercalciferol, fadrozole, formestane, 11-beta hydroxysteroid dehydrogenase 1 inhibitors, 17-alpha hydroxylase/17,20 lyase inhibitors such as abiraterone acetate, 5-alpha reductase inhibitors such as finasteride and epristeride, anti-estrogens such as tamoxifen citrate and fulvestrant, Trelstar, toremifene, raloxifene, lasofoxifene, letrozole, anti-androgens such as bicalutamide, flutamide, mifepristone, nilutamide, Casodex, and anti-progesterones and combinations thereof;
Plant-derived anti-tumor substances include, e.g., those selected from mitotic inhibitors, for example epothilones such as sagopilone, ixabepilone and epothilone B, vinblastine, vinflunine, docetaxel, and paclitaxel;
Cytotoxic topoisomerase inhibiting agents include, but are not limited to, aclarubicin, doxorubicin, amonafide, belotecan, camptothecin, 10-hydroxycamptothecin, 9-aminocamptothecin, diflomotecan, irinotecan, topotecan, edotecarin, epimbicin, etoposide, exatecan, gimatecan, lurtotecan, mitoxantrone, pirambicin, pixantrone, rubitecan, sobuzoxane, tafluposide, and combinations thereof;
Immunologicals include interferons such as interferon alpha, interferon alpha-2a, interferon alpha-2b, interferon beta, interferon gamma-1a and interferon gamma-n1, and other immune enhancing agents such as L19-IL2 and other IL2 derivatives, filgrastim, lentinan, sizofilan, TheraCys, ubenimex, aldesleukin, alemtuzumab, BAM-002, dacarbazine, daclizumab, denileukin, gemtuzumab, ozogamicin, ibritumomab, imiquimod, lenograstim, lentinan, melanoma vaccine (Corixa), molgramostim, sargramostim, tasonermin, tecleukin, thymalasin, tositumomab, Vimlizin, epratuzumab, mitumomab, oregovomab, pemtumomab, and Provenge;
Biological response modifiers are agents that modify defense mechanisms of living organisms or biological responses such as survival, growth or differentiation of tissue cells to direct them to have anti-tumor activity; such agents include, e.g., krestin, lentinan, sizofiran, picibanil, ProMune, and ubenimex;
Anti-angiogenic compounds include, but are not limited to, acitretin, aflibercept, angiostatin, aplidine, asentar, axitinib, bevacizumab, brivanib alaninat, cilengtide, combretastatin, endostatin, fenretinide, halofuginone, pazopanib, ranibizumab, rebimastat, recentin, regorafenib, removab, revlimid, sorafenib, squalamine, sunitinib, telatinib, thalidomide, ukrain, vatalanib, and vitaxin;
Antibodies include, but are not limited to, trastuzumab, cetuximab, bevacizumab, rituximab, ticilimumab, ipilimumab, lumiliximab, catumaxomab, atacicept, oregovomab, and alemtuzumab;
VEGF inhibitors such as, e.g., sorafenib, regorafenib, bevacizumab, sunitinib, recentin, axitinib, aflibercept, telatinib, brivanib alaninate, vatalanib, pazopanib, and ranibizumab;
EGFR (HER1) inhibitors such as, e.g., cetuximab, panitumumab, vectibix, gefitinib, erlotinib, and Zactima;
HER2 inhibitors such as, e.g., lapatinib, tratuzumab, and pertuzumab;
mTOR inhibitors such as, e.g., temsirolimus, sirolimus/Rapamycin, and everolimus;
c-Met inhibitors;
PI3K and AKT inhibitors;
CDK inhibitors such as roscovitine and flavopiridol;
Spindle assembly checkpoints inhibitors and targeted anti-mitotic agents such as PLK inhibitors, Aurora inhibitors (e.g. Hesperadin), checkpoint kinase inhibitors, and KSP inhibitors;
HDAC inhibitors such as, e.g., panobinostat, vorinostat, MS275, belinostat, and LBH589;
HSP90 and HSP70 inhibitors;
Proteasome inhibitors such as bortezomib and carfilzomib;
Serine/threonine kinase inhibitors including MEK inhibitors and Raf inhibitors such as sorafenib;
Farnesyl transferase inhibitors such as, e.g., tipifarnib;
Tyrosine kinase inhibitors including, e.g., dasatinib, nilotibib, regorafenib, bosutinib, sorafenib, bevacizumab, sunitinib, cediranib, axitinib, aflibercept, telatinib, imatinib mesylate, brivanib alaninate, pazopanib, ranibizumab, vatalanib, cetuximab, panitumumab, vectibix, gefitinib, erlotinib, lapatinib, tratuzumab, pertuzumab, and c-Kit inhibitors;
Vitamin D receptor agonists;
Bcl-2 protein inhibitors such as obatoclax, oblimersen sodium, and gossypol;
Cluster of differentiation 20 receptor antagonists such as, e.g., rituximab;
Ribonucleotide reductase inhibitors such as, e.g., gemcitabine;
Tumor necrosis apoptosis inducing ligand receptor 1 agonists such as, e.g., mapatumumab;
5-Hydroxytryptamine receptor antagonists such as, e.g., rEV598, xaliprode, palonosetron hydrochloride, granisetron, Zindol, and AB-1001;
Integrin inhibitors including alpha5-betal integrin inhibitors such as, e.g., E7820, JSM 6425, volociximab, and endostatin;
Androgen receptor antagonists including, e.g., nandrolone decanoate, fluoxymesterone, Android, Prost-aid, andromustine, bicalutamide, flutamide, apocyproterone, apo-flutamide, chlormadinone acetate, Androcur, Tabi, cyproterone acetate, and nilutamide;
Aromatase inhibitors such as, e.g., anastrozole, letrozole, testolactone, exemestane, amino glutethimide, and formestane;
Matrix metalloproteinase inhibitors;
Other anti-cancer agents including, e.g., alitretinoin, ampligen, atrasentan bexarotene, bortezomib, bosentan, calcitriol, exisulind, fotemustine, ibandronic acid, miltefosine, mitoxantrone, I-asparaginase, procarbazine, dacarbazine, hydroxycarbamide, pegaspargase, pentostatin, tazaroten, velcade, gallium nitrate, canfosfamide, darinaparsin, and tretinoin.

In a preferred embodiment, the antibodies of the present invention may be used in combination with chemotherapy (i.e. cytotoxic agents), anti-hormones and/or targeted therapies such as other kinase inhibitors (for example, EGFR inhibitors), mTOR inhibitors and angiogenesis inhibitors.

The compounds of the present invention may also be employed in cancer treatment in conjunction with radiation therapy and/or surgical intervention.

An antibody of the invention or antigen-binding fragment thereof might in some instances itself be modified. For example, an antibody could be conjugated to any of but not limited to the compounds mentioned above or any radioisotope to potentially further increase efficacy. Furthermore, the antibodies of the invention may be utilized, as such or in compositions, in research and diagnostics, or as analytical reference standards, and the like, which are well known in the art.

The inventive antibodies or antigen-binding fragments thereof can be used as a therapeutic or a diagnostic tool in a variety of situations where C4.4a is undesirably expressed or found, e.g. cell proliferative disorders such as cancer. Disorders and conditions particularly suitable for treatment with an antibody of the inventions are solid tumors, such as cancers of the breast, respiratory tract, brain, reproductive organs, digestive tract, urinary tract, eye, liver, skin, head and neck, thyroid, parathyroid, and their distant metastases. Those disorders also include lymphomas, sarcomas and leukemias.

Examples of breast cancer include, but are not limited to invasive ductal carcinoma, invasive lobular carcinoma, ductal carcinoma *in situ,* and lobular carcinoma *in situ.*

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, glioblastoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal and vulvar cancer, as well as sarcoma of the uterus.

Tumors of the digestive tract include, but are not limited to anal, colon, colorectal, esophageal, gallbladder, gastric, pancreatic, rectal, small-intestine, and salivary gland cancers.

Tumors of the urinary tract include, but are not limited to bladder, penile, kidney, renal pelvis, ureter, urethral, and hereditary and sporadic papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cho langiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer, and squamous cell cancer.

Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

The disorders mentioned above have been well characterized in humans, but also exist with a similar etiology in other animals, including mammals, and can be treated by administering pharmaceutical compositions of the present invention.

To treat any of the foregoing disorders, pharmaceutical compositions for use in accordance with the present invention may be formulated in a conventional manner using one or more physiologically acceptable carriers or excipients. An antibody of the invention or antigen-binding fragment thereof can be administered by any suitable means, which can vary, depending on the type of disorder being treated. Possible administration routes include parenteral (e.g., intramuscular, intravenous, intra-arterial, intraperitoneal, or subcutaneous), intrapulmonary and intranasal, and, if desired for local immunosuppressive treatment, intralesional administration. In addition, an antibody of the invention might be administered by pulse infusion, with, *e.g.,* declining doses of the antibody. Preferably, the dosing is given by injections, most preferably intravenous or subcutaneous injections, depending in part on whether the administration is brief or chronic. The amount to be administered will depend on a variety of factors such as the clinical symptoms, weight of the individual, whether other drugs are administered. The skilled artisan will recognize that the route of administration will vary depending on the disorder or condition to be treated.

Determining a therapeutically effective amount of the novel polypeptide, according to this invention, largely will depend on particular patient characteristics, route of administration, and the nature of the disorder being treated. General guidance can be found, for example, in the publications of the International Conference on Harmonization and in REMINGTON'S PHARMACEUTICAL SCIENCES, chapters 27 and 28, pp. 484-528 (18th ed., Alfonso R. Gennaro, Ed., Easton, Pa.: Mack Pub. Co., 1990). More specifically, determining a therapeutically effective amount will depend on such factors as toxicity and efficacy of the medicament. Toxicity may be determined using methods well known in the art and found in the foregoing references. Efficacy may be determined utilizing the same guidance in conjunction with the methods described below in the Examples.

### Diagnostic Methods

C4.4a antibodies or antigen-binding fragments thereof can be used for detecting the presence of C4.4a-expressing tumors. The presence of C4.4a-containing cells or shed C4.4a within various biological samples, including serum, and tissue biopsy specimens, may be detected with C4.4a antibodies. In addition, C4.4a antibodies may be used in various imaging methodologies such as immunoscintigraphy with a ⁹⁹Tc (or other isotope) conjugated antibody. For example, an imaging protocol similar to the one recently described using a ¹¹¹In conjugated anti-PSMA antibody may be used to detect pancreatic or ovarian carcinomas (Sodee et al., Clin. Nuc. Med. 21: 759-766, 1997). Another method of detection that can be used is positron emitting tomography by conjugating the antibodies of the invention with a suitable isotope (see Herzog et al., J. Nucl. Med. 34:2222-2226, 1993).

### Pharmaceutical Compositions and Administration

An embodiment of the present invention are pharmaceutical compositions which comprise C4.4a antibodies or antigen-binding fragment thereof, alone or in combination with at least one other agent, such as stabilizing compound, which may be administered in any sterile, biocompatible pharmaceutical carrier, including, but not limited to, saline, buffered saline, dextrose, and water. A further embodiment are pharmaceutical compositions comprising a C4.4a binding antibody or antigen-binding fragment therof and a further pharmaceutically active compound that is suitable to treat C4.4a related diseases such as cancer. Any of these molecules can be administered to a patient alone, or in combination with other agents, drugs or hormones, in pharmaceutical compositions where it is mixed with excipient(s) or pharmaceutically acceptable carriers. In one embodiment of the present invention, the pharmaceutically acceptable carrier is pharmaceutically inert.

The present disclosure also relates to the administration of pharmaceutical compositions. Such administration is accomplished orally or parenterally. Methods of parenteral delivery include topical, intra-arterial (directly to the tumor), intramuscular, subcutaneous, intramedullary, intrathecal, intraventricular, intravenous, intraperitoneal, or intranasal administration. In addition to the active ingredients, these pharmaceutical compositions may contain suitable pharmaceutically acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Ed. Maack Publishing Co, Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers such as sugars, including lactose, sucrose, mannitol, or sorbitol; starch from corn, wheat, rice, potato, or other plants; cellulose such as methyl, cellulose, hydroxypropylmethylcellulose, or sodium carboxymethyl cellulose; and gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores are provided with suitable coatings such as concentrated sugar solutions, which may also contain gum arabic, talc, polyvinyl pyrrolidone, carbopol gel, polyethylene glycol and/or titanium dioxide, lacquer solutions, and suitable organic solvents or solvent mixtures. Dyestuffs or pigments may be added to the tablets or dragee coatings for product identification or to characterize the quantity of active compound, i.e. dosage.

Pharmaceutical preparations that can be used orally include push-fit capsules made of gelatin, as well as soft, sealed capsules made of gelatin and a coating such as glycerol or sorbitol. Push-fit capsules can contain active ingredients mixed with a filler or binders such as lactose or starches, lubricants such as talc or magnesium stearate, and optionally, stabilizers. In soft capsules, the active compounds may be dissolved or suspended in suitable liquids, such as fatty oils, liquid paraffin, or liquid polyethylene glycol with or without stabilizers.

Pharmaceutical formulations for parenteral administration include aqueous solutions of active compounds. For injection, the pharmaceutical compositions of the invention may be formulated in aqueous solutions, preferably in physiologically compatible buffers such as Hank's solution, Ringer's solution, or physiologically buffered saline. Aqueous injection suspensions may contain substances that increase viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

### Kits

The invention further relates to pharmaceutical packs and kits comprising one or more containers filled with one or more of the ingredients of the aforementioned compositions of the invention. Associated with such container(s) can be a notice in the form prescribed by a governmental agency regulating the manufacture, use or sale of pharmaceuticals or biological products, reflecting approval by the agency of the manufacture, use or sale of the product for human administration.

In another embodiment, the kits may contain DNA sequences encoding the antibodies of the disclosure. Preferably the DNA sequences encoding these antibodies are provided in a plasmid suitable for transfection into and expression by a host cell. The plasmid may contain a promoter (often an inducible promoter) to regulate expression of the DNA in the host cell. The plasmid may also contain appropriate restriction sites to facilitate the insertion of other DNA sequences into the plasmid to produce various antibodies. The plasmids may also contain numerous other elements to facilitate cloning and expression of the encoded proteins. Such elements are well known to those of skill in the art and include, for example, selectable markers, initiation codons, termination codons, and the like.

### Manufacture and Storage.

The pharmaceutical compositions of the present invention may be manufactured in a manner that is known in the art, e.g., by means of conventional mixing, dissolving, granulating, dragee-making, levigating, emulsifying, encapsulating, entrapping or lyophilizing processes.

The pharmaceutical composition may be provided as a salt and can be formed with acids, including by not limited to hydrochloric, sulfuric, acetic, lactic, tartaric, malic, succinic, etc. Salts tend to be more soluble in aqueous or other protonic solvents that are the corresponding free base forms. In other cases, the preferred preparation may be a lyophilized powder in 1 mM-50 mM histidine, 0.1%-2% sucrose, 2%-7% mannitol at a pH range of 4.5 to 5.5 that is combined with buffer prior to use.

After pharmaceutical compositions comprising a compound of the invention formulated in an acceptable carrier have been prepared, they can be placed in an appropriate container and labeled for treatment of an indicated condition. For administration of C4.4a antibodies or antigen-binding fragment thereof, such labeling would include amount, frequency and method of administration.

### Therapeutically Effective Dose.

Pharmaceutical compositions suitable for use in the present invention include compositions wherein the active ingredients are contained in an effective amount to achieve the intended purpose, i.e. treatment of a particular disease state characterized by C4.4a expression. The determination of an effective dose is well within the capability of those skilled in the art.

For any compound, the therapeutically effective dose can be estimated initially either in cell culture assays, e.g., neoplastic cells, or in animal models, usually mice, rabbits, dogs, pigs or monkeys. The animal model is also used to achieve a desirable concentration range and route of administration. Such information can then be used to determine useful doses and routes for administration in humans.

A therapeutically effective dose refers to that amount of antibody or antigen-binding fragment thereof, that ameliorate the symptoms or condition. Therapeutic efficacy and toxicity of such compounds can be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, ED₅₀/LD₅₀. Pharmaceutical compositions that exhibit large therapeutic indices are preferred. The data obtained from cell culture assays and animal studies are used in formulating a range of dosage for human use. The dosage of such compounds lies preferably within a range of circulating concentrations what include the ED₅₀ with little or no toxicity. The dosage varies within this range depending upon the dosage form employed, sensitivity of the patient, and the route of administration.

The exact dosage is chosen by the individual physician in view of the patient to be treated. Dosage and administration are adjusted to provide sufficient levels of the active moiety or to maintain the desired effect. Additional factors that may be taken into account include the severity of the disease state, e.g., tumor size and location; age, weight and gender of the patient; diet, time and frequency of administration, drug combination(s), reaction sensitivities, and tolerance/response to therapy. Long acting pharmaceutical compositions might be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Guidance as to particular dosages and methods of delivery is provided in the literature. See U.S. Pat. No. 4,657,760; 5,206,344; or 5,225,212. Those skilled in the art will employ different formulations for polynucleotides than for proteins or their inhibitors. Similarly, delivery of polynucleotides or polypeptides will be specific to particular cells, conditions, locations, etc. Preferred specific activities for a radiolabelled antibody may range from 0.1 to 10 mCi/mg of protein (Riva et al., Clin. Cancer Res. 5:3275-3280, 1999; Ulaner et al., 2008 Radiology 246(3):895-902)

The present invention is further described by the following examples. The examples are provided solely to illustrate the invention by reference to specific embodiments. These exemplifications, while illustrating certain specific aspects of the invention, do not portray the limitations or circumscribe the scope of the disclosed invention.

All examples were carried out using standard techniques, which are well known and routine to those of skill in the art, except where otherwise described in detail. Routine molecular biology techniques of the following examples can be carried out as described in standard laboratory manuals, such as Sambrook et al., Molecular Cloning: A Laboratory Manual, 2nd Ed.; Cold Spring Harbor Laboratory Press, Cold Spring Harbor, N.Y., 1989.

### EXAMPLES

### EXAMPLE 1: Antibody Generation from n-CoDeR Libraries

The isolation of human antibodies against C4.4a was performed by phage display technology employing the naive antibody library n-CoDeR of BioInvent International AB (Lund, Sweden; described in Söderling et al., Nature Biotech. 2000, 18:853-856) in a cell selection approach. n-CoDeR is a Fab library in which all six CDRs are diversified. CDR sequences were initially obtained from healthy human donors.

Briefly, an aliquot of the Fab antibody library was depleted for unwanted surface binders by pre-incubation with 10⁷ non-C4.4a expressing parental CHO-S cells in PBS / 3% FCS / 0.01 % NaN3 (buffer A) at 4°C by end-over-end rotation for 15 min. After that cells were removed by centrifugation, the supernatant was used for 2 additional rounds of pre-incubation on CHO-S cells. Sub sequential panning on target cells (10⁷ CHO-S:hC4.4a cells) was done by incubation with the phage preparation for 45 min at 4°C in buffer A followed by 10 times washing with buffer A (4°C). Bound phages were eluted by treating the cells during 5 min end-over-end rotation with 76 mM citric acid (4°C). The preparation containing eluted phages was neutralized by addition of 1M Tris/HCl, pH 7.5 and 2 additional rounds of cell panning were performed. In each round eluted phages were propagated and phage titers determined as previously described (Cicortas Gunnarsson et al., PEDS 2004, 17(3) 213-221). Briefly, aliquots of the eluate solution were saved for titration experiments while the rest was used to transform exponentially growing E. coli HB101' for preparation of new phage stocks. For each selection round, both input and output phages were titrated on exponentially growing E. coli HB101' and clones were picked from round 2 and 3 for analysis in Phage ELISA.

### Enzyme-linked immunosorbent assay (ELISA):

### Phage ELISA:

Selected phages from different selection rounds were analyzed for specificity using phage ELISA. Briefly, phage expression was performed by adding 10 µl of overnight culture (in LB-medium supplemented with 100 µg/ml ampicillin and 15 µg/ml tetracyclin) to 100 µl fresh medium (LB-medium supplemented with 100 µg/ml ampicillin, 15 µg/ml tetracyclin and 0,1% glucose) and shaking at 250 rpm and 37°C in 96-well MTP until an OD600 of 0.5 was reached. Subsequently helper phage M13KO7 (Invitrogen) was added and samples were incubated for another 15 min at 37°C without shaking. After addition of IPTG (f.c. of 0.25 mM) cells were incubated for 16 h at 30°C while shaking at 200 rpm.

96-well MTP (Nunc-maxisorb) were coated for 16 h at 4°C with recombinant 50 µl C4.4a (5 µg/ml human or mouse protein). The next day plates were washed 3 times with PBS/0.05% Tween 20 (buffer B), treated with blocking reagent (3% milk powder in buffer B), and washed again 3 times with buffer B. After that 50 µl aliquots from phage expressions were transferred per well and incubated for 1 h at 20 °C. After washing 3 times with buffer B anti M13 antibody coupled to HRP (GE Healthcare, 27-9421-01; 1:2500 diluted in buffer B) was added and incubated for 1 h at 20 °C. Color reaction was developed by addition of 50 µl TMB (Invitrogen) and stopped after 5-15 min at 20°C by adding 50 µl stopping solution (Invitrogen). Colorimetric reaction was recorded at 450 nM in a Tecan reader.

### Screening of sFabs by ELISA:

For the generation of soluble Fabs (sFabs) Phagemid DNA from the selection rounds 2 and 3 was isolated and digested with restriction enzymes EagI and EcoRI according to the providers instructions in order to remove the gene III product. The resulting fragment was re-ligated and constructs were transformed into chemically competent E. coli Top10 using standard methods. A total of ∼1500 clones were picked, transferred to 96-well plates containing LB-media (100 µg/ml, 0.1% glucose) and shaken at 250 rpm and 37°C until an OD600 of 0.5 was reached. After that sFab production was induced by the addition of IPTG (final concentration 0.5 mM) and incubation was continued for 16 h at 30°C while shaking at 200 rpm. Next morning BEL-buffer was added to each well (24.7 g/l boric acid; 18.7 g/l NaCl; 1.49 g/l EDTA pH 8.0; 2.5 mg/ml lysozyme (Roche)) and 50 µl of the treated cultures were analyzed for sFab binding to the target in an ELISA essentially as described for phages, except that detection was performed with an anti-hIgG (Fab-specific) coupled to HRP (Sigma; Product No. A 0293)

### FACS:

Cell binding of sFabs to target cells was analyzed on a FACSarray from BD. Briefly 50 µl cell suspension (2x10⁶ cells/ml) were mixed with 50 µl E. coli supernatant for 1 h at 4°C and 300 rpm. Subsequently 100 µl buffer A was added, cells were centrifuged and washed another 2 times with buffer A. For the detection of bound Fabs R-Phycoerythrin conjugate AffiniPure F(ab')₂ fragment goat anti human F(ab')₂ fragment specific; Jackson Immuno Research; Product No. 109-116-097, diluted 1:100 in buffer A was added to the cells and incubated for 1 h at 4°C. After 3times washing with buffer A the final pellet was resuspended in 150 µl buffer A and 5000 FACS events were recorded per sample. Data analysis was performed using the BD FACSArray system software.

### EXAMPLE 2: Epitope Grouping

Epitope grouping experiments were performed using surface plasmon resonance analysis on a Biacore T100 instrument (GE Healthcare Biacore, Inc.) by monitoring simultaneous binding of pairs of anti-C4.4a antibodies to C4.4a. All following steps were performed at 20°C. Approximately 2000 RU (one RU (resonance unit) represents the binding of 1 pg of protein per square mm) of the first antibody was covalently immobilized onto a CM5 sensor chip through primary amine coupling. The chip was activated with an 1:1 ration of 0,4 M N-ethyl-N-(3-diethylaminopropyl) carbodiimide (EDC) and 0.1 M n-hydroxysuccinamide (NHC) at a flow rate of 10µl/min.for 5-15 min.. Unoccupied binding sites on the surface were then blocked with an excess of 1 M ethanolamine pH 8.5. Soluble C4.4a (concentration of 400 nM in HEPES-EP buffer (GE Healthcare Biacore, Inc.) at a flow rate of 10 µl/min for 3 minutes) was captured on the surface via the immobilized antibody,. Therefore, the epitope of the capture antibody is blocked for all bound C4.4a molecules. Subsequently, a second antibody (at concentration of 200 nM in HEPES-EP buffer at a flow rate of 10 µl/min for 3 minutes) was immediately passed over the surface to bind to the captured C4.4a. Two antibodies recognizing the same or overlapping epitopes cannot bind to the C4.4a, whereas antibodies with distinct epitopes are able to bind. The antibody surface was regenerated with 3M MgCl2 (at a flow rate of 10 µl/min for 30 seconds), to remove all bound proteins and then the process was repeated with other antibodies.

Antibodies M31-B01 and M20-D02 S-A in IgG1 format were tested. Both antibodies were unable to bind simultaneously to C4.4a and therefore compete in binding (see Fig 1).

### EXAMPLE 3: Cross-reactivity to murine C4.4a

Shown in Table 1 are results of Biacore and studies showing cross-reactivity and dissociation constants (K_{D}) of antibodies of the disclosure to murine C4.4a.

Binding affinities of anti C4.4 antibodies were determined by surface plasmon resonance analysis on a Biacore T100 instrument (GE Healthcare Biacore, Inc.). Antibodies were immobilized onto a CM5 sensor chip through an indirect capturing reagent, anti-human IgG Fc. Reagents from the "Human Antibody Capture Kit" (BR-1008-39, GE Healthcare Biacore, Inc.) were used as described by the manufacturer. Approximately 5000 RU monoclonal mouse anti-human IgG (Fc) antibody were immobilized per cell. Anti C4.4 antibodies were injected at a concentration of 5µg/ml at 10µl/min for 10 sec to reach a capturing level of approximately 200 to 600 RU. Various concentrations (400 nM, 200 nM, 100 nM, 50 nM, 25 nM, 12.5 nM, 6.25 nM, and 3.12 nM) in HEPES-EP buffer (GE Healthcare Biacore, Inc.) of human or murine C4.4a were injected over immobilized anti C4.4a antibodies at a flow rate of 60 µl/min for 3 minutes and the dissociation was allowed for 10 minutes. Sensograms were generated after in-line reference cell correction followed by buffer sample subtraction. The dissociation equilibrium constant (KD) was calculated based on the ratio of association (kₒₙ) and dissociation rated (k_{off}) constants, obtained by fitting sensograms with a first order 1:1 binding model using Biavaluation Software (version 4.0).

### EXAMPLE 4: Binding to recombinant human and murine C4.4a

Figure 2 depicts binding curves of anti-C4.4 antibody M31-B01 and M20 D02 S-A on recombinant human C4.4a (A) and murine C4.4a (B) respectively. Briefly, 96-well MTP were coated for 16 h at 4°C with recombinant human or mouse C4.4a (100 ng/well plates were washed 3 times with PBS/0.05% Tween 20 (=buffer B) treated with blocking reagent (PBS + 2 % BSA), and washed again 3 times with buffer B. After that anti C4.4a antibodies of the disclosure were added in concentrations of 0.39 ng to 400 ng /per well and incubated for one hour at 20°C. After washing 3 times with buffer B, 20 ng of Protein A were added in 100 µl buffer B to each well. Color reaction was developed by addition of 50 µl 3,3',5,5'-Tetramethylbenzidine (TMB) (Sigma). Data for EC₅₀ determination were recorded after 18 min by measuring extinction 360 nm a Tecan infinite plate reader. EC₅₀ values for M31-B01 were 0.24 and 0.29 nM on human and murine C4.4a, respectively. And for M20-D02 S-A EC₅₀ values were 0.3 and 0.38 nM on human and murine C4.4a, respectively. This indicates high affinity binding of anti C4.4a antibodies of the disclosure to both human and murine soluble recombinant C4.4a.

### EXAMPLE 5: Binding to different Tumor cells

Figure 3 depicts binding curves and EC₅₀ values of antibodies M31-B01 and M20-D02 S-A to different tumor cells. Analyses were done by FACS using the transfected cell line A549:hC4.4a and natively C4.4a expressing tumor cell lines NCI H292, NCI H332, H1975/BCRP and BxPC3. Figure 3 f) shows that both antibodies of the disclosure bound specifically and with high affinities to this wide range of tumor cells. EC₅₀ values of further antibodies of the disclosure on mC4.4a : CHO cells and NCI H292 cells are depicted in Table 8 .

FACS titration was performed in a 96 well microtiter plate, in which serial dilutions of the primary antibody in a volume of 50 µl of FACS buffer (3% FCS, in PBS) were mixed with 50 µl of a cell suspension consisting of 2x105 cells/ml which had been detached with Cell Dissociation Solution (1x) Non-enzymatic (Sigma), and resuspended in FACS buffer. Incubation was performed at 4 °C for 1 hour with agitation. Cells were pelleted, washed with FACS buffer and resuspended in 100 µl/well of Phycoerythrin labeled goat anti human IgG (Dianova) solution in FACS buffer. Incubation and washing was performed as before. Analysis of cell-bound antibodies was done at the respective detection wavelength using the FACS Array device. EC₅₀ values were determined from fluorescence medians of duplicates using Swift 8.0 software.

### EXAMPLE 6: Internalization

Relative internalization of anti-C4.4a antibodies on transfected C4.4a:A549 cells is shown in Figure 4 and Table 9. Internalization assays were performed with fluorescence labeled C4.4a antibodies.
The pH-sensitive fluorescent dye CypHer 5E (GE Healthcare, PA15401) was chosen as a fluorescent marker, because only at acidic pH, present in endosomes, a fluorescence signal can be detected, while at neutral or basic pH values no fluorescence is measured. For coupling, anti- C4.4a antibodies were incubated for 1h at 20°C with a 2-molar excess of dye in PBS/Na-Carbonate pH 8.3 (9:1) . On average a Lys-coupled dye load of 1.6 was achieved. Effects of coupling on affinity were tested in FACS Assays and changes of EC₅₀ values were considered negligible. 1x10⁴ A549:hC4.4a cells were used to investigate specific C4.4a internalization upon antibody binding. Cells were treated with various concentrations (34nM - 6.6nM) of labeled antibodies at 37°C/ 5% CO₂. Internalization was kinetically measured for up to 24 h using the IN Cell Analyzer 1000. Analysis of internalization was performed microscopically at 40 x magnification and by determination of granularity (granule count/cell; 620 nm excitation, and 700 nm emission for CypHer5E). Nuclei were visualized with DNA staining using cell permeable stain Hoechst 33342 (0.5µg/ml, 30min incubation, emission at 353-365 nm detection at 480 nm).

The corresponding non-C4.4a expressing vector cells were used as controls as well as A549 wild type (A549 wt) cells. A human IgG1 was selected as isotype control and was labeled in parallel. Three independent experiments were carried out; data points represent triplicate determinations. M31-B01 and M20-D02 S-A were internalized efficiently. The time of half maximal internalization was 31 min for M31-B01 and 49 min for M20-D02 S-A, respectively.

### EXAMPLE 7: Conversion from Fab to IgG format

To transfer VH and VL regions from Fab to IgG format and/or to change the expression system from *E. coli* to a mammalian cell system VH and VL were amplified using PCR primers. Flanking restriction enzyme cleavage sites were introduced at both 5' and 3' ends of VH and VL, respectively. These restriction sites were used for cloning of VH and VL into an expression vector containing i.e. an IgG backbone.

*E. coli* cells were added to 100 µl water in a test tube and incubated at 95°C for 10 min and then kept on ice for 5 min. After vortexing, bacterial debris was pelleted by centrifugation. The supernatant was used for DNA amplification. PCR reactions were prepared separately for VH and VL using specific primer pairs with BamHI and HpaI restriction sites for VL and Blp1 and Mfe1 sites for VH respectively. PCR reactions were performed with AccuPrime Pfx polymerase (Invitrogen # 12344-024) according to the manufacturers instructions. PCR products were quality checked on an 1 % agarose gel. Expression vectors and PCR products were digested for 2 h at 37°C with the respective restriction endo-nucleases to create compatible ends, according to the providers instructions. The digestion reaction was stopped by incubation at 70°C for 15 min. Resulting fragments were ligated into an expression vector and constructs were transformed into *E. coli* or mammalian cells using standard methods.

### EXAMPLE 8: Subcutaneous Xenograft cancer Model:

Antitumor effects of anti C4.4a antibodies will be evaluated using subcutaneous xenograft models in immmunodeficient mice. A431 cells are maintained as adherent cultures in DMEM supplemented with 10 % FBS. SCID mice of 6-7 weeks age will be inoculated subcutaneously in the right flank with 1 x 10e7 cells in 0.1 ml of medium. Monoclonal antibodies will be administered i.p. 3x every 3 days at a dose of 5-60 mg/kg. Control mice will be treated with PBS or an irrelevant monoclonal antibody. Tumor size will be measured every 2 days with a sliding caliper. Anti tumor efficacy will be evaluated by comparing tumor size of anti C4.4a antibody treatment versus control treatment.

### EXAMPLE 9: Subcutaneous Xenograft cancer Model with antibody drug conjugates:

Anti C4.4a antibodies can be conjugated to cytotoxic small molecules using protocols that are known in the art (e.g. Liu et al., Proc Natl. Acad. Sci. (1996), 93, 8618-8623). A431 cells are maintained as adherent cultures in DMEM supplemented with 10 % FBS. SCID mice of 6-7 weeks age will be inoculated subcutaneously in the right flank with 1 x 10e7 cells in 0.1 ml of medium. When tumor sizes reach 50 mm³ antibody drug conjugates will be administered i.p. 3x every 3 days at a dose of 1-60 mg/kg. Control mice will be treated with PBS, an irrelevant monoclonal antibody, or a free unconjugated drug Tumor size will be measured every 2 days with a sliding caliper. Anti tumor efficacy will be evaluated by comparing tumor size of anti C4.4a antibody drug conjugate treatment versus control treatment.

### EXAMPLE 10: Cloning, expression and quantification of Fab antibody fragment expression levels for affinity maturation.

The heavy and light chain of the wild-type Fabs M31-B01 and M20-D02 S-A carrying a 3xHA-tag and a hexa-histidine tag at the C-terminus of the heavy chain were subcloned into the pET28a bacterial expression vector (Novagen/Merck Chemicals Ltd., Nottingham, UK) and transformed into Top10F' cells (Invitrogen GmbH, Karlsruhe, Germany). Alternatively, other bacterial expression vectors (e.g. pQE vector system, Qiagen GmbH, Hilden, Germany) and strains (e.g. DH5 α, Invitrogen GmbH, Karlsruhe, Germany) can be used. Variants were generated by standard oligo-based site-directed mutagenesis and confirmed by DNA sequencing. In particular, amino acid residues within or surrounding complementary determining regions were modified within the heavy and/or the light chain.

For expression, variants were transformed into the BL21starDE3 Escherichia coli strain (Invitrogen, C6010-03), inoculated into an overnight culture in LB medium including kanamycin (30 µg/ml) and incubated at 37 °C for 18 hours. Expression cultures were generated by inoculating the culture 1:20 into fresh LB medium with kanamycin (30 µg/ml). After 6 hours at 37°C, 1 mM IPTG was added to induce antibody expression and cultures were incubated for additional 18 hours at 30 °C.

For quantification of expression levels an ELISA approach was used. Briefly, MTP plates (Nunc maxisorp black, 460518) were incubated with a HA epitope tag specific mAb (Covance, MMS-101P) diluted in coating buffer (Candor Bioscience GmbH, 121125) at 4°C for 16h, washed with PBST (phosphate buffered saline containing: 137mM NaCl, 2.7mM KCl, 10mM Na2HPO4, 2mM KH2PO4, pH 7.4, 0.05% Tween 20), blocked with 100% Smart Block (Candor Bioscience GmbH, 113500) for 1h at 20°C and washed again. Cultures were diluted in 10% Smart Block in PBST and bound to the MTP plates for 1h at 20°C. After washing with PBST, captured antibodies were incubated with a HRP-coupled anti-lambda antibody (Sigma, A5175) and detected by incubating the plate with 10µM amplex red substrate (Invitrogen, A12222) for 30 minutes at 20°C in the dark followed by fluorescence measurement. Determined quantification signals were checked to be in the dynamic range of an calibration series built up with concentrated parental Fab (M31-B01 or M20-D02-S-A) expression culture supernatant allowing a relative quantification of each individual Fab expression sample.

### EXAMPLE 11: Determination of activity and interspecies cross reactivity of generated Fab antibody fragment variants

To determine the activity of the mutated Fab variants on recombinant soluble human or mouse C4.4a a direct ELISA assay format was used. Briefly, MTP plates (Nunc maxisorp black, 460518) were coated with 2 µg/ml either human or mouse C4.4a diluted in coating buffer (Candor Bioscience GmbH, 121125) and incubated for 15 h at 4 °C. After washing 3 times with PBST, plates were blocked with 100% Smart Block (Candor Bioscience GmbH, 113500) for 1h at 20°C and the washing steps were repeated. For binding of the Fab antibody fragments 20 µl of culturing supernatants were added to the plates for 1h at 20°C followed by washing. In some cases the washing stringency was increased by an additional incubation step with 10% Smart Block in PBST for 90min followed by washing 3 times with PBST ("process B" in Table 10b). Bound parental Fabs and variants were then detected by a HA-epitope tag specific antibody_HRP conjugate (Bethyl, A190-108P). 10µM amplex red substrate (Invitrogen, A12222) was added to the plates and the fluorescence signal was detected using a common fluorescence reader, e.g. Tecan Ultra. Quantity normalized affinity signals were calculated as ratios of background corrected affinity and quantification signals in the respective ELISA: (Signalaffinity - Backgroundaffinity) / (Signalquantity - Backgroundquantity). The resulting values correlate mainly with the affinity in a positive manner, whereas not normalized values correlate additionally with the concentration of the binding Fab in the sample. Hence, the quantity normalized affinity signals serve as very good guidance for the determination of variants improved in affinity.

### EXAMPLE 12: Single and multiple amino acid substitutions

Provided in Table 10a and 10b are several examples of single amino acid substitutions generated in the heavy and light chains of M20-D02 S-A. HC D101K, LC A90Q, LC V97A and LC V97G showed the strongest improvement regarding the normalized affinity signals on human C4.4a, with a corresponding signal on mouse C4.4a of at least 1/8 compared with the signal on human C4.4a (Table 10a). Beyond the CDR3s two substitutions, HC D31S and HC V51I showed the strongest improvement regarding the normalized affinity signals on human C4.4a, with a corresponding signal on mouse C4.4a of 30% in the case of HC V51I (Table 10b).

Table 10a. Analysis of single amino acid substitutions within the CDR3 of heavy and light chains of M20-D02 S-A. Normalized affinity signals on human and mouse C4.4a the average and respective standard deviation of two measurements in at least quadruplets are listed.

**Tabel 10a:**

| | | | | | human | | mouse | |
|---|---|---|---|---|---|---|---|---|
| pos | in SEQ ID NO: | pos | in SEQ ID NO: | Name | average | *stdev* | average | *stdev* |
| 9 | 14 | 105 | 34 | HC S100aR | 0.43 | *0.13* | 0.02 | *0.01* |
| 9 | 14 | 105 | 34 | HC S100aK | 0.45 | *0.21* | 0.06 | *0.01* |
| 10 | 14 | 106 | 34 | HC A100bR | 0.87 | *0.21* | 0.01 | *0.01* |
| 10 | 14 | 106 | 34 | HC A100bS | 0.34 | *0.17* | 0.02 | *0.00* |
| 14 | 14 | 110 | 34 | HC D101E | 0.31 | *0.24* | 0.05 | *0.00* |
| 14 | 14 | 110 | 34 | HC D101K | 1.65 | *0.48* | 0.21 | *0.09* |
| 14 | 14 | 110 | 34 | HC D101R | 0.61 | *0.18* | 0.03 | *0.01* |
| 3 | 26 | 91 | 30 | LC A90Q | 1.41 | *0.67* | 0.29 | *0.15* |
| 3 | 26 | 91 | 30 | LC A90R | 0.94 | *0.47* | 0.17 | *0.04* |
| 5 | 26 | 93 | 30 | LC D92G | 0.64 | *0.25* | 0.10 | *0.04* |
| 9 | 26 | 97 | 30 | LC N95aW | 0.11 | *0.06* | 0.00 | *0.01* |
| 12 | 26 | 100 | 30 | LC V97A | 1.32 | *0.21* | 0.52 | *0.11* |
| 12 | 26 | 100 | 30 | LC V97G | 1.21 | *0.31* | 0.30 | *0.13* |
| | | | 34 - VH | M20-D02 S-A | 0.04 | *0.03* | 0.01 | *0.01* |
| | | | 30 - VL | | | | | |

Table 10b. Analysis of single amino acid substitutions beyond the CDR3 of heavy and light chains of M20 D02-S-A. Normalized affinity signals on human and murine C4.4a, respectively are listed; given numbers are medians of a measurement in quadruplets. The difference between process B and A is an additional incubation step in buffer for 90 min in process B.

**Table 10b:**

| | | | | | process A | | process B | |
|---|---|---|---|---|---|---|---|---|
| pos | in SEQ ID NO: | pos | in SEQ ID NO: | name | human | mouse | Human | mouse |
| 3 | 6 | 31 | 34 | HC D31S | | | 0.84 | 0.04 |
| 4 | 10 | 51 | 34 | HC V51I | 0.52 | 0.16 | | |
| 9 | 10 | 56 | 34 | HC A55G | 0.15 | 0.04 | | |
| 10 | 10 | 57 | 34 | HC R56S | 0.21 | 0.06 | | |
| | | | 34 - VH | M20-D02 S-A | 0.15 | 0.04 | 0.06 | 0.00 |
| | | | 30 - VL | | | | | |

Provided in Table 11a and 11b are several examples of single amino acid substitutions generated in the heavy and light chains of B01. LC W91E, LC W91F, LC W91Y and LC G95bR showed the strongest improvement regarding the normalized affinity signals on human C4.4a, with a corresponding signal on mouse C4.4a of at least 1/8 compared with the signal on human C4.4a, the latter three of 40% and more (Table 11a). Beyond the CDR3s the substitution HC A32Y showed the strongest improvement regarding the normalized affinity signals on human C4.4a (Table 11b).

Table 11a. Analysis of single amino acid substitutions within the CDR3 of heavy and light chains of M31-B01. Normalized affinity signals on human and mouse C4.4a are listed; the average and respective standard deviation of two measurements result from at least quadruplets.

**Table 11 a:**

| | | | | | human | | mouse | |
|---|---|---|---|---|---|---|---|---|
| pos | in SEQ ID NO: | pos | in SEQ ID NO: | name | average | *stdev* | averag e | *stdev* |
| 10 | 13 | 106 | 33 | HC Y102K | 1.49 | *0.12* | 0.21 | *0.02* |
| 10 | 13 | 106 | 33 | HC Y102W | 1.65 | *0.15* | 0.13 | *0.03* |
| 4 | 25 | 93 | 29 | LC W91E | 3.17 | *0.44* | 0.40 | *0.02* |
| 4 | 25 | 93 | 29 | LC W91F | 4.30 | *0.78* | 2.53 | *0.39* |
| 4 | 25 | 93 | 29 | LC W91Y | 4.97 | *0.91* | 2.79 | *0.68* |
| 7 | 25 | 96 | 29 | LC R94M | 1.30 | *0.20* | 0.13 | *0.02* |
| 9 | 25 | 98 | 29 | LC N95aK | 1.11 | *0.01* | 0.12 | *0.04* |
| 10 | 25 | 99 | 29 | LC G95bR | 2.45 | *0.49* | 1.01 | *0.12* |
| 11 | 25 | 100 | 29 | LC P96A | 0.95 | *0.47* | 0.32 | *0.16* |
| | | | 33 - VH | M31- B01 | 1.11 | *0.34* | 0.13 | *0.07* |
| | | | 29 - VL | | | | | |

Table 11b. Analysis of single amino acid substitutions beyond the CDR3 of heavy and light chains of B01. Normalized affinity signals on human and mouse C4.4a are listed; given numbers are medians of a measurement in at least triplicates.

**Table 11b:**

| pos | in SEQ ID NO: | pos | in SEQ ID NO: | name | human | mouse |
|---|---|---|---|---|---|---|
| 3 | 5 | 31 | 33 | HC N31S | 1.53 | 0.32 |
| 4 | 5 | 32 | 33 | HC A32Y | 2.31 | 0.01 |
| 10 | 9 | 57 | 33 | HC T56S | 1.58 | 0.74 |
| 11 | 9 | 58 | 33 | HC I57T | 1.50 | 0.73 |
| 1 | 17 | 23 | 29 | LC T24S | 0.77 | 0.23 |
| 4 | 21 | 55 | 29 | LC K53Q | 1.12 | 0.35 |
| | | | 33 - VH | B01 | 1.23 | 0.16 |
| | | | 29 - VL | | | |

Provided in Table 12a and 12b are some examples of combined amino acid substitutions within M20-D02 S-A anti-C4.4a antibodies. Some substitutions were analyzed as single amino acid substitution (Table 10a and 10b), further were only analyzed as part of this combined substitutions. While not every combination is provided in Table 12a and 12b, it is contemplated that the anti-C4.4a antibody may comprise any combination of modifications provided in Table 10a and 10b.

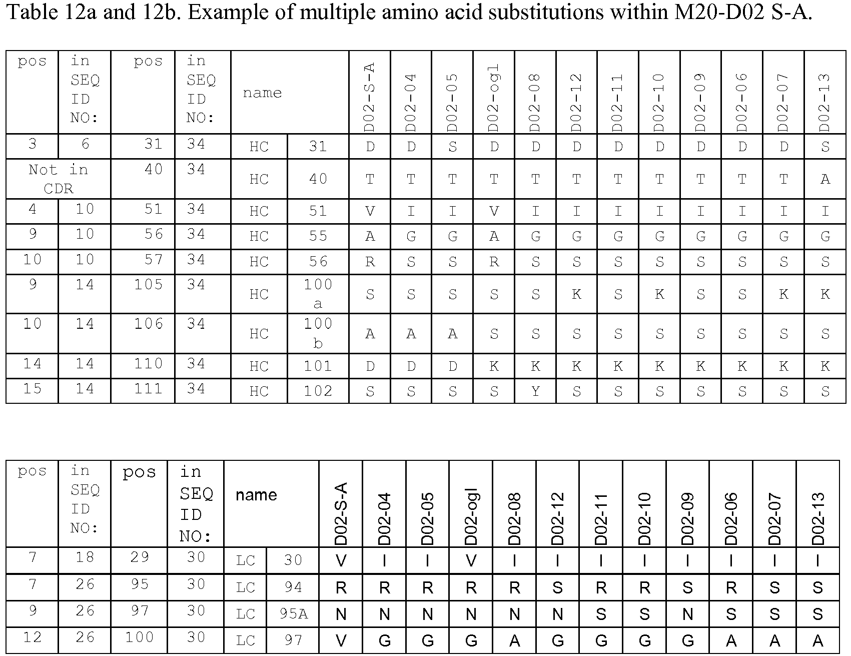

Provided in Table 13a and 13b are some examples of combined amino acid substitutions within M31-B01 anti-C4.4a antibodies. Some substitutions were analyzed as single amino acid substitution (Table 11a and 11b), further were only analyzed as part of this combined substitutions. While not every combination is provided in Table 13a and 13b, it is contemplated that the anti-C4.4a antibody may comprise any combination of modifications provided in Table 11a and 11b.

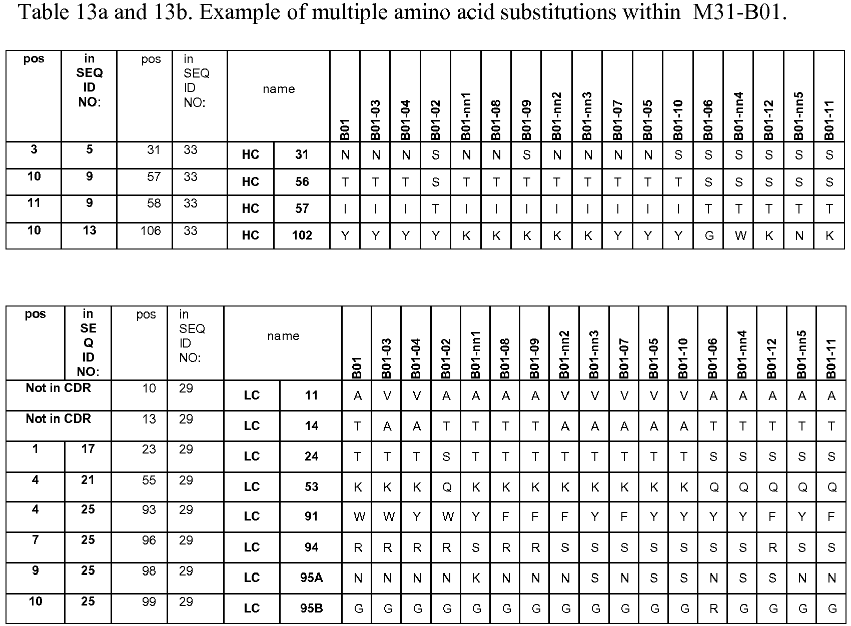

Provided in Table 14a and 14b are CDR sequence stretches with potential deamidation sites, specifically Asn-Gly ("NG") and Asn-Ala ("NA") in M20-D02 S-A and M31-B01, as well as the respective sequences of some corresponding variants with multiple amino acid substitutions.

In M20 D02-S-A there are two potential deamidation sites in CDRs, one in CDR-L3 and one in CDR-H2. In D02-11, -10, -06, -07 and -13 the site in CDR-L3 is removed by an N>S substitution.

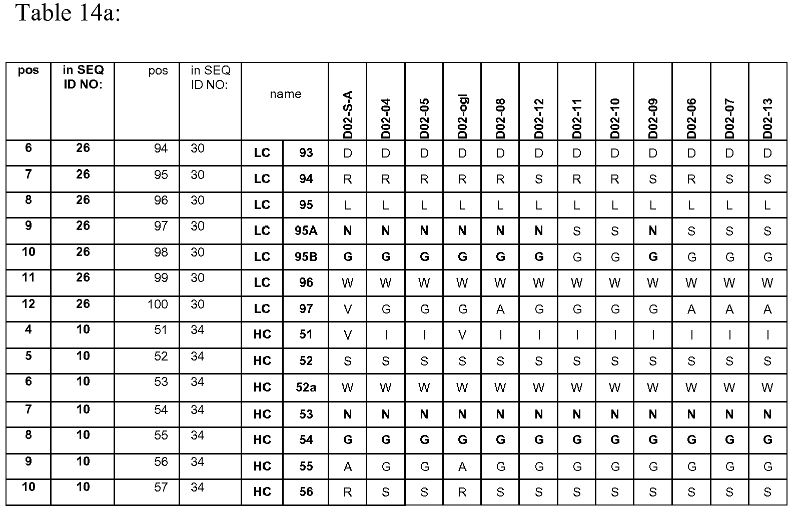

In M31-B01 there are two potential deamidation sites in CDRs, one in CDR-L3 and one in CDR-H1. In B01-nn3, -05, -10, -nn4 and -12 the site in CDR-L3 is removed by a N>S substitution, in B01-nn1 by a N>K substitution and in B01-06 by a G>R substitution. In B01-02, -09, -10, -06, -nn4, -12, -nn5 and -11 the site in CDR-H1 is removed by a N>S substitution. B01-10, -06, -nn4 and -12 show none of these both potential deamidations sites.

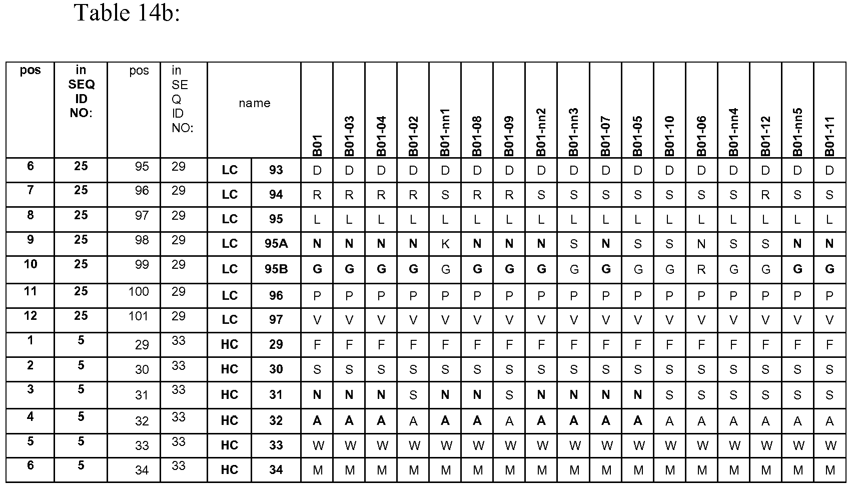

The methods described in examples 10-12 were used to generate affinity matured antibodies by single mutations and combinations of single mutations thereof. These methods are well suitable for generation of competing antibodies, derived from a parental antibody. The antibodies of the above mentioned examples are depicted in table 7. These examples provide further C4.4a binding antibodies or antigen-fragment-binding fragments which comprise CDR H1 sequences that are at least 77% identical to the CDR H1 of M31 B01, CDR H2 sequences that are at least 90% identical to the CDR H2 of M31 B01, CDR H3 sequences that are at least 90% identical to the CDR H3 of M31 B01, CDR L1 sequences that are at least 92% identical to the CDR L1 of M31 B01, CDR L2 sequences that are at least 85% identical to the CDR L2 of M31 B01 and CDR L3 sequences that are at least 58% identical to the CDR L3 of M31 B01 (those antibody variants compete in binding with M31 B01) and antibodies or antigen-fragment-binding proteins which comprise CDR H1 sequences that are at least 88% identical to the CDR H1 of M20 D02 S-A, CDR H2 sequences that are at least 85% identical to the CDR H2 of M20 D02 S-A, CDR H3 sequences that are at least 73% identical to the CDR H3 of M20 D02 S-A, CDR L1 sequences that are at least 92% identical to the CDR L1 of M20 D02 S-A, CDR L2 sequences that are at least 100% identical to the CDR L2 of M20 D02 S-A and CDR L3 sequences that are at least 58% identical to the CDR L3 M20 D02 S-A (those antibody variants compete in binding with M20 D02 S-A).

Table 15a: This table summarizes the amino acid variations of CDRs 1-3 of heavy and light chains for M20 D02 S-A as described in method 12.

The following represents the above results in form of a consensus sequence of the respective CDR sequences derived from M20 D02 S-A. The consensus for CDR H1 is depicted in Table 15a (i) (SEQ ID NO: 297), for CDR H2 is depicted in Table 15a (ii) (SEQ ID NO: 298), for CDR H3 is depicted in Table 15a (iii) (SEQ ID NO: 299), for CDR L1 is depicted in Table 15a (iv) (SEQ ID NO: 300), for CDR L2 is identical to SEQ ID NO: 22 (Table 15a (v)), for CDR L3 is depicted in Table 15a (v) (SEQ ID NO: 301), respectively.

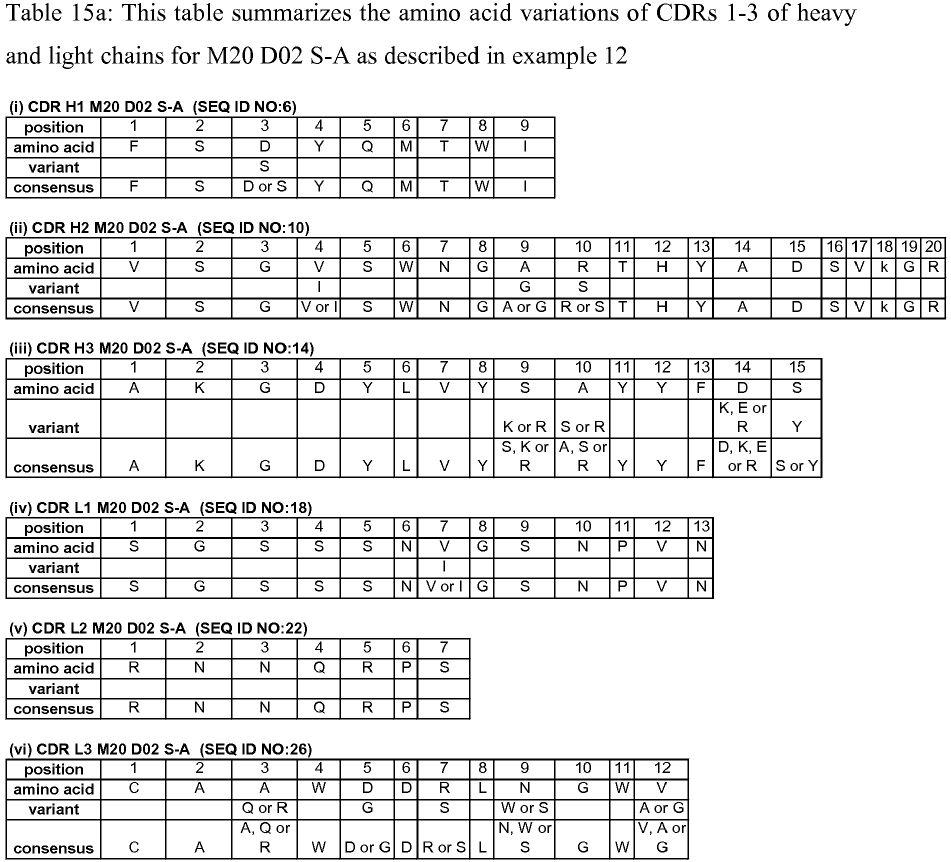

The following represents the above results in form of a consensus sequence of the respective CDR sequences derived from M31 B01. The consensus for CDR H1 is depicted in Table 15b (i) (SEQ ID NO: 302), for CDR H2 is depicted in Table 15b (ii) (SEQ ID NO: 303), for CDR H3 is depicted in Table 15b (iii) (SEQ ID NO: 304), for CDR L1 is depicted in Table 15b (iv) (SEQ ID NO: 305), for CDR L2 is depicted in Table 15b (v) (SEQ ID NO: 306), for CDR L3 is depicted in Table 15b (v) (SEQ ID NO: 307), respectively.

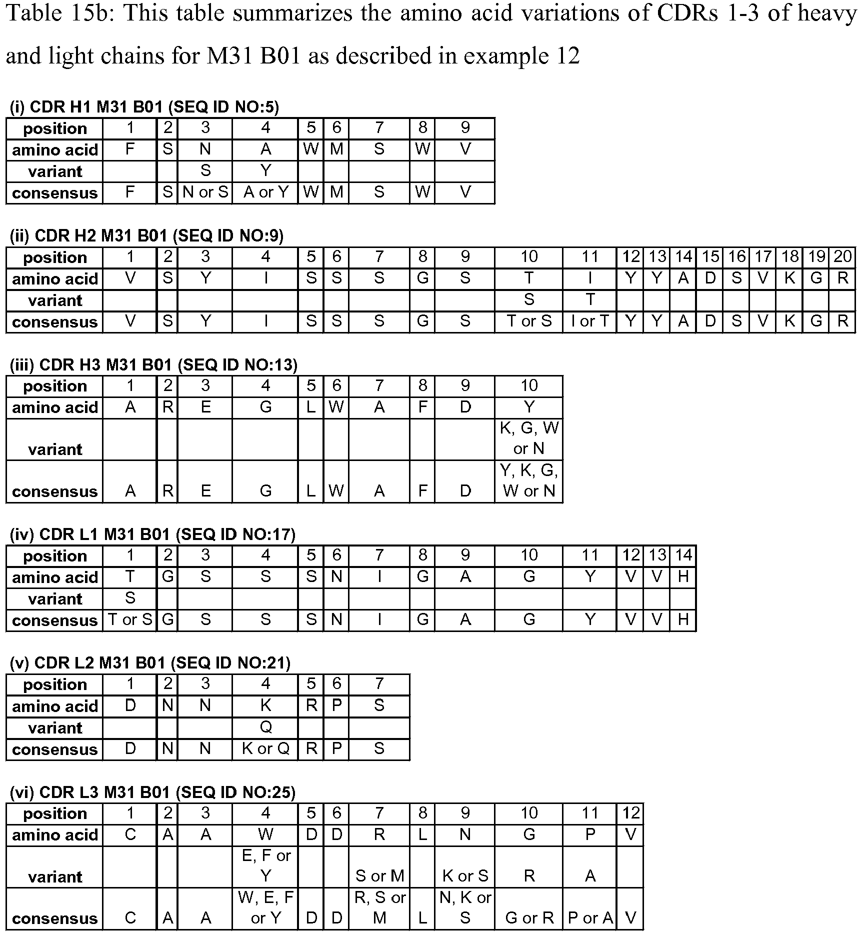

### EXAMPLE 13: Determination of antibody binding to C4.4a single domains by Western Blot

To characterize the antibody binding domain of M31-B01 and M20-D02 S-A constructs consisting of C4.4a domain S1 amino acids 1-85 and C4.4 domain S2 108-193 of human C4.4a SEQ ID NO: 1 respectively, were generated as C-terminal human IgG1 Fc-6xHisTag fusions, cloned into a mammalian expression vector containing a CMV5 promotor and expressed transiently in HEK293 6E cells by standard methods. The expressed S1 and S2 proteins were then purified from cell supernatants by metal chelate chromatography using a 5 ml NiNTA superflow column (Qiagen) at 20°C. The samples were pH adjusted to pH 8.0 with 1M NaOH and then applied to the column previously equilibrated with 50mM NaH2PO4 + 300mM NaCl pH 8.0 at 1ml /min. The column was washed with 15 CV of equilibration buffer and bound HisTagged proteins were eluted with 50mM NaH2PO4 + 300mM NaCl + 250mM Imidazol pH 8.0. The eluted proteins were further purified on a Tricorn Superdex 75 10/300 GL (GE Healthcare) by size exclusion chromatography.

For further analysis full length (C4.4a SEQ ID NO: 1), the purified S1-Fc-6xHisTag fusion and S2 Fc-6xHisTag were applied to different lanes of a precast NuPage Bis-Tris 4 - 12% gradient gel (Invitrogen, No. NP0322Box) and separated by electrophoresis, using the NuPage MES SDS Running Puffer (Invitrogen NP0002) according to the manufacturer's instructions.

The SDS gel was then blotted to a iblot gel transfer membrane (Invitrogen IB3010-02) using an iBlot device (Invitrogen). The membrane was blocked in DPBS pH 7.4 + 4 % milk powder + 0.1 % Tween 20 for 16 h at 4°C, followed by three washing steps for 5 min at 20°C in DPBS pH 7.4 + 0.1 % Tween 20. M31-B01 (5.76 mg/ml) or M20-D02 S-A (3.49mg/ml) were applied in dilutions of 1/5000 and 1/2000 respectively and incubated for 1.5 h at 20°C, followed by 2x washes in DPBS pH 7.4 + 0.1 % Tween 20. The second antibody (alk. Phosphatase. Conjugate Goat anti human IgG, Fab specific; 109-055-097 Jackson ImmunoResearch) was incubated at a dilution of 1/1000 for 1.5 h at 20°C. Followed by 3 washing steps for 5 min at 20°C in DPBS pH 7.4 + 0.1 % Tween 20. Staining was performed with Sigma Fast BCIP/NBT / 1 tablet in 10 ml water at 20°C till the bands became visible. Staining reaction was stopped by washing with water.

Both antibodies M31-B01 and M20-D02 S-A bind to full length human and mouse C4.4a. in addition they bind to domain S1 of C4.4a but not to domain S2 of C4.4a as depicted in Fig. 6. Binding can be exclusively detected in non DTT reduced samples, strongly indicating presence of a conformation dependent epitope which is stabilized by one or more disulfide bridges.

### EXAMPLE 14: Antibody dependent cell mediated cytotoxicity assays (ADCC assays)

Anti tumor activity of anti-C4.4a IgGs can be mediated by ADCC. C4.4a expressing A549:hC4.4a cells and non C4.4a expressing parental cells are incubated with 250 ng/ml, 1000 ng/ml or 2000 ng/ml human anti-C4.4a or control IgG1 antibody (e.g. anti-digoxin antibody). Human PBMCs are added to theses cells at effector-target ratios of 50:1, 25:1 and 5:1 ratios. A chromium-51 release assay is performed to determine the level of target lysis. If the rate of lysis in the presence of anti-C4.4a antibody and PBMCs is higher than the rate of (spontaneous) lysis in the presence of mock antibody or no antibody and PBMCs this indicates ADCC is effective.

### EXAMPLE 15: Antibody dependent inhibition of proliferation - Cell Proliferation measured by the xCELLigence System

The xCELLigence System (RTCA analyzer CatNo.05228972001, Roche) monitors cellular events in real time without the incorporation of labels. The system measures electrical impedance across interdigitated micro-electrodes integrated on the bottom of tissue culture E-Plates. Thee impedance measurement provides quantitative information about the biological status of the cells, including cell number, viability and morphology. To determine the rate of cell proliferation of A549:hC4.4a cells were incubated either with of an non-binding hIgG1 isotype control antibody or 100 nM of B01-3 (as hIgG1). First 50µl of cell culture medium (RPMI (Biochrom FG1640) + 10 % FCS (Biochrom #S0145) + 1 µg/ml Puromycin (Sigma 8833)) was added to each well of an E-Plate 96. The E-Plate 96 was inserted into the RTCA MP Station (CatNo.05331625001, Roche) and the background impedance of each well was determined. Solutions where removed again from the E-Plate 96 and 50µl of cell suspension (5000 cells/well of A549:hC4.4a in cell culture medium) were added to the wells. Plates were reinserted and measured for 4 hours at 37°C + 5 % CO2. The E-Plate 96 was removed again and 100µl of antibodies (100nM of B01-3 or non binding control hIgG1) in cell culture medium were added. All samples were run in triplicates. After reinsertion of the E-plate the measurement was conducted for 92 hours at 37°C + 5 % CO₂. Results were analyzed with the built-in software package. Results are depicted in figure 6. Antibody B01-3 clearly decreases cell proliferation compared to a non-binding control antibody. This shows that the antibodies of the invention effectively inhibit cellular proliferation of C4.4a expressing cells.

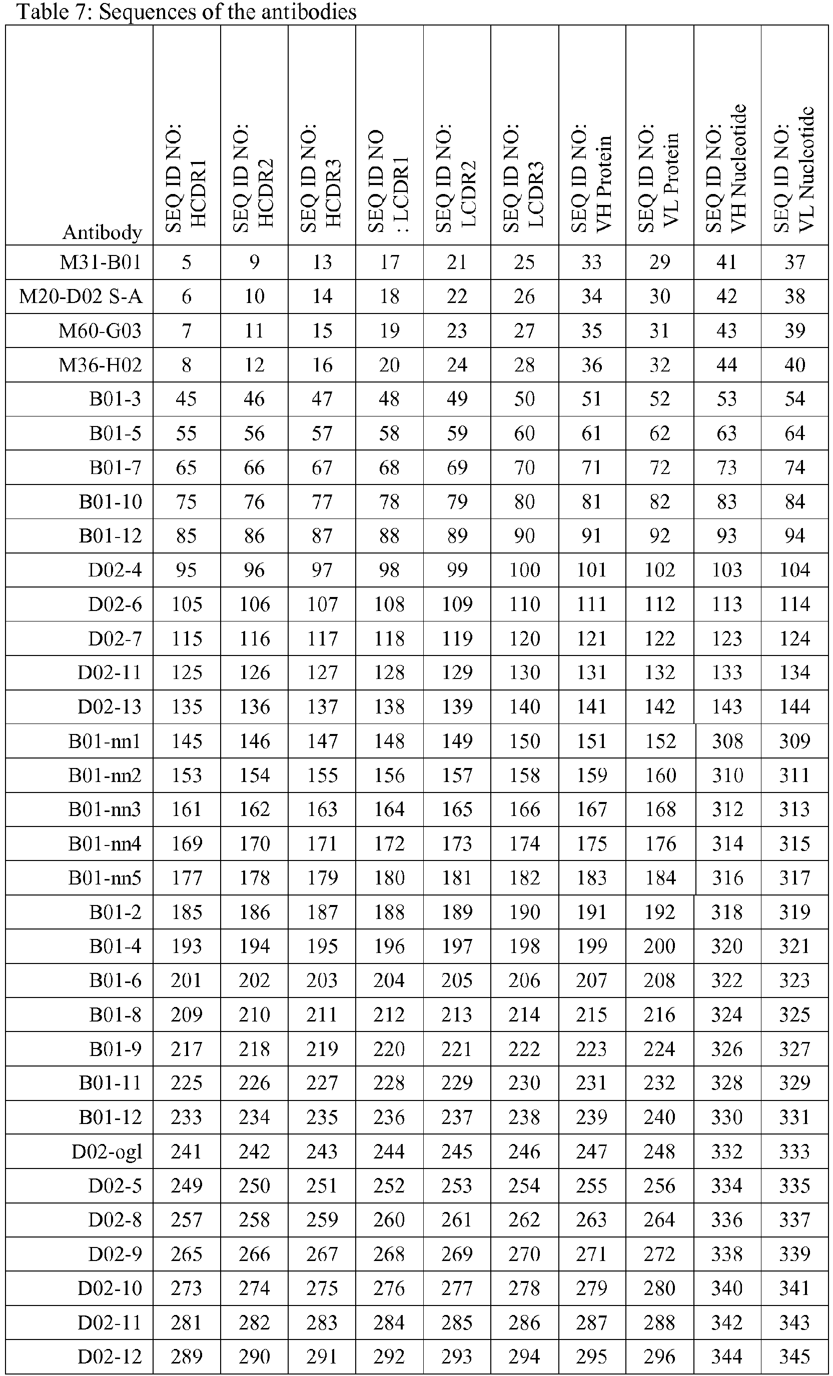

## Claims

1. An isolated antibody or antigen-binding fragment thereof specifically binding to amino acids 1 - 85 of SEQ ID NO:1,
wherein said antibody or antigen-binding fragment thereof is internalized following binding to C4.4a expressing cells,
and wherein said antibody or antigen-binding fragment thereof comprises the variable heavy chain CDR sequences:
H-CDR1 comprising SEQ ID NO:45, H-CDR2 comprising SEQ ID NO:46, and H-CDR3 comprising SEQ ID NO:47,
and wherein said antibody or antigen-binding fragment thereof comprises the variable light chain CDR sequences:
L-CDR1 comprising SEQ ID NO:48, L-CDR2 comprising SEQ ID NO:49, and
L-CDR3 comprising SEQ ID NO:50.

2. The antibody or antigen binding fragment according to claim 1 comprising
a variable heavy chain sequence as presented by SEQ ID NO: 33 and a variable light chain sequence as presented by SEQ ID NO: 29,
or a variable heavy chain sequence as presented by SEQ ID NO: 51 and a variable light chain sequence as presented by SEQ ID NO: 52.

3. The antibody according to claim 1 or 2, which is an IgG antibody.

4. The antigen-binding fragment according to claim 1 or 2, which is an scFv, Fab, Fab' fragment or a F(ab')₂ fragment.

5. The antibody or antigen-binding fragment according to any one of the preceding claims, which is a monoclonal antibody or antigen-binding fragment.

6. The antibody or antigen-binding fragment according to any one of the preceding claims, which is human, humanized or chimeric antibody or antigen-binding fragment.

7. An antibody-drug conjugate, comprising an antibody or antigen binding fragment thereof according to claims 1 to 6.

8. An isolated nucleic acid sequence that encodes the antibody or antigen-binding fragment according to claims 1 to 6.

9. A vector comprising a nucleic acid sequence according to claim 8.

10. An isolated cell expressing an antibody or antigen-binding fragment according to any one of the claims 1 - 6 and /or comprising a nucleic acid according to claim 8 or a vector according to claim 9.

11. An isolated cell according to claim 10, wherein said cell is a prokaryotic or an eukaryotic cell.

12. A method of producing an antibody or antigen-binding fragment according to any one of the claims 1 - 6 comprising culturing of a cell according to claim 11 and purification of the antibody or antigen-binding fragment.

13. An antibody or antigen-binding fragment according to claims 1 - 6 or an antibody-drug conjugate according to claim 7 for use as a medicament.

14. An antibody or antigen-binding fragment according to claims 1 - 6 for use as a diagnostic agent.

15. An antibody or antigen-binding fragment according to claims 1 - 6 or an antibody-drug conjugate according to claim 7 for use as a medicament for the treatment of cancer.

16. A pharmaceutical composition comprising an antibody or antigen-binding fragment according to claims 1 - 6 or an antibody-drug conjugate according to claim 7.

17. A combination of a pharmaceutical composition according to claim 16 and one or more therapeutically active compounds.

## Patentansprüche

1. Isolierter Antikörper oder antigenbindendes Fragment davon mit spezifischer Bindung an Aminosäuren 1 - 85 von SEQ ID NO: 1,
wobei der Antikörper bzw. das antigenbindende Fragment davon nach Bindung an C4.4a exprimierende Zellen internalisiert wird
und wobei der Antikörper bzw. das antigenbindende Fragment davon die folgenden variablen Schwere-Kette-CDR-Sequenzen umfasst:
H-CDR1, umfassend SEQ ID NO: 45, H-CDR2, umfassend SEQ ID NO: 46 und H-CDR3, umfassend SEQ ID NO: 47,
und wobei der Antikörper bzw. das antigenbindende Fragment davon die folgenden variablen Leichte-Kette-CDR-Sequenzen umfasst:
L-CDR1, umfassend SEQ ID NO: 48, L-CDR2, umfassend SEQ ID NO: 49 und L-CDR3, umfassend SEQ ID NO: 50.

2. Antikörper oder antigenbindendes Fragment nach Anspruch 1, umfassend
eine variable Schwere-Kette-Sequenz gemäß SEQ ID NO: 33 und eine variable Leichte-Kette-Sequenz gemäß SEQ ID NO: 29
oder eine variable Schwere-Kette-Sequenz gemäß SEQ ID NO: 51 und eine variable Leichte-Kette-Sequenz gemäß SEQ ID NO: 52.

3. Antikörper nach Anspruch 1 oder 2, bei dem es sich um einen IgG-Antikörper handelt.

4. Antigenbindendes Fragment nach Anspruch 1 oder 2, bei dem es sich um ein scFv-, Fab-, Fab'-Fragment oder ein F(ab')₂-Fragment handelt.

5. Antikörper oder antigenbindendes Fragment nach einem der vorhergehenden Ansprüche, bei dem es sich um einen monoklonalen Antikörper bzw. ein monoklonales antigenbindendes Fragment handelt.

6. Antikörper oder antigenbindendes Fragment nach einem der vorhergehenden Ansprüche, bei dem es sich um einen menschlichen, humanisierten oder chimären Antikörper bzw. ein menschliches, humanisiertes oder chimäres antigenbindendes Fragment handelt.

7. Antikörper-Arzneistoff-Konjugat, umfassend einen Antikörper oder ein antigenbindendes Fragment davon nach Anspruch 1 bis 6.

8. Isolierte Nukleinsäuresequenz, die den Antikörper bzw. das antigenbindende Fragment nach Anspruch 1 bis 6 codiert.

9. Vektor, umfassend eine Nukleinsäuresequenz nach Anspruch 8.

10. Isolierte Zelle, exprimierend einen Antikörper oder ein antigenbindendes Fragment nach einem der Ansprüche 1 - 6 und/oder umfassend eine Nukleinsäure nach Anspruch 8 oder einen Vektor nach Anspruch 9.

11. Isolierte Zelle nach Anspruch 10, wobei es sich bei der Zelle um eine prokaryontische oder eine eukaryontische Zelle handelt.

12. Verfahren zur Herstellung eines Antikörpers oder antigenbindenden Fragments nach einem der Ansprüche 1 - 6, umfassend Kultivieren einer Zelle nach Anspruch 11 und Aufreinigung des Antikörpers bzw. antigenbindenden Fragments.

13. Antikörper oder antigenbindendes Fragment nach Anspruch 1 - 6 oder Antikörper-Arzneistoff-Konjugat nach Anspruch 7 zur Verwendung als Arzneimittel.

14. Antikörper oder antigenbindendes Fragment nach Anspruch 1 - 6 zur Verwendung als Diagnostikum.

15. Antikörper oder antigenbindendes Fragment nach Anspruch 1 - 6 oder Antikörper-Arzneistoff-Konjugat nach Anspruch 7 zur Verwendung als Arzneimittel für die Krebsbehandlung.

16. Pharmazeutische Zusammensetzung, umfassend einen Antikörper oder ein antigenbindendes Fragment nach Anspruch 1 - 6 oder ein Antikörper-Arzneistoff-Konjugat nach Anspruch 7.

17. Kombination einer pharmazeutischen Zusammensetzung nach Anspruch 16 und einer oder mehrerer therapeutisch wirksamer Verbindungen.

## Revendications

1. Anticorps isolé ou fragment de celui-ci de liaison à l'antigène se liant spécifiquement aux acides aminés 1 à 85 de la SEQ ID n° : 1,
dans lequel ledit anticorps ou fragment de celui-ci de liaison à l'antigène est internalisé après une liaison à des cellules exprimant un C4.4a,
et dans lequel ledit anticorps ou fragment de celui-ci de liaison à l'antigène comprend les séquences variables de CDR de chaîne lourde :
H-CDR1 comprenant la SEQ ID n° : 45, H-CDR2 comprenant la SEQ ID n° : 46 et H-CDR3 comprenant la SEQ ID n° : 47,
et dans lequel ledit anticorps ou fragment de celui-ci de liaison à l'antigène comprend les séquences variables de CDR de chaîne légère :
L-CDR1 comprenant la SEQ ID n° : 48, L-CDR2 comprenant la SEQ ID n° : 49 et L-CDR3 comprenant la SEQ ID n° : 50.

2. Anticorps ou fragment de liaison à l'antigène, selon la revendication 1, comprenant une séquence variable de chaîne lourde, telle que présentée par la SEQ ID n° : 33, et une séquence variable de chaîne légère telle que présentée par la SEQ ID n° : 29,
ou une séquence variable de chaîne lourde, telle que présentée par la SEQ ID n° : 51, et une séquence variable de chaîne légère telle que présentée par la SEQ ID n° : 52.

3. Anticorps, selon la revendication 1 ou la 2, qui est un anticorps IgG.

4. Fragment de liaison à l'antigène, selon la revendication 1 ou la 2, qui est un fragment scFv, Fab, Fab' ou un fragment F(ab')₂.

5. Anticorps ou fragment de liaison à l'antigène, selon l'une quelconque des revendications précédentes, qui est un anticorps monoclonal ou un fragment de liaison à l'antigène.

6. Anticorps ou fragment de liaison à l'antigène, selon l'une quelconque des revendications précédentes, qui est un anticorps humain, humanisé ou chimère ou un fragment de liaison à l'antigène.

7. Conjugué d'anticorps-substance médicamenteuse, comprenant un anticorps ou un fragment de celui-ci de liaison à l'antigène selon les revendications 1 à 6.

8. Séquence d'acide nucléique isolé qui code pour l'anticorps ou un fragment de liaison à l'antigène selon les revendications 1 à 6.

9. Vecteur comprenant une séquence d'acide nucléique selon la revendication 8.

10. Cellule isolée exprimant un anticorps ou un fragment de liaison à l'antigène, selon l'une quelconque des revendications 1 à 6, et/ou comprenant un acide nucléique, selon la revendication 8, ou un vecteur selon la revendication 9.

11. Cellule isolée selon la revendication 10, dans laquelle ladite cellule est une cellule procaryote ou eucaryote.

12. Procédé de production d'un anticorps ou d'un fragment de liaison à l'antigène, selon l'une quelconque des revendications 1 à 6, comprenant la culture d'une cellule, selon la revendication 11, et la purification de l'anticorps ou d'un fragment de liaison à l'antigène.

13. Anticorps ou fragment de liaison à l'antigène, selon les revendications 1 à 6, ou conjugué d'anticorps-substance médicamenteuse, selon la revendication 7, destiné à être utilisé en tant que médicament.

14. Anticorps ou fragment de liaison à l'antigène, selon les revendications 1 à 6, destiné à être utilisé comme agent diagnostique.

15. Anticorps ou fragment de liaison à l'antigène, selon les revendications 1 à 6, ou conjugué d'anticorps-substance médicamenteuse, selon la revendication 7, destiné à être utilisé en tant que médicament pour le traitement d'un cancer.

16. Composition pharmaceutique comprenant un anticorps ou un fragment de liaison à l'antigène, selon les revendications 1 à 6, ou un conjugué d'anticorps-substance médicamenteuse selon la revendication 7.

17. Combinaison d'une composition pharmaceutique, selon la revendication 16, et d'un ou de plusieurs composé(s) actif(s) d'un point de vue thérapeutique.
